# EUROPEAN PATENT SPECIFICATION

(11) **EP 2 689 024 B1**
(45) Date of publication and mention of the grant of the patent: **13.01.2016**
(21) Application number: 12710995.7
(22) Date of filing: 26.03.2012
(51) Int. Cl.: C12Q 1/00

(54) **ENZYME ASSAYS**
ENZYMTESTS
ESSAIS ENZYMATIQUES

(30) Priority: 25.03.2011 GB 201105118; 27.06.2011 GB 201110920; 16.09.2011 GB 201116030
(43) Date of publication of application: 29.01.2014
(73) Proprietor: Almac Sciences (Scotland) Limited, Edinburgh EH1 2EN (GB)
(72) Inventor: COTTON, Graham, Craigavon BT63 5QD (GB); DUNSMORE, Colin, Craigavon BT63 5QD (GB)
(74) Representative: Boult Wade Tennant
(86) International application number: PCT/GB2012/050661
(87) International publication number: WO 2012/131345

(56) References cited:
- WO-A2-2007/049057
- WIGLE TIM J ET AL: "Accessing protein methyltransferase and demethylase enzymology using microfluidic capillary electrophoresis.", CHEMISTRY & BIOLOGY 30 JUL 2010 LNKD- PUBMED:20659682, vol. 17, no. 7, 30 July 2010 (2010-07-30), pages 695-704, XP002679990, ISSN: 1879-1301
- MALTMAN BEATRICE A ET AL: "9-Aminoacridine peptide derivatives as versatile reporter systems for use in fluorescence lifetime assays.", 7 October 2010 (2010-10-07), CHEMICAL COMMUNICATIONS (CAMBRIDGE, ENGLAND) 7 OCT 2010 LNKD- PUBMED:20733984, VOL. 46, NR. 37, PAGE(S) 6929 - 6931, XP002679976, ISSN: 1364-548X page 6930 - page 6931; figures 2,3
- Berthold Detection Systems, Germany: "LF502 NanoScan FLT Multimode Fluorescence Microplate Reader", , 1 March 2010 (2010-03-01), XP002679977, Retrieved from the Internet: URL:www.berthold-ds.com/download.php?id=10 1 [retrieved on 2012-06-21]
- PATERSON MICHAEL J ET AL: "A fluorescence lifetime-based assay for serine and threonine kinases that is suitable for high-throughput screening", July 2010 (2010-07), ANALYTICAL BIOCHEMISTRY, VOL. 402, NR. 1, PAGE(S) 54-64, XP009160374, ISSN: 0003-2697
- AHRENS THOMAS ET AL: "Efficient hit-finding approaches for histone methyltransferases: the key parameters.", January 2012 (2012-01), JOURNAL OF BIOMOLECULAR SCREENING JAN 2012 LNKD- PUBMED:21990582, VOL. 17, NR. 1, PAGE(S) 85 - 98, XP009161174, ISSN: 1552-454X the whole document

## Description

### Field of the invention

The invention is concerned with methods of assaying the activity of enzymes based on measurement of fluorescence lifetime (FLT). In particular, the invention relates to assays for enzymes which are capable of modifying the structure of peptide substrates, including for example enzymes catalysing methylation, demethylation, acetylation, deacetylation or deimination of peptide substrates.

### Background

Enzymes which catalyse structural modifications of other biological molecules, are important targets for drug discovery. For example, enzymes which catalyse post-translational modifications of proteins, in particular enzymes which modify the structure of histone proteins, are promising drug targets due to their relevance to human diseases such as neurodegeneration and cancer.

Post-translational modification of the amino acids that make up histone proteins is of particular importance in the field of "Epigenetics", which is the study of heritable changes in phenotype (appearance) or gene expression caused by mechanisms other than changes in the underlying DNA sequence. Genetic material requires protection, packaging, and methods for regulating processes such as transcription, replication and repair. In Eukaryotes, these roles are carried out in a large part by the histone proteins, which assemble the genomic DNA into nucleosomes. The nucleosomes, together with a number of associated proteins, are packaged to form the chromatin fibre. Each nucleosome consists of DNA wrapped almost two times around a cylindrical protein core containing two copies of each histone: H2A, H2B, H3 and H4. The N-terminal tails of the histone proteins protrude from the nucleosome core, where the structured, globular domains reside.

To enable their regulation of a diverse array of processes, the histone proteins are altered by a variety of post-translational modifications. Although histone modifications occur throughout the entire sequence, the unstructured N-termini of histones (the histone tails) are particularly highly modified. These modifications include acetylation, methylation, deimination, ubiquitylation, phosphorylation and sumoylation. Acetylation is the most highly studied of these modifications. For example, acetylation of the K14 and K9 lysines of the tail of histone H3 by histone acetyltransferase enzymes (HATs) is generally correlated with transcriptional competence.

Histone acetylation regulates many cellular processes, and specific acetylation patterns produce distinct outcomes, for example, the acetylation pattern on newly synthesised histones is important for their assembly into nucleosomes by histone chaperones. Additionally, the degree of chromatin compaction and folding may be regulated by acetylation of histone H4 at lysine 16. Finally, histone acetylation is critical for gene transcription. Lysine acetylation of histones is generally related to open chromatin structures with higher rates of transcription.

Histone acetylation is regulated by the activities of histone acetyltransferases (HATs) and deacetylases, with differing preferences for the various histone proteins and for specific sites on individual histones (see Shahbazian and Grunstein (2007) Annual review of Biochemistry, Vol. 76: 75-100).

In addition to acetylation, methylation of histones, in particular H3 and H4, is also important for the regulation of chromatin functions, such as gene expression, DNA replication and chromosome segregation. Methylation of histone H3 is generally associated with chromatin condensation, leading to transcriptional repression.

Histones are methylated on arginine and lysine residues only. Arginine can either be mono- or di-methylated, with the latter in symmetric or asymmetric configurations. The enzymes that catalyse this process have been divided into two types with the type I enzyme catalysing the formation of N^{G}-mono-methyl arginine and asymmetric N^{G},N^{G}-dimethyl arginine residues, whereas the type II enzyme catalyses the formation of N^{G}-mono-methyl arginine and symmetric N^{G},N^{G}-dimethyl arginine residues. Similar to arginine methylation, lysine methylation on the ε-nitrogen can also occur as mono- di- or tri-methylated forms.

The histone lysine methyltransferases (PKMTs) are responsible for methylation of histones H3 and H4 and utilize a catalytically active site called the SET domain (Supressor of variegation, Enhancer of zeste, Trithorax). The SET domain is a 130-amino acid sequence involved in modulating gene activities. This domain has been demonstrated to bind to the histone tail and causes the methylation of the histone. Histones H3 and H4 can also be manipulated through demethylation using histone lysine demethylase (PKDM). One family of this recently identified enzyme class has a catalytically active site called the Jumonji domain (JmjC). The demethylation occurs when JmjC utilizes multiple cofactors to hydroxylate the methyl group, thereby removing it. JmjC is capable of demethylating mono-, di-, and tri-methylated substrates.

There is a growing body of evidence that associates the activity of both histone methyltransferases (HMTs) and acetyltransferases (HATs) with different pathologies, from malignancies to neuropathies. Thus, the identification of inhibitors of these enzymes may provide a starting point to drug discovery.

The peptidyl arginine deiminases (PADs) are a family of calcium-dependent enzymes that post-translationally convert arginine residues on substrate proteins to the non-standard amino acid citrulline. PAD-catalysed citrullination, with concomitant loss of the positive imine group, converts the strongly basic arginine residue to a neutral amino acid. Loss of basic charge caused by citrullination is thought to disrupt charge distribution within the substrate protein and alter its ability to interact with other molecules. Deimination of histone proteins, H2A, H3 and H4, by enzymes of the PAD class, antagonises arginine methylation. In particular, the PAD4 isoform is capable of catalysing the citrullination of arginine residues on histones H2A, H3, and H4 (Thompson, P.R. and Fast, W., ACS Chem Biol.2006, 1, 433-441). Hence, the PAD group of enzymes may have a key role to play in the modification of the 'histone code' and thereby affect gene transcription.

The PAD enzymes and citrullinated proteins are also associated with multiple human diseases including rheumatoid arthritis, multiple sclerosis, Alzheimer's disease and, more recently, with cancer (Vossaar, E.R. et al, Citrullination of synovial proteins in mouse models of rheumatoid arthritis (2003). Arthritis Rheum, Vol. 48: 2489-2500; Musse, A.A. et al., Peptidyl arginine deiminase 2 (PAD2) over expression in transgenic mice leads to myelin loss in the central nervous system (2008), Dis Model Mech. Vol. 1: 229-240; Ishigami,A. et al., Abnormal accumulation of citrullinated proteins catalysed by peptidyl arginine deiminase in hippocampal extracts from patients with Alzheimer's disease (2005) J Neurosci Res. Vol 80; 120-128; and Chang X. et al. Expression of peptidyl arginine deiminase type 4 (PAD4) in various tumours (2006) Mol Carinog. Vol. 45: 183-196).

Accordingly, there is an ongoing need for robust and sensitive assays for such enzymes which modify the structure of other biological molecules, particularly the classes of enzymes which catalyse modifications of histone proteins as summarised above. In particular there is an ongoing need for robust, sensitive assays for such enzymes which can be readily applied in a high-throughput screening format, enabling efficient screening for enzyme inhibitors.

A variety of radioactive-based enzymatic assays have been reported for identifying compounds that modulate the activity of histone-modifying enzymes. For example, assays have been developed for monitoring the activity of histone methyl transferases by measuring the transfer of the methyl group from the regular labelled cofactor, S-adenosyl-L-methionine (SAM) to the ε-amino residue of lysines on histone-derived peptide substrates. However, as histone methyltransferases can perform either mono- di- or tri-methylation of lysines, measuring the incorporation of radioactive methyl groups does not provide information of the nature of the final reaction products. There are also significant hazards associated with the use of radioactive assays and subsequent disposal of radionuclides.

Mass spectroscopy (MS) has also been employed for studying the activity of histone methyl transferases and histone acetyltransferases. The use of MS allows the simultaneous characterisation of the different methyl-lysine forms produced by certain histone methyl transferases, i.e. mono-, di-, or tri-methylated lysine. However, this technology is not amenable to high throughput screening applications.

Non-radioactive assays for histone acetylation or methylation have been developed based on *in vitro* enzymatic modification of histone H3 derived substrates. In such assays, detection of the methylated reaction products is carried out using a labelled antibody. Examples of such assays are the Lance *Ultra*™ assays and AlphaLISA™ assays developed by PerkinElmer™.

WO 2007/076302 describes a high throughput assay for modulators of peptidyl arginine deiminase activity. The assay is based on fluorescence resonance energy transfer (FRET).

WO 2007/049057 relates to fluorescent dyes based on acridine derivatives and use of such dyes, for example, in biochemical and/or cell based assays.

Wigle T. et al. (Chem Biol. 2010 Jul 30;17(7):695-704) developed a quantitative microfluidic capillary electrophoresis assay to enable mechanistic studies of enzymes and the kinetics of their inhibition employing a methylation-sensitive endoproteinase strategy.

Maltman B. et a/. (Chem Commun (Camb). 2010 Oct 7;46(37):6929-31) describe a fluorescence reporter based on 9-aminoacridine, the lifetime of which can be modulated when in proximity to a tryptophan residue enabling fluorescence lifetime based biochemical assays.

### Summary of the invention

The present invention provides assays for the activity of enzymes, in particular enzymes which are capable of modifying the structure of peptide substrates, including for example enzymes catalysing methylation, demethylation, acetylation, deacetylation or deimination of peptide substrates, based on measurement of fluorescence lifetime (FLT).

In a first aspect the invention provides a method of assaying the activity of a modifying enzyme in a test sample, comprising:
(a) contacting the test sample with a fluorescent-modulated enzyme substrate comprising a linker molecule conjugated to a fluorescent moiety and a fluorescence lifetime modulator moiety configured to modulate the fluorescence lifetime of the fluorescent moiety, wherein the substrate is modified by the action of the modifying enzyme to form a modified substrate, the linker molecule of said modified substrate either being rendered susceptible to cleavage by a second enzyme or protected from cleavage by a second enzyme between the fluorescent moiety and the fluorescence lifetime modulator moiety as a result of the modification, wherein cleavage of the substrate or the modified substrate by the second enzyme separates a portion of the substrate containing the fluorescence lifetime modulator moiety from a portion of the substrate containing the fluorescent moiety; and
(b) detecting formation of the modified substrate by detecting changes in the fluorescence lifetime of the fluorescent moiety as a result of the action of the second enzyme on the modified substrate and/or the substrate, wherein formation of the modified substrate provides an indication of the activity of the modifying enzyme.

In a second aspect the invention provides a method of screening for inhibitors of an enzyme, the method comprising assaying the activity of said enzyme using a method according to the first aspect in the presence of a test compound, wherein a reduction in enzyme activity in the presence of the test compound identifies the test compound as an inhibitor of said enzyme.

In a third aspect the invention provides a fluorescent-modulated enzyme substrate comprising a linker molecule conjugated to a fluorescent moiety and a fluorescence lifetime modulator moiety configured to modulate the fluorescence lifetime of the fluorescent moiety, wherein the substrate is modified by the action of a modifying enzyme to form a modified substrate, the linker molecule of said modified substrate either being rendered susceptible to cleavage by a second enzyme or protected from cleavage by a second enzyme between the fluorescent moiety and the fluorescence lifetime modulator moiety as a result of the modification, wherein cleavage of the substrate or the modified substrate by the second enzyme separates a portion of the substrate containing the fluorescence lifetime modulator moiety from a portion of the substrate containing the fluorescent moiety, wherein the substrate has the structure 9AA-TARK⁹STGW-CONH₂.

In a fourth aspect the invention provides a kit for use in assaying the activity of a methyl transferase, the kit comprising a fluorescent-modulated enzyme substrate according to the third aspect and a protease which cleaves said fluorescent-modulated enzyme substrate between the fluorescent moiety and the fluorescence lifetime modulator moiety.

### Brief description of the drawings

Figure 1. Schematically illustrates an example of a fluorescence lifetime method for assaying deiminase class of enzymes. This specific example of the method is based on the use of 9-aminoacridine as the fluorescent moiety and the indolyl side chain of a tryptophan residue as the fluorescence lifetime modulator.
Figure 2. Shows fluorescent-modulated peptide substrates synthesised for assaying PAD4 deiminase activity.
Figure 3. Shows fluorescence lifetime measurements of peptides **1-3** in PAD4 assay buffer over time.
Figure 4. Illustrates trypsin cleavage of peptides **1-3** and their citrullinated analogues. Each peptide was incubated with varying concentrations of trypsin (shown in the legend) and fluorescence lifetime monitored over time: (A) peptide **1;** (B) peptide **2;** (C) peptide **3.** (D) fluorescence lifetime of citrullinated versions of peptides **1-3** after incubation with 5 nM trypsin. Citrullinated analogues comprise the peptide sequences 1-3 with the Arginine substituted with Citrulline.
Figure 5. RP-HPLC spectra of 1:1 mixtures of PAD4 peptide substrate and corresponding citrullinated product in assay buffer: (A) peptide **1;** (B) peptide **2;** (C) peptide **3.** NB: Arg = arginine containing peptide; Cit = citrulline containing peptide.
Figure 6. RP-HPLC spectra of samples taken at regular intervals from a PAD4 assay mixture containing peptide **1** as the substrate: (A) t = 0 min; (B) t = 30 min; (C) t = 60 min.
Figure 7. RP-HPLC spectra of samples taken at regular intervals from a PAD4 assay mixture containing peptide **2** as the substrate: (A) t = 0 min; (B) t = 30 min; (C) t = 60 min.
Figure 8. RP-HPLC spectra of samples taken at regular intervals from a PAD4 assay mixture containing peptide **3** as the substrate: (A) t = 0 min; (B) t = 30 min; (C) t = 60 min.
Figure 9. Electrospray mass spectra of the major 9AA-labelled peptide peak from RP-HPLC analysis of the PAD4 assay with peptide 1 as substrate. (A) t = 0 min, t_{R} = 14.3 min; (B) t = 60 min, t_{R} = 14.6 min.
Figure 10. Electrospray mass spectra of the major 9AA-labelled peptide peak from RP-HPLC analysis of the PAD4 assay with peptide **3** as substrate. (A) t = 0 min, t_{R} = 13.8 min; (B) t = 60 min, t_{R} = 13.9 min.
Figure 11. Comparison of fluorescence lifetimes of PAD4 assay mixtures before and after incubation with trypsin. No enzyme controls (minus PAD4) were included for comparison.
Figure 12. PAD4 assay time-course with peptide 1 as substrate. (A) fluorescence lifetime before addition of 10 nM trypsin; (B) fluorescence lifetime after incubation with 10 nM trypsin for 10 min at room temperature.
Figure 13. Schematically illustrates examples of a fluorescence lifetime method for assaying protein lysine methyltransferases (PKMT) and protein arginine methyltransferases (PRMT). These examples of the methods are based on the use of 9-aminoacridine as the fluorescent moiety and the indolyl side chain of a tryptophan residue as the fluorescence lifetime modulator.
Figure 14. Shows fluorescent-modulated peptide substrates synthesised for assaying G9a methyltransferase activity.
Figure 15. Evaluation of peptides **4-9** as substrates in a G9a fluorescence lifetime-based protease protection assay.
Figure 16. G9a fluorescence lifetime (FLT) protease protection assay with peptides **4** and **9** as substrates. (A) FLTs as a function of G9a concentration before the addition of Endo-Lys C protease. (B) FLTs as a function of G9a concentration after incubation with Endo-LysC for 10 min. (C) FLTs as a function of G9a concentration after incubation with Endo-LysC for 60 min. (D) and (E) As for (B) and (C) but with X-axis expanded for clarity and uncleaved peptide control included to demonstrate extent of protease protection.
Figure 17. RP-HPLC spectra of samples taken at the following intervals from a G9a assay mixture containing peptide **4** as the substrate: (A) t = 0 min; (B) t = 6 h.
Figure 18. RP-HPLC spectra of samples taken at the following intervals from a G9a assay mixture containing peptide **9** as the substrate: (A) t = 0 min; (B) t = 6 h.
Figure 19. Electrospray mass spectra of the 9AA-labelled peptide peak from RP-HPLC analysis of the G9a assay with peptide **4** as substrate. (A) t = 0 min, t_{R} = 21.4 min; (B) t = 6 h, t_{R} = 24.3 min.
Figure 20. Electrospray mass spectra of the 9AA-labelled peptide peak from RP-HPLC analysis of the G9a assay with peptide **9** as substrate. (A) t = 0 min, t_{R} = 19.6 min; (B) t = 6 h, t_{R} = 19.5 min.
Figure 21. Assay time-courses using peptide **4** as substrate and varying concentrations of G9a. (A) Before cleavage with 2 nM Endo-LysC; (B) After cleavage with 2 nM Endo-LysC.
Figure 22. Assay time-courses using peptide **9** as substrate and varying concentrations of G9a. (A) Before cleavage with 2 nM Endo-LysC; (B) After cleavage with 2 nM Endo-LysC.
Figure 23. Average lifetime as a function of SAM concentration for FLT G9a protease protection assays conducted with peptides **4** and **9** as substrates.
Figure 24. Average lifetime as a function of inhibitor concentration for FLT G9a protease protection assays conducted with peptides **4** and **9** as substrates.
Figure 25. Schematically illustrates examples of a fluorescence lifetime method for assaying protein lysine demethylases (PKDM) and protein arginine demethylases (PRDM). These specific examples of the method are based on the use of 9-aminoacridine as the fluorescent moiety and the indolyl side chain of a tryptophan residue as the fluorescence lifetime modulator.
Figure 26. Shows fluorescence lifetime substrates based on H3 N-terminal tail sequence for assaying JHDM1A lysine demethylase which acts on lysine-36.
Figure 27. Schematically illustrates an example of a fluorescence lifetime method for assaying histone acetyltransferases. This specific example of the method is based on the use of 9-aminoacridine as the fluorescent moiety and the indolyl side chain of a tryptophan residue as the fluorescence lifetime modulator.
Figure 28. Shows a proposed fluorescent-modulated peptide substrate based on H3 N-terminal tail sequence for assaying HAT enzymes that acetylate lysine-14.
Figure 29. Schematically illustrates an example of a fluorescence lifetime method for assaying histone deacetylases. This specific example of the method is based on the use of 9-aminoacridine as the fluorescent moiety and the indolyl side chain of a tryptophan residue as the fluorescence lifetime modulator.
Figure 30. Shows a proposed fluorescent-modulated peptide substrate for assaying HDACs via a FLT protease protection approach. Xaa = any amino acid.
Figure 31. RP-HPLC spectra of samples taken at regular intervals from a PAD2 assay mixture containing peptide **1** as the substrate: (A) t = 0 min; (B) t = 30 min; (C) t = 120 min.
Figure 32. Electrospray mass spectra of the major 9AA-labelled peptide peak from RP-HPLC analysis of the PAD2 assay with peptide **1** as substrate. (A) t = 0 min, t_{R} = 14.4 min; (B) t = 120 min, t_{R} = 14.7 min.
Figure 33. Fluorescence lifetime measurements of PAD2 assay mixture following incubation with trypsin. Peptide only controls were included for comparison.
Figure 34. PAD2 assay time-course with peptide **1** as substrate.
Figure 35. Fluorescence lifetime measurements of JHDM1A assay mixture with peptide **10** as substrate following incubation with Endo-LysC. Peptide only controls were included for comparison.
Figure 36. Overlay of RP-HPLC spectra of samples taken at regular intervals from a Set7/9 assay mixture containing peptide **12** as the substrate: (a) t = 0 h; (b) t = 2 h; (c) t = 6 h.
Figure 37. Electrospray mass spectra of the major 9AA-labelled peptide peak from RP-HPLC analysis of the Set7/9 assay with peptide **12** as substrate. (A) t = 0 h, t_{R} = 18.3 min; (B) t = 6 h, t_{R} = 18.4 min.
Figure 38. Fluorescence lifetime measurements of Set7/9 assay mixture following incubation with Endo-LysC. Peptide only controls were included for comparison.
Figure 39. Assay time-courses using peptide **12** as substrate and varying concentrations of Set7/9. (A) Before cleavage with 2 nM Endo-LysC; (B) After cleavage with 2 nM Endo-LysC.
Figure 40. Assay time-courses using peptide **13** as substrate and varying concentrations of Set7/9. (A) Before cleavage with 2 nM Endo-LysC; (B) After cleavage with 2 nM Endo-LysC.
Figure 41. Average lifetime as a function of SAM concentration for FLT Set7/9 protease protection assay conducted with peptide **13** as substrate.
Figure 42. Average lifetime as a function of SAH inhibitor concentration for FLT Set7/9 protease protection assay conducted with peptide **13** as substrate.
Figure 43. Z'-factor for FLT Set7/9 protease protection assay conducted with peptide **13** as substrate.
Figure 44. Shows fluorescent-modulated peptide substrates synthesised for assaying Set7/9 methyltransferase activity.
Figure 45. Average lifetime against time for the cleavage of peptide 15 by varying concentrations of Endo-ArgC.
Figure 46. Overlay of RP-HPLC spectra of samples taken at regular intervals from a PRMT5 assay mixture containing peptide **15** as the substrate: (a) t = 0 h; (b) t = 3.5 h; (c) t = 17.5 h.
Figure 47. Electrospray mass spectra of the major 9AA-labelled peptide peak from RP-HPLC analysis of the PRMT5 assay with peptide **15** as substrate. (A) t = 0 h, t_{R} = 20.0 min; (B) t = 17.5 h, t_{R} = 20.3 min.
Figure 48. Fluorescence lifetime measurements of PRMT5 assay mixture following incubation with Endo-ArgC. Peptide only controls were included for comparison.
Figure 49. Assay time-courses using peptide **15** as substrate and varying concentrations of PRMT5. (A) Before cleavage with 1.25 nM Endo-ArgC; (B) After cleavage with 1.25 nM Endo-ArgC.
Figure 50. Shows fluorescent-modulated peptide substrates synthesised for assaying PRMT5 methyltransferase activity.
Figure 51. Average lifetime as a function of sinefungin inhibitor concentration for PRMT5 FLT protease protection assay conducted with peptide **15** as substrate.
Figure 52. Average lifetime against time for the cleavage of peptide **16** with Endo-LysC.
Figure 53. Shows fluorescent modulated peptide substrate synthesised for assaying PCAF acetyltransferase activity.
Figure 54. Average lifetime against time for the cleavage of peptide **17** with Endo-LysC.
Figure 55. RP-HPLC spectra of samples taken at (A) 0 min, (B) 30 min, (C) 60 min, (D) 90 min intervals from a pCAF assay mixture containing peptide **17** as the substrate.
Figure 56. Electrospray mass spectra of peaks from RP-HPLC analysis of a pCAF assay with peptide **17** as the substrate. (A).t_{R} = 16.1 min; (B) t_{R} = 16.7 min.
Figure 57. Assay time-courses using peptide **17** as substrate and varying concentrations of PCAF. (A) Before cleavage with 25 nM Endo-LysC; (B) After cleavage with 25 nM Endo-LysC.
Figure 58. Determination of *K*_{M} (app) for AcCoA for PCAF FLT protease protection assay using peptide **17** as the substrate.
Figure 59. Shows fluorescent-modulated peptide substrate synthesised for assaying HDAC1 deacetylase activity.
Figure 60. Deacetylated product peptide.
Figure 61. Average lifetime against time for the cleavage of peptide **18** (A) and peptide **19** (B) with varying concentrations of trypsin.
Figure 62. Overlay of RP-HPLC spectra of samples taken at regular intervals from a HDAC1 assay mixture containing peptide **18** as the substrate.
Figure 63. Electrospray mass spectra of peaks from RP-HPLC analysis of a HDAC1 assay with peptide **18** after 2 h incubation at room temperature. (A) t_{R} = 19.1 min; (B) t_{R} = 21.2 min.
Figure 64. Assay time-courses using peptide **18** as substrate and varying concentrations of HDAC1. (A) Before cleavage with 10 nM trypsin; (B) After cleavage with 10 nM trypsin.
Figure 65. Determination of substrate *K*_{M} for HDAC1 FLT protease protection assay using peptide **18** as the substrate.
Figure 66. Average lifetime as a function of TSA inhibitor concentration for HDAC1 FLT protease protection assay conducted with peptide **18** as substrate.
Figure 67. Z'-factor for HDAC1 FLT protease protection assay conducted with peptide **18** as substrate.
Figure 68. Shows fluorescent-modulated peptide substrate synthesised for assaying EZH2 methyltransferase activity.
Figure 69. Average lifetime against time for the cleavage of peptide 20 with varying concentrations of Endo-LysC.
Figure 70. Assay time-courses using peptide **20** as substrate and varying concentrations of EZH2 complex.
Figure 71. Determination of *K*_{M} (app) for SAM for EZH2 FLT protease protection assay using peptide **20** as the substrate.
Figure 72. Average lifetime as a function of SAH inhibitor concentration for EZH2 FLT protease protection assay conducted with peptide **20** as substrate.
Figure 73. Determination of substrate K_{M} for peptide **1** in a PAD4 FLT protease protection assay.
Figure 74. Z'-factor for PAD4 FLT protease protection assay conducted with peptide **1** as substrate.
Figure 75. Z'-factor for G9a FLT protease protection assay conducted with peptide **9** as substrate.

### Detailed description of the invention

There is provided herein a method for assaying the activity of an enzyme, referred to herein as a modifying enzyme, based on detecting changes in fluorescence lifetime of a fluorescent moiety attached to a substrate for the modifying enzyme.

The method involves first contacting a test sample known to contain or suspected of containing the modifying enzyme with a substrate which is modified by the action of the modifying enzyme to form a modified substrate. The substrate, and the modified substrate formed by action of the modifying enzyme on the substrate, differ in structure such that one or other but not both of the substrate and the modified substrate can be cleaved by the action of a second enzyme at a cleavage site located *between* the fluorescent moiety and the lifetime modulator moiety, whilst the other is protected from cleavage by the second enzyme at any location *between* the fluorescent moiety and the fluorescence lifetime modulator moiety. Formation of the modified substrate by the action of the modifying enzyme on the substrate can thus be determined by assaying the action of the second enzyme on the modified substrate and/or the substrate from which it was formed.

As used herein the term "test sample" simply refers to any sample to be tested for activity of the modifying enzyme. This could, for example, be a pure or semi-pure sample of the enzyme in a suitable liquid medium, e.g. enzyme buffer, or it could refer to a biological sample to be tested for activity of the enzyme, e.g. a clinical sample of patient material.

The substrate, and the modified substrate formed by action of the modifying enzyme on the substrate, is conjugated to a fluorescent moiety and a fluorescence lifetime modulator moiety configured to modulate the fluorescence lifetime of the fluorescent moiety. Cleavage of either the substrate, or the modified substrate, by a second enzyme at a cleavage site *between* the site of attachment of the fluorescent moiety and the site of attachment of the fluorescence lifetime modulator moiety separates the substrate into at least two parts; including a first portion containing the fluorescent moiety and a second portion containing the fluorescence lifetime modulator moiety, such that the modulator moiety no longer modulates the fluorescence lifetime of the fluorescent moiety. The final read-out of the assay is thus based on measurement of the fluorescence lifetime of the fluorescent moiety, providing an indication of the action of the second enzyme on the modified substrate (or the substrate), which in turn provides an indication of the formation of the modified substrate by the action of the modifying enzyme on the substrate.

The term "modifying enzyme" refers to any enzyme which is capable of modifying a substrate to form a modified substrate, which differs in structure such that one or other but not both of the substrate and the modified substrate can be cleaved by the action of a second enzyme at a cleavage site located *between* the fluorescent moiety and the lifetime modulator moiety, whilst the other is protected from cleavage by the second enzyme at any location *between* the fluorescent moiety and the fluorescence lifetime modulator moiety. The modifying enzyme will typically be an enzyme which is capable of modifying the structure of a peptide or protein, e.g. an enzyme which catalyses a post-translational modification of a protein or peptide. In particular embodiments, described in detail herein, the modifying enzyme may be selected from the group consisting of protein methyl transferases, protein acetyltransferases, deiminases, protein demethylases and protein deacetylases. More specifically, the assay methodology described herein in general terms may be used to assay activity of enzymes which catalyse post-translational modification of histone proteins, such enzymes being important targets in the field of epigenetics. Therefore, in specific embodiments the modifying enzyme may belong to one of the following enzyme classes: histone-lysine *N*-methyttransferases (PKMTs), histone-arginine *N*-methyltransferases (PRMTs), histone acetyltransferases (HATs), histone demethylases, in particular the histone-lysine demethylases (KDMs) and histone-arginine demethylases, histone deacetylases (HDACs) and peptidyl arginine deiminases (PADs).

The substrate comprises a linker molecule conjugated to both a fluorescent moiety and a fluorescence lifetime modulator moiety. The fluorescent moiety and the fluorescence lifetime modulator moiety are configured such that the fluorescence lifetime modulator moiety can modulate the fluorescence lifetime of the fluorescent moiety. The substrate may therefore be referred to herein as a "fluorescent-modulated substrate" or "fluorescent-modulated enzyme substrate", these terms being used interchangeably.

As used herein the term "fluorescent moiety" refers to a fluorescent label conjugated to the fluorescent-modulated enzyme substrate.

Since the assays described herein use a read-out based on changes in fluorescence lifetime of the fluorescent moiety, it may be advantageous to select a fluorescent moiety which exhibits a long fluorescence lifetime (in the absence of any lifetime modulation), typically greater than 10ns and preferably within the range of from 10ns to 25ns, in order to increase the dynamic range of the assay. In this context, "fluorescence lifetime" is defined as the average time taken for the fluorophore to decay from the excited state to the ground state. However, the use of fluorescent moieties which exhibit a shorter fluorescence lifetime is also expressly encompassed within the scope of the invention.

Preferred fluorescent moieties include, but are not limited to, 9-aminoacridine and derivatives thereof, as described in WO 2007/049057. acridone derivatives and quinacridone derivatives, including the acridone and quinacridone compounds and other fluorescent moieties described in US 7,727,739 B2, WO 02/099432 and WO 03/089663 to which the skilled reader is directed, acridine and acridinium moieties, the Puretime™ series of dyes available from AssayMetrics Limited, oxazine (e.g. MR 121, JA 242, JA 243) and rhodamine derivatives (e.g. JA 165, JA 167, JA 169) as described in WO 02/081509,

Other examples (as described in WO 02/056670) include, but are not limited to Cy5, Cy5.5 and Cy7 (Amersham); IRD41 and IRD700 (Licor); NIR-1 and IC5-OSu (Dojindo); Alexa fluor 660 & Alexa fluor 680 (Molecular Probes); LaJolla Blue (Diatron); FAR-Blue, FAR-Green One & FAR-Green Two (Innosense); ADS 790-NS and ADS 821-NS (American Dye Source); indocyanine green (ICG) and its analogs (U.S. Pat. No. 5,968,479); indotricarbocyanine (ITC, WO 98/47538); fluorescent quantum dots (zinc sulfide-capped cadmium selenide nanocrystals-QuantumDot Corp.) and chelated lanthanide compounds (fluorescent lanthanide metals include europium and terbium). This list is intended to be illustrative rather than limiting.

As used herein the term "fluorescence lifetime modulator" means a compound, conjugate or moiety which is capable of changing the fluorescence lifetime of a fluorescent moiety when both are present in a fluorescent-modulated enzyme substrate. The mechanism of such fluorescence modulation could be, but is not limited to, energy transfer, electron transfer or molecular interactions, or combinations thereof. Preferred fluorescence lifetime modulators include, but are not limited to, indolyl moieties and derivatives thereof, including the indolyl moiety present in the side-chain of the amino acid tryptophan, phenolic moieties and derivatives thereof, including the phenolic moiety present in the side-chain of the amino acid tyrosine, imidazole moieties and derivatives thereof, including the imidazole moiety present in the side-chain of the amino acid histidine, benzyl moieties and derivatives thereof, including the benzyl side-chain of the amino acid phenylalanine, phenoxy moieties, naphthyl moieties and derivatives thereof, naphthylalanine moieties, carbazole moieties and phenothiazine moieties. This list is intended to be illustrative rather than limiting.

The fluorescent moiety and the fluorescence lifetime modulating moiety are selected such that the fluorescence lifetime of the fluorescent moiety is reduced in the presence of the fluorescence lifetime modulator, compared to when the fluorescence lifetime modulator is absent. Preferably the fluorescence lifetime of the fluorescent moiety is changed, and more preferably it is reduced, by at least 0.1 ns, by at least 0.5 ns, more preferably by at least 2ns, more preferably still by at least 5ns, or by at least 10ns in the presence of a fluorescence lifetime modulator, compared to when the fluorescence lifetime modulator is absent. The modulation effect is dependent on proximity and spatial orientation of the fluorescent moiety and the modulator within the fluorescent-modulated enzyme substrate. There is no explicit requirement for the absorption spectra of the fluorescence lifetime modulator to overlap with the emission spectrum of the fluorescent moiety. Fluorescence resonance energy transfer (FRET) processes, in contrast, require not only proximity between the donor and acceptor moieties but also spectral overlap between the absorption spectrum of the acceptor moiety and the emission spectrum of the donor moiety.

A significant modulation effect (e.g. a change in fluorescence lifetime of the fluorescent moiety of 5ns or greater) is an important determinant of the dynamic range of the enzyme assay. In this regard, the degree of modulation, i.e. the magnitude of the change in fluorescence lifetime of the fluorescent moiety when the modulator is present, may be more important that the absolute length of the fluorescence lifetime of the fluorescent moiety. Hence, if a combination of fluorescent moiety/modulator is selected such that the modulator moiety shortens the fluorescence lifetime of the fluorescent moiety to a significant extent, e.g. at least 2ns difference as a result of the modulation, then the absolute length of the fluorescence lifetime of the fluorescent moiety may not be critical. Therefore, fluorescent moieties which exhibit a relatively "short" fluorescence lifetime in the absence of modulation (e.g. less than 10ns, or less than 5ns) may still be utilised in the fluorescent-modulated substrate if the chosen modulator causes a significant reduction in this fluorescence lifetime (e.g. 2ns or more) when both are conjugated to the linker.

However, as the background interference in fluorescence assays arising from fluorescent compound libraries, autofluorescence or inner filter effects usually have short life-times in the region of 5 ns or less, the use of fluorophores with long fluorescence lifetimes (> 10 ns) may have advantages. Indeed, the ability to discriminate against such background and compound interference based on the lifetime of the fluorescence moiety is seen in the field as a key advantage of this technique as compared to fluorescence intensity based methods such as but not limited to FRET and fluorescence polarization. Fluorophores with long fluorescence lifetimes facilitate this discrimination from background fluorescence and compound interference as opposed to fluorophores with short fluorescence lifetimes, where discrimination is often not possible.

Suitable combinations of the fluorescent moiety and the fluorescence lifetime modulating moiety for inclusion into a fluorescent-modulated substrate are selected such that the fluorescence lifetime of the fluorescent moiety is reduced in the presence of the fluorescence lifetime modulator. When selecting an appropriate combinations, it is emphasised that there is no explicit requirement for the absorption spectra of the fluorescence lifetime modulator to overlap with the emission spectrum of the fluorescent moiety. The fluorescent-modulated substrate may, if appropriate for a particular enzyme assay, include more than one fluorescent moiety and/or more than one fluorescence lifetime modulator moiety

Preferred combinations of fluorescent moiety and fluorescence lifetime modulator moiety for inclusion into a fluorescent-modulated enzyme substrate include, but are not limited to, the fluorescent moiety 9-aminoacridine (9-AA) with a modulator moiety which is an indolyl moiety, in particular the indolyl side-chain of a tryptophan residue, the fluorescent moiety 9-aminoacridine (9-AA) with the modulator moiety which is naphthylalanine or a derivative thereof, the fluorescent moiety 9-aminoacridine (9-AA) with the modulator moiety which is a carbazole moiety or a derivative thereof and the fluorescent moiety 9-aminoacridine (9-AA) with the modulator moiety phenothiazine or a derivative thereof. Other suitable combinations include a fluorescent moiety which is acridone or a derivative thereof with a modulator moiety which is an indolyl moiety, in particular the indolyl side-chain of a tryptophan residue, and also a fluorescent moiety which is quinacridone or a derivative thereof with a modulator moiety which is an indolyl moiety, in particular the indolyl side-chain of a tryptophan residue. The fluorescence lifetime of acridone-based fluorophores has been shown to be modulated by the indolyl side-chain of a tryptophan residue (WO 03/089663; Hassiepien, U et al. Screening. Vol. 4, 2009, 11; Doering, K. et al. J Biomol. Screen., 2009, 14, 1). Any of the listed combinations may be included in any of the fluorescent-modulated enzyme substrates described herein, including the specific substrates defined below on the basis of structure. For the avoidance of doubt, it is explicitly intended that the fluorescent moiety/modulator moiety combinations described in the preceding section can correspond to the moieties **FI** and **M** in the structural definitions used hereinafter.

The linker molecule component of the fluorescent-modulated enzyme substrate serves to position the fluorescent moiety and the fluorescence lifetime modulator moiety in the appropriate configuration to achieve an effective modulation of the fluorescence lifetime of the fluorescent moiety. The linker molecule is also the site of modification of the fluorescent-modulated enzyme substrate by the modifying enzyme to form the modified substrate.

In preferred embodiments the linker molecule component of the substrate is a peptide linker which can be modified by the action of the modifying enzyme to form a modified peptide. In embodiments wherein the linker molecule component of the substrate is a peptide linker, the modified form resulting from the action of the modifying enzyme may be referred to as a "modified peptide linker". The peptide linker may include at least one amino acid residue which is modified by the action of the modifying enzyme. As described in detail below, types of peptide modifications which may be catalysed by the modifying enzyme include, but are not limited to, methylation, demethylation, acetylation, deacetylation and deimination.

The amino acid residue within the peptide linker which is modified by the action of the modifying enzyme generally occurs in an amino acid sequence context which forms a recognition site for the second enzyme, which is typically a protease. The recognition site for the second enzyme is positioned such that cleavage occurs between the site of attachment of the fluorescent moiety to the peptide linker and the site of attachment of the fluorescence lifetime modulator moiety to the peptide linker, such that cleavage of the peptide linker at the cleavage site (in either the substrate or the modified substrate) separates the fluorescent-modulated enzyme substrate (or the modified substrate) into a first portion comprising the fluorescent moiety and a second portion comprising the fluorescence lifetime modulator moiety. As a consequence of this separation, the fluorescence lifetime modulator moiety no longer modulates the fluorescence lifetime of the fluorescent moiety, hence the fluorescence lifetime of the fluorescent moiety may be observed to increase.

A fluorescent-modulated enzyme substrate comprising a peptide linker will typically conform to the following general structure:
**FI**-Xₙ₁-N-Xₙ₂-**M** or structure **M**-Xₙ₁-N-Xₙ₂-FI in which the orientations of **FI** and **M** are reversed
wherein Xₙ₁ represents a first sequence of one or more amino acids, FI represents a fluorescent moiety which is conjugated to Xₙ₁ (or Xₙ₂ if the orientations of **FI** and **M** are reversed), N represents an amino acid residue which is modified by the action of the modifying enzyme, Xₙ₂ represents a second sequence of one or more amino acids and **M** represents a fluorescence lifetime modulator which is conjugated to Xₙ₂ (or Xₙ₁ if the orientations of **FI** and **M** are reversed). Following treatment with modifying enzyme, the substrate is converted into a modified substrate represented by the general structure:

**FI**-Xₙ₁-N_{mod}-Xₙ₂-**M** or **M**-Xₙ₁-N_{mod}-Xₙ₂-**FI**

wherein N_{mod} represents a modified derivative of residue N.

The fluorescent moiety **FI** is "conjugated to" Xₙ₁, whilst the fluorescence lifetime modulator moiety **M** is "conjugated to" Xₙ₂, or *vice versa* if the positions of **FI** and **M** relative to residue N are reversed. The essential requirement is that **FI** and **M** are separated upon cleavage of the substrate. Hence the term "conjugated to" means that **FI** can be joined to the peptide linker of the substrate within any part of the amino acid sequence represented as Xₙ₁ and that **M** can be joined to the peptide linker of the substrate within any part of the amino acid sequence represented as Xₙ₂ or *vice versa.* It is not intended to limit the substrate structure to embodiments wherein **FI** and **M** are joined to the C-terminal and N-terminal residues of the peptide linker, although such structures are encompassed. **FI** and **M** can also be joined to internal amino acid residues within Xₙ₁ or Xₙ₂. The fluorescence lifetime modulator **M** may also be provided by the side chain of an amino acid residue in Xₙ₂ (or Xₙ₁ when the positions are reversed), which may be an internal residue, or the C-terminal residue or the N-terminal residue of the peptide linker. For example, **M** may be provided by the indolyl, phenolic, imidazole and benzyl side-chains of tryptophan, tyrosine, histidine or phenylalanine residues incorporated into Xₙ₁ or Xₙ₂. This definition/interpretation of the positioning of FI and **M** is to be applied to all of the specific embodiments of the substrate described in detail below.

Xₙ₁ and Xₙ₂ are chosen such that the final structure Xₙ₁-N-Xₙ₂ provides an appropriate sequence context for modification of residue N by the modifying enzyme. If the activity of the modifying enzyme is highly sequence-dependent that it may be appropriate for the overall amino acid sequence of Xₙ₁**-**N**-**Xₙ₂ to mimic the amino acid sequence of a portion of a natural substrate for the modifying enzyme. For example, if the natural substrate for the modifying enzyme is a histone protein, then Xₙ₁-N-Xₙ₂ may mimic the sequence of a short peptide fragment of the histone protein surrounding the amino acid residue which is modified by the action of the modifying enzyme.

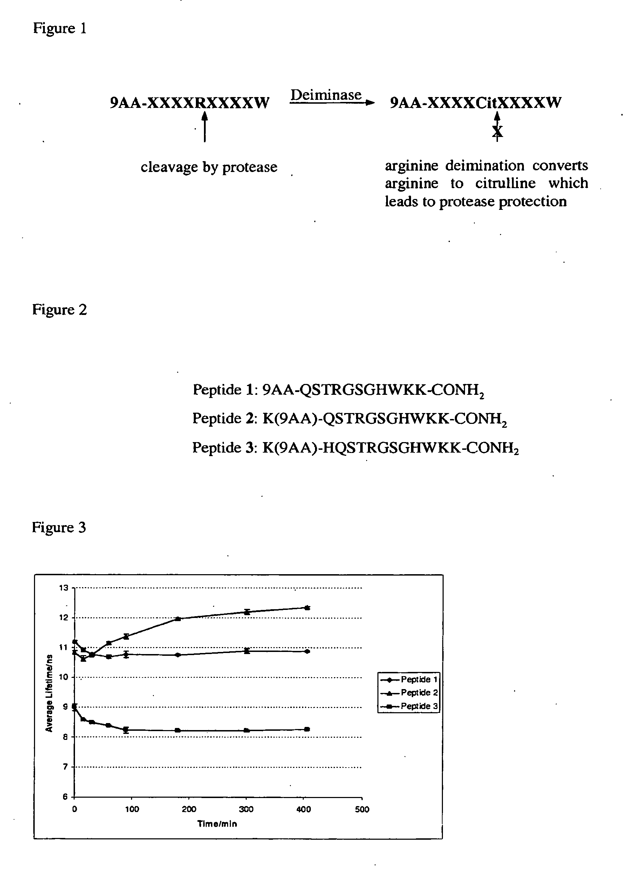

It is also important that either the sequence Xₙ₁-N-Xₙ₂, or the modified form Xₙ₁-N_{mod}-Xₙ₂, but not both, form a substrate for cleavage by the second enzyme *between* the fluorescent moiety and the fluorescence lifetime modulator moiety. In embodiments based on use of a peptide linker the second enzyme will generally be a protease. A wide range of protease enzymes having differing substrate specificities are available in the art. Hence, the protease can be selected to match the nature of the modification catalysed by the modifying enzyme. It is critical for correct operation of the assay that conversion of the linker from the form which is not cleaved by the second enzyme to the form which is cleaved by the second enzyme (or *vice versa)* is dependent solely on the activity of the modifying enzyme. Therefore, it is essential to ensure that the linker does not contain any sites permitting cleavage of the linker by the second enzyme *between* the fluorescent moiety and the fluorescence lifetime modulator moiety in a manner which is independent of the modification at residue N, to ensure that separation of **FI** and **M** via cleavage of the linker is critically dependent on modification of residue N. It is not excluded that the second enzyme might cleave at a second cleavage site elsewhere in the substrate or the modified substrate, with substrate cleavage at the second site not being dependent on modification of the substrate. Cleavage at a second site which is independent of substrate modification can be permitted provided that cleavage at this second site does not result in separation of the fluorescent moiety and the fluorescence lifetime modulator moiety. For example, the substrate shown in Figure 2 may be susceptible to trypsin cleavage at the lysine residues located between the tryptophan residue that contributes the fluorescence lifetime modulator moiety (the indolyl side chain of tryptophan) and the C-terminus of the peptide.

The amino acid residues represented by Xₙ₁-N-Xₙ₂ together constitute the peptide linker component of the substrate. The peptide linker may be formed primarily from amino acid residues linked by conventional peptide bonds. The amino acid residues themselves may be natural or non-natural amino acid residues or a mixture thereof. It is obviously a requirement that the amino acid residue represented by N can be modified by the action of the modifying enzyme, but modifications of other amino acid residues are also permitted, provided that the substrate is still susceptible to modification at residue N. It is also permissible to include one or more non-peptide linkages, provided that the peptide linker in either the substrate or the modified substrate remains cleavable by the second enzyme (e.g. protease).

It is also an important requirement that within the overall structure of the fluorescent-modulated enzyme substrate the fluorescent moiety **FI** and the fluorescence lifetime modulator moiety **M** are maintained in an appropriate spatial configuration to achieve effective modulation of the fluorescence lifetime of moiety **FI** by moiety **M.** Typically, both **FI** and **M** will be attached to the peptide linker (Xₙ₁**-**N**-**Xₙ₂) via a direct covalent attachment. Suitable methods for direct covalent attachment of fluorescent moieties to peptides are described in WO 2007/049057

Other methods of attaching fluorescent moieties to proteins and peptides are described in Bioconjugate Techniques, G. T. Hermanson, Academic Press (1996).

In embodiments wherein the fluorescence lifetime modulator **M** is provided by the indolyl, phenolic, imidazole and benzyl side-chains of tryptophan, tyrosine, histidine or phenylalanine residues incorporated into Xₙ₁ or Xₙ₂ then the amino acid residue that contributes the side-chain which acts as the modulator **M** may itself form part of the amino acid sequence represented by Xₙ₂ (or Xₙ₁ in embodiments where the position of **FL** and **M** are reversed). The amino acid residue which acts as the fluorescence lifetime modulator **M** may therefore be joined to the remainder of the peptide linker via a covalent peptide bond.

It is a particular advantage of the assay platform described herein that the amino acid sequence of the peptide linker represented by Xₙ₁-N-Xₙ₂ can be adapted for a range of different modifying enzymes of interest, yet the general principle of the assay, based on FLT detection, remains common to all the assays.

Conventional detection methods may be employed to measure fluorescence lifetime and fluorescence intensity. These methods include instruments using photomultiplier tubes as detection devices. Several approaches are possible using these methods; e.g.
i) methods based upon time correlated single photon counting (cf. Principles of Fluorescence Spectroscopy, (Chapter 4) ed. J R Lakowicz, Second Edition, 1999, Kluwer/Academic Press);
ii) methods based upon frequency domain/phase modulation (cf. Principles of Fluorescence Spectroscopy, (Chapter 5) ed. J R Lakowicz, Second Edition, 1999, Kluwer/Academic Press); and
iii) methods based upon time gating (cf. Sanders et al., (1995) Analytical Biochemistry, 227 (2), 302-308).

Suitable devices for measurement of fluorescence lifetime are the Edinburgh Instruments FLS920 fluorimeter, and the Edinburgh Instruments NanoTaurus™ Fluorescence Lifetime Plate Reader, (Edinburgh Instruments, UK) which employ time-correlated single photon counting methods, and also the IOM Nanoscan, which utilises time-gating.

Measurement of fluorescent intensity/fluorescence lifetime may be performed by means of a charge coupled device (CCD) imager, such as a scanning imager or an area imager, to image all of the wells of a multiwell plate.

The read-out of the assay method is reported by a change in fluorescence lifetime of the fluorescent moiety. In certain embodiments the assay may be based on measurement of the average fluorescence lifetime, which is a combination of the fluorescent lifetimes of all fluorescent species present, i.e. the fluorescent-modulated substrate, and the modified substrate formed by action of the modifying enzyme on the substrate, plus the fluorescent product produced when either the fluorescent-modulated substrate or the modified substrate is cleaved by the action of the second enzyme (e.g. protease).

It will be appreciated that the fluorescence lifetime of the fluorescent moiety within the modified substrate (formed by action of the modifying enzyme of the fluorescent-modulated substrate) is substantially the same length as the fluorescence lifetime of the fluorescent moiety within the fluorescent-modulated substrate from which it is derived, since the modification catalysed by the modifying enzyme (e.g. methylation, acetylation, demethylation, deacetylation, deimination) does not, in general, significantly alter the orientation of the fluorescent moiety and the fluorescence lifetime modulator moiety and hence the degree of fluorescence lifetime modulation. The fluorescence lifetime of the fluorescent moiety is modulated in both the fluorescent-modulated substrate and the modified substrate. A change in fluorescence lifetime is only detectable when cleavage of the fluorescent-modulated substrate or the modified substrate takes place, separating a portion of the substrate conjugated to the fluorescent moiety from a portion of the substrate conjugated to the fluorescence lifetime modulator, abolishing modulation. The cleaved portion of the substrate conjugated to the fluorescent moiety exhibits a longer fluorescent lifetime than the fluorescent-modulated substrate.

The *average* fluorescence lifetime measurement therefore varies depending on the relative proportions of fluorescent species exhibiting the "shortened" fluorescence lifetime due to modulation (fluorescent-modulated substrate plus modified substrate) and fluorescent species exhibiting the longer (unmodulated) fluorescence lifetime (the cleaved substrate portion conjugated to the fluorescent moiety). The relative proportion of "short" and "long" lifetime fluorescent species present after treatment with the second enzyme is directly related to the amount of "cleavable" fluorescent-modulated substrate (or modified substrate) present at the point at which the second enzyme is added. Hence, it is also directly related to activity of the modifying enzyme. When referring to fluorescent lifetime measurement, the terms "long" and "short" are relative terms, referring to the fluorescent lifetime of the same fluorescent moiety when unmodulated and when modulated by the presence of a fluorescence lifetime modulator, and do not imply particular absolute values.

In a time-course assay, the average fluorescence lifetime may be observed to increase or decrease over time relative to a t=0 measurement for 100% unmodified substrate, depending on whether the modified substrate is rendered susceptible to cleavage or protected from cleavage by the second enzyme (e.g. protease), as compared to the unmodified substrate. In an end point assay, the average lifetime measurement at the endpoint may be higher or lower than a baseline measurement corresponding to 100% unmodified substrate, depending on whether the modified substrate is rendered susceptible to cleavage or protected from cleavage by the second enzyme (e.g. protease), as compared to the unmodified substrate.

As an alternative to measurement of average lifetime, assays can also be based on measurement of the characteristic fluorescence lifetime of a specific component. For example, one can elect to measure only the amount of "long" fluorescence lifetime component present. Since the long fluorescence lifetime is characteristic of cleaved products in which the fluorescent moiety is separated from the fluorescence lifetime modulator, this measurement can be directly related to the amount of the cleaved substrate present, in concentration units. In a time-course assay, the absolute amount of the long lifetime characteristic component (i.e. cleaved product conjugated to the fluorescent moiety) present may be observed to increase or decrease over time relative to a t=0 measurement for 100% unmodified substrate, depending on whether the modified substrate is rendered susceptible to cleavage or protected from cleavage by the second enzyme (e.g. protease). In an end point assay, the absolute amount of the long lifetime characteristic component (i.e. cleaved product conjugated to the fluorescent moiety) present may be higher or lower than a baseline measurement corresponding to 100% unmodified substrate, depending on whether the modified substrate is rendered susceptible to cleavage or protected from cleavage by the second enzyme (e.g. protease), as compared to the unmodified substrate.

Fluorescence lifetime measurements offer significant advantages over conventional fluorescence techniques that are based solely on quantifying fluorescence intensity. Fluorescence lifetime is determined from the same spectrally resolved intensity signal, but is additionally resolved in the temporal domain. This intrinsic fluorescence property is independent of probe concentration and volume, and is unaffected by auto-fluorescence, light scattering and inner filter effects and photo-bleaching. Consequently, the inherent properties of this method should lead to more robust assays with better sensitivity and enable background interference from fluorescent compound libraries to be minimised, leading to fewer false positives in drug screening applications.

Non-limiting embodiments of the assay will now be described in further detail:

### (A) Assays based on protease protection

A first group of embodiments of the assay are based on use of a substrate comprising a peptide linker molecule conjugated to a fluorescent moiety and a fluorescence lifetime modulator moiety configured to modulate the fluorescence lifetime of the fluorescent moiety, the peptide being modified by the action of the modifying enzyme to form a modified peptide wherein the peptide in the substrate is capable of being cleaved by the second enzyme (i.e. a protease) and modification of the peptide by the modifying enzyme protects the modified peptide from cleavage by the second enzyme (e.a. protease) between the fluorescent moiety and the fluorescence lifetime modulator moiety. In such embodiments, the substrate is susceptible to cleavage by the second enzyme (protease) between the fluorescent moiety and the fluorescence lifetime modulator moiety, cleavage of the substrate producing an increase in the fluorescence lifetime of the fluorescent moiety, but the modified substrate formed by action of the modifying enzyme on the substrate is not cleaved by the second enzyme (protease) between the fluorescent moiety and the fluorescence lifetime modulator moiety. Formation of the modified peptide (by the action of the modifying enzyme on the substrate) is thus indicated by a time-dependent decrease in the fluorescence lifetime of the fluorescent moiety after protease treatment, as compared to unmodified peptide.

Non-limiting embodiments of this assay are as follows:

### (1) Methyl transferase assay

In one embodiment, the assay method described herein may be adapted to assay the activity of a methyltransferase enzyme.

The substrate for the methyltransferase assay comprises a peptide linker, and may have the general structure:

**FI**-X1-N1-X2-**M** (I)

wherein X1 represents a first sequence of amino acids, **FI** represents a fluorescent moiety which is conjugated to X1, N1 represents an amino acid residue which is methylated by the action of the methyl transferase, X2 represents a second sequence of amino acids and **M** represents a fluorescence lifetime modulator which is conjugated to X2. Following treatment with methyl transferase, the substrate is converted into a modified (methylated) substrate represented by the general structure:

**FI**-X1-N1(Me)-X2-**M** (II)

wherein N(Me) represents methylation on residue N.

It will be appreciated that **FI** and **M** may in fact be present in either orientation. Residue N1 is typically either lysine (in the case of assays for lysine methyltransferases) or arginine (in the case of assays for arginine methyltransferases).

Amino acid sequences X1 and X2 are selected both to place residue N1 in an appropriate sequence context for methylation, and to provide a recognition site for cleavage by a protease (in unmethylated structure (1)), and also to provide optimum orientation of the fluorescent moiety **(FI)** and the fluorescence lifetime modulator **(M)** to achieve effective modulation of the fluorescence lifetime of **FI.** The sequences of X1 and X2 may be based around the sequence of a native substrate for the methyl transferase enzyme being assayed.

It will be appreciated that although fluorescent moiety **(FI)** and the fluorescence lifetime modulator **(M)** need to positioned at an appropriate spacing for effective modulation of the fluorescence lifetime, it is not essential for either fluorescent moiety **(FI)** or fluorescence lifetime modulator **(M)** to occur at the end (N-terminus or C-terminus) of the peptide linker. Hence **FI** may be attached to an amino acid residue within X1 and **M** may be attached to an amino acid residue within X2, or *vice versa.* If the fluorescence lifetime modulator **M** is itself provided by the side-chain of an amino acid residue, e.g. tryptophan, tyrosine, phenylalanine, naphthylalanine or histidine or a derivative thereof, then the amino acid residue which acts as the modulator **M** may itself form part of the amino acid sequence represented by X2. Amino acid sequences X1 and X2 may contain naturally-occurring or non-natural amino acid residues, and other modifications such as non-peptide linkages.

In the case of assays based on methylation of arginine, the arginine residue represented by N can either be mono- or di-methylated, with the latter in symmetric or asymmetric configurations, depending on the activity of the arginine methyl transferase enzyme in question. The precise degree of methylation and the configuration of di-methylated forms is not material to the performance of the assay, hence the residue N(Me) may, in the case of arginine methylation, represent any of the mono-, di-methylated forms.

In the case of assays based on methylation of lysine, the lysine residue represented by N can either be mono-, di- or tri- methylated, depending on the activity of the lysine methyl transferase enzyme in question. The precise degree of methylation and the configuration of di-methylated or tri-methylated forms is not material to the performance of the assay, hence the residue N(Me) may, in the case of lysine methylation, represent any of the mono-, di- or tri-methylated forms.

In the substrate of structure (I), the fluorescence lifetime of fluorescent moiety **FI** (which is preferably 9-aminoacridine (9AA) but may be any other suitable fluorescent moiety) is modulated by the presence of a fluorescence lifetime modulator **M** in the substrate **(M** is preferably tryptophan but may be any other known modulator of fluorescence lifetime). Treatment of the substrate of structure (I) with a protease that cleaves adjacent to residue N1 (e.g. lysine or arginine) will induce proteolysis and remove modulation of the fluorescence lifetime by the modulator **M.** After methylation of residue N1 (e.g. lysine or arginine) by an appropriate methyl transferase enzyme, treatment of the modified peptide of structure (II) with the protease does not induce cleavage after residue N1 and the lifetime of the fluorescent moiety **(FI)** remains modulated. Where there is continual conversion of substrate (I) into modified substrate (II), as in a time-course assay, the fluorescence lifetime of the fluorescent moiety **(FI)** will decrease as a function of time (and concentration of methyl transferase enzyme). A shift in fluorescence lifetime (decrease) relative to a base-line measurement for 100% unmodified substrate (I) indicates that some conversion from substrate (I) to modified substrate (II) has occurred. The magnitude of this shift can thus be used to assess conversion from substrate (I) to modified substrate (II) due to the action of the methyltransferase.

Accordingly, there is provided herein a method of assaying the activity of a protein methyl transferase enzyme which comprises:
(a) contacting a test sample with a fluorescent-modulated enzyme substrate of the general formula (I)

   **FI -** X1-N1-X2- **M** (I)

   wherein X1 represents a first sequence of amino acids, **FI** represents a fluorescent moiety which is conjugated to X1, N1 represents an amino acid residue which is methylated by the action of the methyl transferase, X2 represents a second sequence of amino acids and **M** represents a fluorescence lifetime modulator which is conjugated to X2 under conditions which permit enzymatic conversion of the fluorescent-modulated enzyme substrate to a modified substrate of general formula (II)

   **FI** - X1-N1 (Me)-X2- **M** (II)

   wherein N(Me) represents methylation on residue N, by the action of the protein methyl transferase enzyme;
(b) contacting the products of step (a) with a protease enzyme which is capable of cleaving the fluorescent-modulated enzyme substrate of the general formula (I) adjacent to residue N1, but is not capable of cleaving the modified substrate of general formula (II) between **FI** and **M;** and
(c) detecting changes in the fluorescence lifetime of the fluorescent moiety **FI,** thereby assaying the activity of the protein methyl transferase enzyme.

This method can be readily applied to screen for inhibitors of the protein methyl transferase enzyme, by adding candidate compounds to be tested for inhibitor activity in step (a).

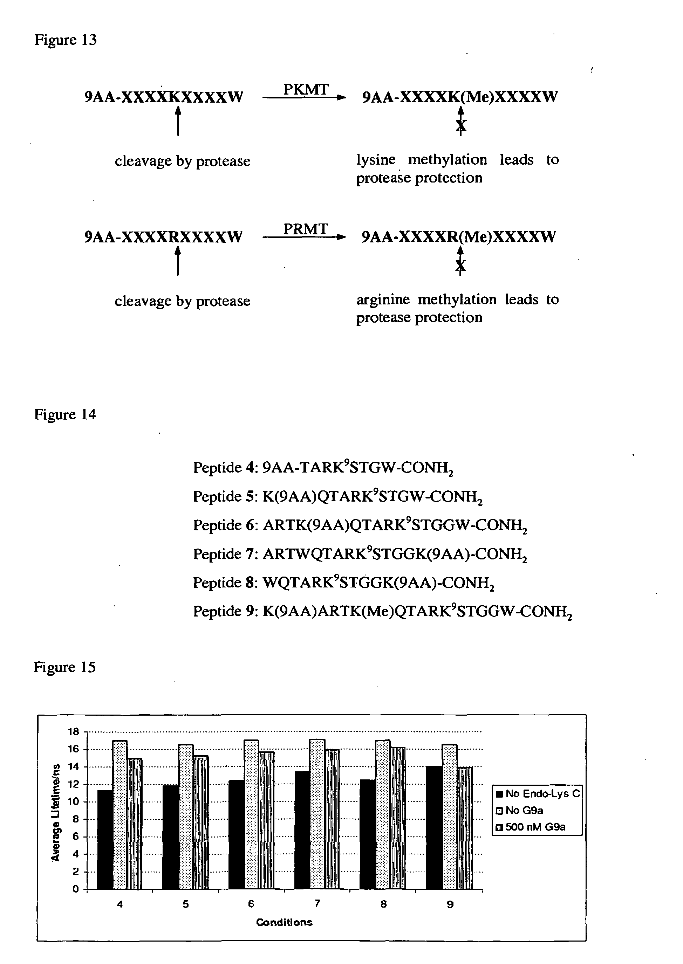

A specific example of a method of assaying protein methyltransferases (PMT), which transfer one or more methyl groups to lysine or arginine residues in peptide/protein substrates, via a fluorescence lifetime protease protection approach is shown schematically in accompanying Figure 13. This example is based upon the use of 9-aminoacridine as the fluorescent moiety and the indolyl side-chain of a tryptophan residue as the fluorescence lifetime modulator. However, it will be appreciated that these specific fluorescent moieties and fluorescence lifetime modulator moieties are shown by way of example only and can be replaced by alternative fluorescent moieties and fluorescence lifetime modulator moieties as described herein.

The general methodology described above can be adapted for use with any methyltransferase enzyme of interest, including both methyltransferases which methylate on lysine (collectively referred to as PKMT) and methyltransferases which methylate on arginine (collectively referred to as PRMT). In particular embodiments the assay can be used to determine the activity of histone methyl transferases which methylate histone proteins on either lysine or arginine. The method may be applied to any histone-lysine *N*-methyl transferase, including all histone-lysine *N*-methyl transferases belonging to the enzyme class EC 2.1.1.43. Specific PKMT enzymes which may be assayed using this method include, for example, G9a, Set7/9, GLP and EZH2.

Protein arginine methyltransferases (PRMTs) catalyse the transfer of methyl groups from S- adenosyl-L-methionine (SAM) to the guanidino nitrogens of arginine residues. PRMTs can be divided into two types on the basis of whether they catalyse symmetric or asymmetric dimethylation. The H3 specific arginine methyltransferase CARM1/PRMT4 belongs to the type 1 protein N-methyltransferase family. Suitable PRMT enzymes also include, for example, PRMT1, PRMT3, and PRMT5. The assay method may be used to determine the activity of any histone-arginine N-methyltransferase, including any histone-arginine *N*-methyltransferase belonging to the enzyme class EC 2.1.1.125.

Example substrates for use with the representative PKMT enzyme G9a based on use of 9-aminoacridine **(9AA)** as the fluorescent moiety and tryptophan **(W)** as the fluorescence lifetime modulator moiety include the following:
Peptide **4**: **9AA**-TARK⁹STG**W**-CONH₂
Peptide **5**: K(**9AA**)QTARK⁹STG**W**-CONH₂
Peptide **6**: ARTK(**9AA**)QTARK⁹STGG**W**-CONH₂
Peptide **7**: ART**W**QTAR**K**⁹STGGK(**9AA**)-CONH₂
Peptide **8**: **W**QTARK⁹STGGK(**9AA**)-CONH₂
Peptide **9**: K(**9AA**)ARTK(Me)QTARK⁹STGG**W**-CONH₂

K⁹ represents a lysine residue which is the site of methylation by the action of the PMT enzyme G9a and mimics the sequence context in which lysine methylation occurs within histone H3, which forms a natural substrate for G9a.

Example substrates for use with the representative PKMT enzyme Set 7/9 based on use of 9-aminoacridine **(9AA)** as the fluorescent moiety and tryptophan **(W)** as the fluorescence lifetime modulator moiety include the following:
Peptide **12: W**ARTK⁴QTARK(**9AA**)STGGKAPRKQLAK-CONH₂
Peptide **13**: **W**RTK⁴QTARK(**9AA**)STGGKAPRKQLAK-CONH₂

K⁴ represents a lysine residue which is the site of methylation by the action of the PKMT enzyme Set 7/9 and mimics the sequence context in which lysine methylation occurs within histone H3, which forms a natural substrate for Set 7/9.

Example substrates for use with the representative PRMT enzyme PRMT5 based on use of 9-aminoacridine **(9AA)** as the fluorescent moiety and tryptophan **(W)** as the fluorescence lifetime modulator moiety include the following:
Peptide **14: W**SGR³GKGGK(**9AA**)GLGKGGAKRHRK-CONH₂
Peptide **15:** Ac-**W**SGR³GKGGK(**9AA**)GLGKGGAKRHRK-CONH₂

R³ represents an arginine residue which is the site of methylation by the action of the PRMT enzyme PRMT5 and mimics the sequence context in which arginine methylation occurs within histone H4, which forms a natural substrate for PRMT5. Peptide **15** is acetylated at the N-terminus (Ac).

Example substrates for use with the representative PKMT enzyme EZH2 based on use of 9 aminoacridine **(9AA)** as the fluorescent moiety and tryptophan **(W)** as the fluorescence lifetime modulator moiety include the following:
Peptide **20:** PRKQLATK(9AA)AARK²⁷SAPATGGW-CONH₂

K²⁷ represents a lysine residue which is the site of methylation by the action of the PKMT enzyme EZH2 and mimics the sequence context in which lysine methylation occurs within histone H3, which forms a natural substrate for EZH2.

Peptide substrates suitable for use in assaying other PKMT and PRMT enzymes can be prepared following the general principles illustrated in the accompanying examples.

Suitable proteases for use in the general approach outlined above include, but are not limited to, Erido-LysC which cleaves after lysine (for assays based on methylation of lysine) and Endo-ArgC which cleaves after arginine (for assays based on methylation of arginine).

### (2) Acetyl transferase assay

In one embodiment, the assay method described herein may be adapted to assay the activity of an acetyltransferase enzyme.

The substrate for this assay comprises a peptide linker, and has the general structure (III):

**FI -** X3-N2-X4- **M** (III)

wherein X3 represents a first sequence of amino acids, **FI** represents a fluorescent moiety which is conjugated to X3, N2 represents an amino acid residue which is acetylated by the action of the acetyl transferase, X4 represents a second sequence of amino acids and **M** represents a fluorescence lifetime modulator which is conjugated to X4. Following treatment with acetyl transferase, the substrate is converted into a modified (acetylated) substrate represented by the general structure:

**FI -** X3-N2(Ac)-X4- **M** (IV)

wherein N2(Ac) represents acetylation on residue N2.

It will be appreciated that **FI** and **M** may in fact be present in either orientation. Residue N2 is typically lysine (in the case of assays for lysine acetylases) .

Amino acid sequences X3 and X4 are selected both to place residue N2 in an appropriate sequence context for acetylation, and cleavage by protease (in unacetylated structure (III)), and also to provide optimum orientation of the fluorescent moiety **(FI)** and the fluorescence lifetime modulator **(M)** to achieve effective modulation of the fluorescence lifetime of **FI.** The sequences of X3 and X4 may be based around the sequence of a native substrate for the acetyl transferase enzyme being assayed. It will be appreciated that although fluorescent moiety **(FI)** and the fluorescence lifetime modulator **(M)** need to positioned at an appropriate spacing for effective modulation of the fluorescence lifetime, it is not essential for either fluorescent moiety **(FI)** or fluorescence lifetime modulator **(M)** to occur at the end (N-terminus or C-terminus) of the peptide linker. Hence FI may be attached to an amino acid residue within X3 and **M** may be attached to an amino acid residue within X4, or *vice versa.* If the fluorescence lifetime modulator **M** is itself provided by the side-chain of an amino acid residue, e.g. tryptophan, tyrosine, phenylalanine, naphthylalanine or histidine or a derivative thereof, then the amino acid residue which acts as the modulator **M** may itself form part of the amino acid sequence represented by X4. Amino acid sequences X3 and X4 may contain naturally-occurring or non-natural amino acid residues, and other modifications such as non-peptide linkages.

In the substrate of structure (III), the fluorescence lifetime of fluorescent moiety **FI** (which is preferably 9-aminoacridine (9AA) but may be any other suitable fluorescent moiety) is modulated by the presence of a fluorescence lifetime modulator **M** in the substrate **(M** is preferably tryptophan but may be any other known modulator of fluorescence lifetime). Treatment of the substrate of structure (III) with a protease that cleaves after residue N2 (e.g. lysine) will induce proteolysis and remove modulation of the fluorescence lifetime by the modulator **M.** After acetylation on residue N2 (e.g. lysine) by an appropriate acetyl transferase enzyme, then treatment of the modified peptide of structure (IV) with the protease does not induce cleavage after residue N2 and the lifetime of the fluorescent moiety **(FI)** remains modulated. Where there is continual conversion of substrate (III) into modified substrate (IV), as in a time-course assay, the fluorescence lifetime of the fluorescent moiety **(FI)** will decrease as a function of time (and concentration of acetyltransferase enzyme). A shift in fluorescence lifetime (decrease) relative to a base-line measurement for 100% unmodified substrate (III) indicates that some conversion from substrate (III) to modified substrate (IV) has occurred. The magnitude of this shift can thus be used to assess conversion from substrate (III) to modified substrate (IV) due to the action of the acetyltransferase.

Accordingly, there is provided herein a method of assaying the activity of a protein acetyltransferase enzyme which comprises:
(a) contacting a test sample with a fluorescent-modulated enzyme substrate of the general formula (III),

   **FI -** X3-N2-X4- **M** (III)

   wherein X3 represents a first sequence of amino acids, **FI** represents a fluorescent moiety which is conjugated to X3, N2 represents an amino acid residue which is acetylated by the action of the acetyl transferase, X4 represents a second sequence of amino acids and **M** represents a fluorescence lifetime modulator which is conjugated to X4, under conditions which permit enzymatic conversion of the fluorescent-modulated enzyme substrate to a modified substrate of general formula (IV),

   **FI -** X3-N2(Ac)-X4- **M** (IV)

   wherein N2(Ac) represents acetylation on residue N2, by the action of the protein acetyl transferase enzyme;
(b) contacting the products of step (a) with a protease enzyme which is capable of cleaving the fluorescent-modulated enzyme substrate of the general formula (III) adjacent to residue N2, but is not capable of cleaving the modified substrate of general formula (IV) between **FI** and **M;** and
(c) detecting changes in the fluorescence lifetime of the fluorescent moiety **FI,** thereby assaying the activity of the protein acetyltransferase enzyme.

This method can be readily applied to screen for inhibitors of the protein acetyl transferase enzyme, by adding candidate compounds to be tested for inhibitor activity in step (a).

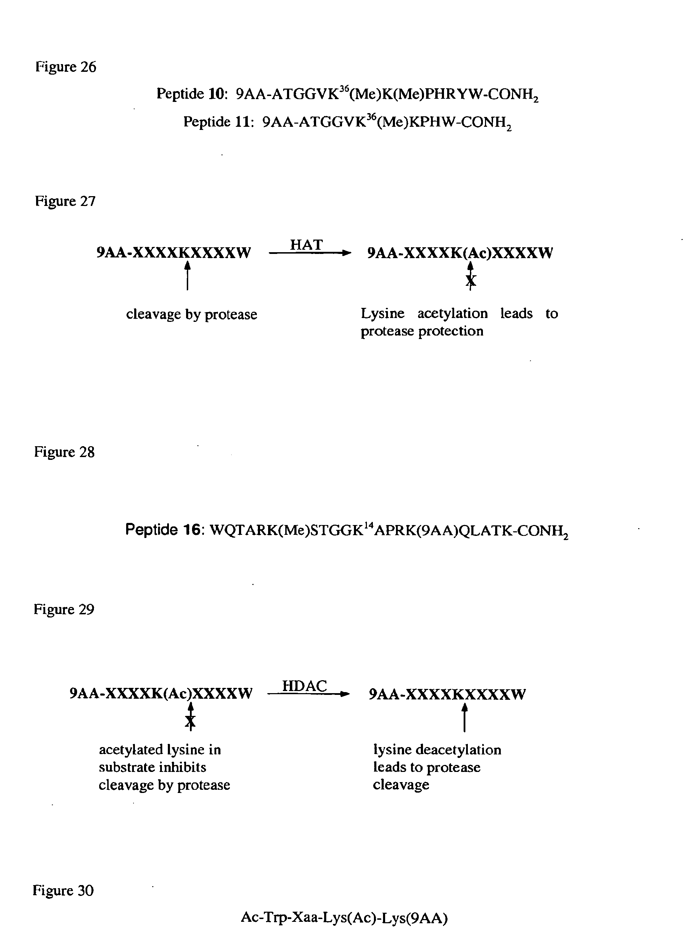

A specific example of a method of assaying histone acetyltransferases (HAT), which acetylate lysine residues in peptide/protein substrates, via a FLT protease protection approach is shown in accompanying Figure. 27. This example is based upon the use of 9-aminoacridine as the fluorescent moiety and the indolyl side-chain of a tryptophan residue as the fluorescence lifetime modulator. However, it will be appreciated that these specific fluorescent moieties and fluorescence lifetime modulator moieties are shown by way of example only and can be replaced by alternative fluorescent moieties and fluorescence lifetime modulator moieties as described herein.

The general methodology described above can be applied to determine the activity of any acetyltransferase enzyme of interest, including but not limited to the any known histone acetyltransferase (HAT) enzyme, including any of the histone acetyltransferase enzymes belonging to the enzyme class EC 2.3.1.48. In particular, the assay method may be applied to the histone acetyltransferases Gcn5, PCAF, Hat1 and p300, given as non-limiting examples.

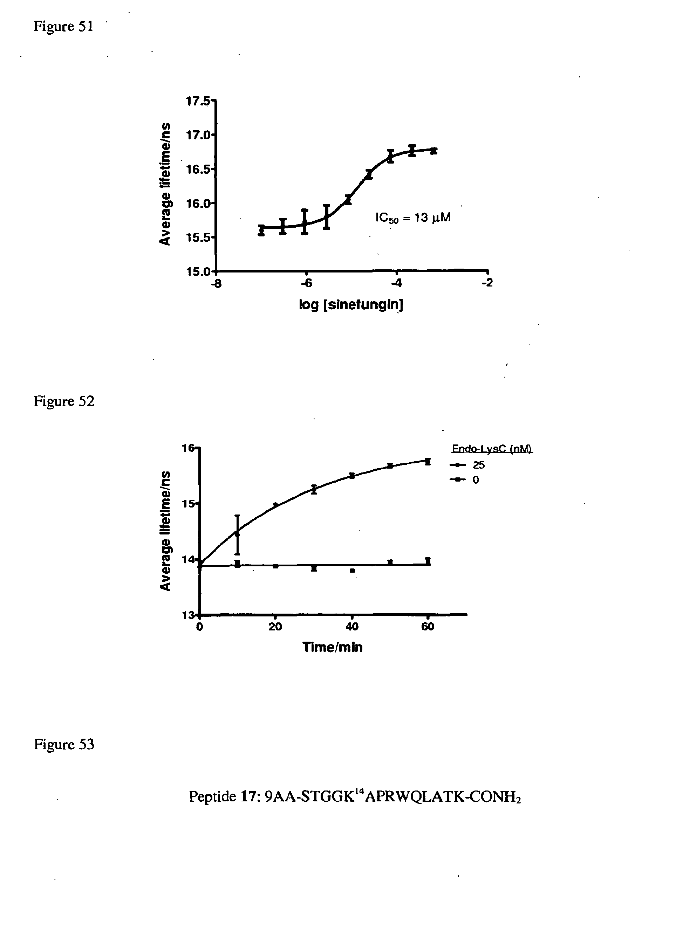

Peptide substrates based on histone N-terminal sequences incorporating a fluorophore **(FI)** and modulator moiety **(M)** flanking a lysine residue which is the site of acetylation for a particular HAT would form the basis of a FLT protease protection assay for this class of enzymes. Examples of such a substrate are shown below, and in Figures 28 and 53, based on use of 9-aminoacridine **(9AA)** as the fluorescent moiety and tryptophan **(W)** as the fluorescence lifetime modulator moiety and histone H3 sequence with K¹⁴ the site of acetylation by a HAT such as PCAF.
Peptide **16: W**QTARK(Me)STGGK¹⁴APRK(**9AA**)QLATK-CONH₂
Peptide **17**:**9AA**-STGGK¹⁴APR**W**QLATK-CONH₂

Peptide substrates suitable for use in assaying other acetyl transferase enzymes can be prepared following the general principles illustrated in the accompanying examples.

Suitable proteases for use in the general approach outlined above include, but are not limited to, Endo-LysC which cleaves after lysine (for assays based on acetylation of lysine).

### (3) Deiminase assay

In one embodiment, the assay method described herein may be adapted to assay the activity of a deiminase enzyme.

The substrate for this assay comprises a peptide linker, and has the general structure:

**FI -** X5-R-X6- **M** (V)

wherein X5 represents a first sequence of amino acids, **FI** represents a fluorescent moiety which is conjugated to X5, R represents an arginine residue which is converted to citrulline by the action of the deiminase, X6 represents a second sequence of amino acids and **M** represents a fluorescence lifetime modulator which is conjugated to X6. Following treatment with deiminase, the substrate is converted into a modified (deiminated) substrate represented by the general structure:

**FI -** X5-R(Cit)-X6- **M** (VI)

wherein R(Cit) represents conversion of residue R to citrulline by deimination.

It will be appreciated that **FI** and **M** may in fact be present in either orientation. Amino acid sequences X5 and X6 are selected both to place residue R in an appropriate sequence context for deimination to citrulline, and cleavage by protease (in structure (V), and also to provide optimum orientation of the fluorescent moiety (FI) and the fluorescence lifetime modulator **(M)** to achieve effective modulation. The sequences of X5 and X6 may be based around the sequence of a native substrate for the deiminase enzyme being assayed. It will be appreciated that although fluorescent moiety (FI) and the fluorescence lifetime modulator **(M)** need to positioned at an appropriate spacing for effective modulation of the fluorescence lifetime, it is not essential for either fluorescent moiety **(FI)** or fluorescence lifetime modulator **(M)** to occur at the end (N-terminus or C-terminus) of the peptide linker. Hence **FI** may be attached to an amino acid residue within X5 and **M** may be attached to an amino acid residue within X6, or *vice versa.* If the fluorescence lifetime modulator **M** is itself provided by the side-chain of an amino acid residue, e.g. tryptophan, tyrosine, phenylalanine, naphthylalanine or histidine or a derivative thereof, then the amino acid residue which acts as the modulator **M** may itself form part of the amino acid sequence represented by X6. Amino acid sequences X5 and X6 may contain naturally-occurring or non-natural amino acid residues, and other modifications such as non-peptide linkages.

In the substrate of structure (V), the fluorescence lifetime of fluorescent moiety **FI** (which is preferably 9-aminoacridine (9AA) but may be any other suitable fluorescent moiety) is modulated by the presence of a fluorescence lifetime modulator **M** in the substrate **(M** is preferably tryptophan but may be any other known modulator of fluorescence lifetime). Treatment of the substrate of structure (V) with a protease that cleaves after residue R will induce proteolysis and remove modulation of the fluorescence lifetime by the modulator **M.** After conversion of residue R to citrulline, by an appropriate deiminase enzyme, then treatment of the modified peptide of structure (VI) with the protease does not induce cleavage after residue R and the lifetime of the fluorescent moiety **(FI)** remains modulated. Where there is continual conversion of substrate (V) into modified substrate (VI), as in a time-course assay, the fluorescence lifetime of the fluorescent moiety **(FI)** will decrease as a function of time (and concentration of deiminase enzyme). A shift in fluorescence lifetime (decrease) relative to a base-line measurement for 100% unmodified substrate (V) indicates that some conversion from substrate (V) to modified substrate (VI) has occurred. The magnitude of this shift can thus be used to assess conversion from substrate (V) to modified substrate (VI) due to the action of the deiminase.

Accordingly, there is provided herein a method of assaying the activity of a deiminase enzyme which comprises:
(a) contacting a test sample with a fluorescent-modulated enzyme substrate of the general formula (V),

   **FI -** X5-R-X6- **M** (V)

   wherein X5 represents a first sequence of amino acids, **FI** represents a fluorescent moiety which is conjugated to X5, R represents an arginine residue which is converted to citrulline by the action of the deiminase, X6 represents a second sequence of amino acids and **M** represents a fluorescence lifetime modulator which is conjugated to X6, under conditions which permit enzymatic conversion of the fluorescent-modulated enzyme substrate to a modified substrate of general formula (VI),

   **FI -** X5-R(Cit)-X6- **M** (VI)

   wherein R(Cit) represents citrulline, formed by the action of the deiminase enzyme;
(b) contacting the products of step (a) with a protease enzyme which is capable of cleaving the fluorescent-modulated enzyme substrate of the general formula (V) adjacent to residue R, but is not capable of cleaving the modified substrate of general formula (VI) between **FI** and **M;** and
(c) detecting changes in the fluorescence lifetime of the fluorescent moiety **FI,** thereby assaying the activity of the deiminase enzyme.

This method can be readily applied to screen for inhibitors of the deiminase enzyme, by adding candidate compounds to be tested for inhibitor activity in step (a).

A specific example of a method of assaying the deiminase class of enzymes, specifically the peptidyl arginine deiminases which convert arginine residues in peptide/protein substrates to citrulline, via a FLT protease protection approach, is shown schematically in Fig. 1. This example is based upon the use of 9-aminoacridine as the fluorescent moiety and the indolyl side-chain of a tryptophan residue as the fluorescence lifetime modulator. However, it will be appreciated that these specific fluorescent moieties and fluorescence lifetime modulator moieties are shown by way of example only and can be replaced by alternative fluorescent moieties and fluorescence lifetime modulator moieties as described herein.

The general methodology described above can be adapted for use with any deiminase enzyme of interest, including but not limited to, the peptidylarginine deiminase (PAD) enzymes, for example PAD1, PAD2, PAD3, PAD4 and PAD6. In particular, the method can be applied to any peptidyl arginine deiminase (PAD) belonging to the enzyme class EC 3.5.1.15. The PAD enzymes may be referred to alternatively as protein-arginine deiminases.

Examples of suitable substrates for use in assaying activity of PAD2 or PAD4 are shown below, based on use of 9-aminoacridine **(9AA)** as the fluorescent moiety and tryptophan **(W)** as the fluorescence lifetime modulator moiety:
Peptide 1: **9AA**-QSTRGSGH**W**KK-CONH₂
Peptide 3: K(**9AA**)-HQST**R**GSGH**W**KK-CONH₂

Peptide substrates suitable for use in assaying other deiminase enzymes, specifically other peptidyl arginine deiminases, can be prepared following the general principles outlined above and illustrated in the accompanying examples.

Suitable proteases for use the general approach outlined above include, but are not limited to, trypsin and Endo-ArgC which cleave after arginine.

### (B) Assays based on modification to remove protease protection

A second group of embodiments of the assay are based on use of a substrate comprising a peptide linker molecule conjugated to a fluorescent moiety and a fluorescence lifetime modulator moiety configured to modulate the fluorescence lifetime of the fluorescent moiety, the peptide being modified by the action of the modifying enzyme to form a modified peptide wherein the peptide in the substrate is not capable of being cleaved by the second enzyme (i.e. a protease) between the fluorescent moiety and the fluorescence lifetime modulator moiety and modification of the peptide by the modifying enzyme converts the peptide to a modified peptide which is cleaved by the second enzyme (i.e. protease) between the fluorescent moiety and the fluorescence lifetime modulator moiety. In such embodiments, the substrate is not susceptible to cleavage by the second enzyme (protease) between the fluorescent moiety and the fluorescence lifetime modulator moiety, but the modified substrate formed by action of the modifying enzyme on the substrate is cleaved by the second enzyme (protease) between the fluorescent moiety and the fluorescence lifetime modulator moiety, which produces an increase in the fluorescence lifetime of the fluorescent moiety. Formation of the modified peptide (by the action of the modifying enzyme on the substrate) is thus indicated by a time-dependent increase in the fluorescence lifetime of the fluorescent moiety after protease treatment, as compared to unmodified peptide.

Non-limiting embodiments of this assay are as follows:

### (4) Demethylase assay

In one embodiment, the assay method described herein may be adapted to assay the activity of a demethylase enzyme.

The substrate for this assay comprises a peptide linker, and has the general structure (VII):

**FI -** X7-N3(Me)-X8- **M** (VII)

wherein X7 represents a first sequence of amino acids, **FI** represents a fluorescent moiety which is conjugated to X7, N3(Me) represents a methylated amino acid residue (e.g. methylated lysine or methylated arginine) which is demethylated by the action of the demethylase, X8 represents a second sequence of amino acids and **M** represents a fluorescence lifetime modulator which is conjugated to X8. Following treatment with demethylase, the substrate is converted into a modified (demethylated) substrate represented by the general structure:

**FI -** X7-N3-X8- **M** (VIII)

wherein N3 represents the demethylated amino acid, e.g. lysine or arginine.

It will be appreciated that **FI** and **M** may in fact be present in either orientation. Amino acid sequences X7 and X8 are selected both to place residue N3(Me) in an appropriate sequence context for demethylation, and cleavage by protease (in the modified substrate of structure (VIII), and also to provide optimum orientation of the fluorescent moiety **(FI)** and the fluorescence lifetime modulator **(M)** to achieve effective modulation. The sequences of X7 and X8 may be based around the sequence of a native substrate for the demethylase enzyme being assayed. It will be appreciated that although fluorescent moiety **(FI)** and the fluorescence lifetime modulator **(M)** need to positioned at an appropriate spacing for effective modulation of the fluorescence lifetime, it is not essential for either fluorescent moiety **(FI)** or fluorescence lifetime modulator **(M)** to occur at the end (N-terminus or C-terminus) of the peptide linker. Hence **FI** may be attached to an amino acid residue within X7 and **M** may be attached to an amino acid residue within X8, or *vice versa.* If the fluorescence lifetime modulator **M** is itself provided by the side-chain of an amino acid residue, e.g. tryptophan, tyrosine, phenylalanine, naphthylalanine or histidine or a derivative thereof, then the amino acid residue which acts as the modulator **M** may itself form part of the amino acid sequence represented by X8. Amino acid sequences X7 and X8 may contain naturally-occurring or non-natural amino acid residues, and other modifications such as non-peptide linkages.

In the case of assays based on demethylation of arginine, the arginine residue represented by N(Me) can either be mono- or di-methylated, with the latter in symmetric or asymmetric configurations, depending on the substrate-specificity of the demethylase enzyme whose activity is being assayed. The precise degree of methylation of the starting substrate and the configuration of di-methylated forms is not material to the performance of the assay, provided that the demethylase enzyme being assayed is able to demethylate the substrate down to a fully demethylated form which can be cleaved by protease. Hence the residue N(Me) may, in the case of arginine methylation, represent any of the mono- or di- methylated forms, as appropriate in the context of the assay.

In the case of assays based on demethylation of lysine, the lysine residue represented by N(Me) can either be mono-, di- or tri-methylated, depending on the substrate-specificity of the lysine demethylase enzyme whose activity is being assayed. The precise degree of methylation of the starting substrate and the configuration of di-methylated forms is not material to the performance of the assay, provided that the lysine demethylase enzyme being assayed is able to demethylate the substrate down to a fully demethylated form which can be cleaved by protease. Hence the residue N(Me) may, in the case of lysine methylation, represent any of the mono-, di- or tri- methylated forms, as appropriate in the context of the assay.

In the substrate of structure (VII), the fluorescence lifetime of fluorescent moiety **FI** (which is preferably 9-aminoacridine (9AA) but may be any other suitable fluorescent moiety) is modulated by the presence of a fluorescence lifetime modulator **M** in the substrate **(M** is preferably tryptophan but may be any other known modulator of fluorescence lifetime). In the absence of any demethylase enzyme, treatment of the substrate of structure (VII) with a protease that cleaves after residue N3 (e.g. lysine or arginine) will NOT induce proteolysis of structure (VII) between **FI** and **M,** since the substrate of structure (VII) contains the methylated form of N3 and is thus resistant to proteolysis. The fluorescence lifetime modulation of the fluorescent moiety **FI** (e.g. 9AA) by the fluorescence lifetime modulator **M** (e.g. tryptophan) therefore remains. In the presence of demethylase, residue N3(Me) is demethylated to form residue N3 in the modified substrate of structure (VIII). Treatment of the modified substrate of structure (VIII) with the protease induces cleavage after residue N3 (e.g. lysine or arginine) and the fluorescence lifetime of the fluorophore (e.g. 9AA) is no longer modulated. Where there is continual conversion of substrate (VII) into modified substrate (VIII), as in a time-course assay, the fluorescence lifetime of the fluorescent moiety **(PI)** will increase as a function of time (and concentration of demethylase enzyme) as more of the modified substrate is formed and cleaved by the action of the protease. A shift in fluorescence lifetime (increase) relative to a base-line measurement for 100% unmodified substrate (VII) indicates that some conversion from substrate (VII) to modified substrate (VIII) has occurred. The magnitude of this shift can thus be used to assess conversion from substrate (VII) to modified substrate (VIII) due to the action of the demethylase.

Accordingly, there is provided herein a method of assaying the activity of a protein demethylase enzyme which comprises:
(a) contacting a test sample with a fluorescent-modulated enzyme substrate of the general formula (VII),

   **FI -** X7-N3(Me)-X8- **M** (VII)

   wherein X7 represents a first sequence of amino acids, **FI** represents a fluorescent moiety which is conjugated to X7, N3(Me) represents a methylated amino acid residue (e.g. methylated lysine or methylated arginine) which is demethylated by the action of the demethylase, X8 represents a second sequence of amino acids and **M** represents a fluorescence lifetime modulator which is conjugated to X8, under conditions which permit enzymatic conversion of the fluorescent-modulated enzyme substrate to a modified substrate of general formula (VIII),

   **FI -** X7-N3-X8- **M** (VIII)

   wherein N3 represents the demethylated amino acid, by the action of the demethylase enzyme;
(b) contacting the products of step (a) with a protease enzyme which is capable of cleaving the modified substrate of the general formula (VIII) adjacent to residue N3, but is not capable of cleaving the fluorescent-modulated enzyme substrate of general formula (VII) between **FI** and **M;** and
(c) detecting changes in the fluorescence lifetime of the fluorescent moiety **FI,** thereby assaying the activity of the demethylase enzyme.

This method can be readily applied to screen for inhibitors of the demethylase enzyme, by adding candidate compounds to be tested for inhibitor activity in step (a).

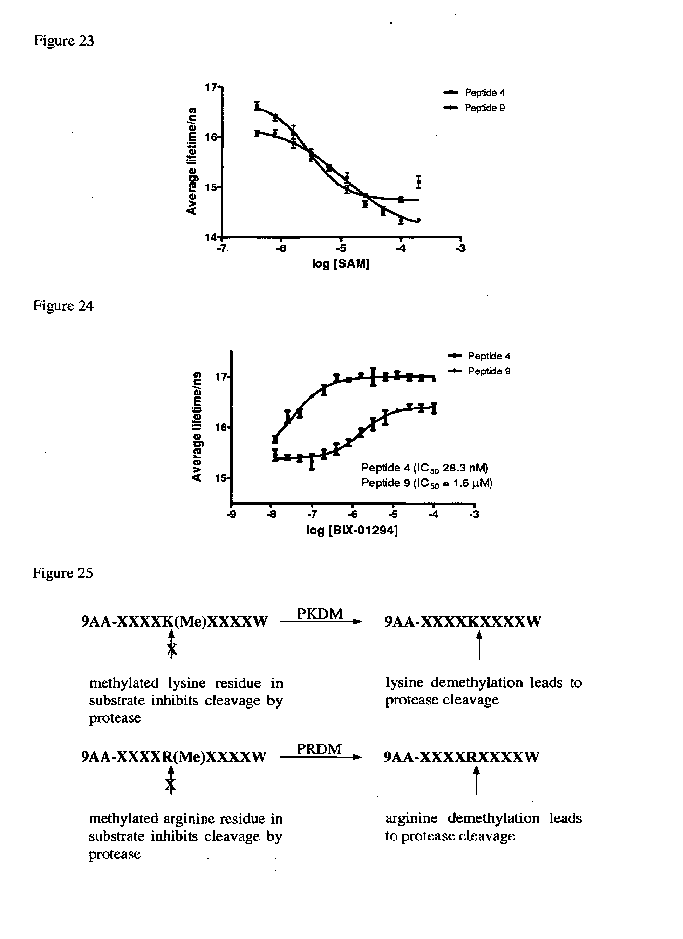

A specific example of a method of assaying protein demethylases (PDM), which remove one or more methyl groups from lysine or arginine residues in peptide/protein substrates, via a FLT protease protection approach is shown schematically in Fig. 25. This example is based upon the use of 9-aminoacridine as the fluorescent moiety and the indolyl side-chain of a tryptophan residue as the fluorescence lifetime modulator. However, it will be appreciated that these specific fluorescent moieties and fluorescence lifetime modulator moieties are shown by way of example only and can be replaced by alternative fluorescent moieties and fluorescence lifetime modulator moieties as described herein.

The general methodology described above can be adapted for use with any demethylase enzyme of interest. Particularly preferred are PKDM enzymes which demethylate peptide substrates methylated on lysine. In particular, the assay method can be applied to histone-lysine demethylases. The histone-lysine demethylases can be classified according to substrate specificity. The histone-lysine demethylases which demethylate lysine residues histone protein H3 may demethylate lysine 4 (referred to as [histone-H3]-lysine-4 demethylases; EC 1.14.11.B2), lysine 9 (referred to as [histone-H3]-lysine-9 demethylases; EC 1.14.11.B1), lysine 36 (referred to as [histone-H3]-lysine-36 demethylases; EC 1.14.11.27) or lysine 27 (referred to as [histone-H3]-lysine-27 demethylases. The histone-lysine demethylases which demethylate lysine residues in histone protein H4 may demethylate lysine 20. The assay method described above can be applied to enzymes belonging to any of these classes of histone-lysine demethylases, including but not limiting to LSD1, JHDM1A, JMJD1A, JMJD2A, JMJD2B, JMJD2C, JMJD3(KDM6), FBXL(KDM2) and KDM5, listed by way of example only.

The assay may also be applied to arginine demethylase enzymes (PRDM enzymes) which demethylate peptide substrates methylated on arginine, e.g. JMJD6.

A suitable substrate for an FLT assay of the representative PKDM enzyme JHDM1A is shown below. JHDM1A preferentially demethylates lysine 36 of histone H3 converting it from di-or monomethylated states to the native unmethylated residue. Hence, the substrate is based on residue lysine-36 in an appropriate sequence context.
Peptide **10: 9AA**-ATGGVK³⁶(Me)K(Me)PHRY**W**-CONH₂
In this example substrate, the fluorescence lifetime of the fluorescent moiety 9AA (or another suitable fluorescent moiety) is modulated by the presence of the tryptophan residue (or another suitable fluorescence lifetime modulator) incorporated close to the C-terminus. Demethylation of lysine-36 by JHDM1A permits protease-induced cleavage at this residue, thereby relieving modulation and resulting in an increase in the fluorescence lifetime of 9AA. In contrast, the presence of a JHDM1A inhibitor would prevent lysine demethylation thereby leaving the peptide resistant to protease-induced cleavage at lysine-36. Hence, the fluorescence lifetime of 9AA would remain modulated and the extent of the modulation could be correlated to the degree of demethylation. A suitable protease for use in this assay would be Endo-LysC, which will not cleave methylated lysines (lysine-37 is protected as the monomethyl derivative to prevent non-specific cleavage at this residue).

A second suitable substrate for an FLT assay of the representative PKDM enzyme JHDM1A is shown below.
Peptide **11: 9AA**-ATGGVK³⁶(Me)KPH**W**-CONH₂
In this example substrate, the fluorescence lifetime of the fluorescent moiety 9AA (or another suitable fluorescent moiety) is modulated by the presence of the tryptophan residue (or another suitable fluorescence lifetime modulator) incorporated close to the C-terminus. Demethylation of lysine-36 by JHDM1A permits protease-induced cleavage at this residue, thereby relieving modulation and resulting in an increase in the fluorescence lifetime of 9AA. In contrast, the presence of a JHDM1A inhibitor would prevent lysine demethylation thereby leaving the peptide resistant to protease-induced cleavage at lysine-36. Hence, the fluorescence lifetime of 9AA would remain modulated and the extent of the modulation could be correlated to the degree of demethylation. A suitable protease for use in this assay would be trypsin, which is reported not to cleave lysines where the adjacent C-terminal residue is proline (for example Keil, B. Specificity of Proteolyis) or have severely compromised cleavage activity (Rodriguez, J., et al, J. Prot. Research, 2008, 7, 300-305), thereby ensuring non-specific cleavage at lysine-37 is prevented, or reduced to an insignificant level.

Peptide substrates suitable for use in assaying other demethylase enzymes can be prepared following the general principles outlined above.

### (5) Deacetylase assay

In one embodiment, the assay method described herein may be adapted to assay the activity of a deacetylase enzyme.

The substrate for this assay comprises a peptide linker, and has the general structure:

**FI -** X9-N4(Ac)-X10- **M** (IX)

wherein X9 represents a first sequence of amino acids, **FI** represents a fluorescent moiety which is conjugated to X9, N4(Ac) represents a acetylated amino acid residue (e.g. acetylated lysine) which is deacetylated by the action of the deacetylase, X10 represents a second sequence of amino acids and **M** represents a fluorescence lifetime modulator which is conjugated to X10. Following treatment with deacetylase, the substrate is converted into a modified (deacetylated) substrate represented by the general structure:

**FI -** X9-N4-X10- **M** (X)

wherein N4 represents the deacetylated amino acid, e.g. lysine.

It will be appreciated that **FI** and **M** may in fact be present in either orientation. Amino acid sequences X9 and X10 are selected both to place residue N4(Ac) in an appropriate sequence context for deacetylation, and cleavage by protease (in the modified substrate of structure (X), and also to provide optimum orientation of the fluorescent moiety **(FI)** and the fluorescence lifetime modulator **(M)** to achieve effective modulation. The sequences of X9 and X10 may be based around the sequence of a native substrate for the deacetylase enzyme being assayed. It will be appreciated that although fluorescent moiety **(FI)** and the fluorescence lifetime modulator **(M)** need to positioned at an appropriate spacing for effective modulation of the fluorescence lifetime, it is not essential for either fluorescent moiety **(FI)** or fluorescence lifetime modulator **(M)** to occur at the end (N-terminus or C-terminus) of the peptide linker. Hence FI may be attached to an amino acid residue within X9 and **M** may be attached to an amino acid residue within X10, or *vice versa.* If the fluorescence lifetime modulator **M** is itself provided by the side-chain of an amino acid residue, e.g. tryptophan, tyrosine, phenylalanine, naphthylalanine or histidine or a derivative thereof, then the amino acid residue which acts as the modulator **M** may itself form part of the amino acid sequence represented by X10. Amino acid sequences X9 and X10 may contain naturally-occurring or non-natural amino acid residues, and other modifications such as non-peptide linkages.

In the substrate of structure (IX), the fluorescence lifetime of fluorescent moiety FI (which is preferably 9-aminoacridine (9AA) but may be any other suitable fluorescent moiety) is modulated by the presence of a fluorescence lifetime modulator **M** in the substrate (**M** is preferably tryptophan but may be any other known modulator of fluorescence lifetime). In the absence of any deacetylase enzyme, treatment of the substrate of structure (IX) with a protease that cleaves after residue N4 (e.g. lysine) will NOT induce proteolysis of structure (IX) between FI and **M,** since the substrate of structure (IX) contains the acetylated form of N4 and is thus protected from proteolysis. The fluorescence lifetime modulation of the fluorescent moiety **FI** (e.g. 9AA) by the fluorescence lifetime modulator **M** (e.g. tryptophan) therefore remains intact. In the presence of deacetylase, residue N4(Ac) is deacetylated to form residue N4 in the modified substrate of structure (X). Treatment of the modified substrate of structure (X) with the protease induces cleavage after residue N4 (e.g. lysine) and the fluorescence lifetime of the fluorophore (e.g. 9AA) is no longer modulated.

Where there is continual conversion of substrate (IX) into modified substrate (X), as in a time-course assay, the fluorescence lifetime of the fluorescent moiety **(FI)** will increase as a function of time (and concentration of deacetylase enzyme) as more of the modified substrate is formed and cleaved by the action of the protease. A shift in fluorescence lifetime (increase) relative to a base-line measurement for 100% unmodified substrate (IX) indicates that some conversion from substrate (IX) to modified substrate (X) has occurred. The magnitude of this shift can thus be used to assess conversion from substrate (IX) to modified substrate (X) due to the action of the deacetylase.

Accordingly, there is provided herein a method of assaying the activity of a protein deacetylase enzyme which comprises:
(a) contacting a test sample with a fluorescent-modulated enzyme substrate of the general formula (IX),

   **FI -** X9-N4(Ac)-X10- **M** (IX)

   wherein X9 represents a first sequence of amino acids, **FI** represents a fluorescent moiety which is conjugated to X9, N4(Ac) represents a acetylated amino acid residue (e.g. acetylated lysine) which is deacetylated by the action of the deacetylase, X10 represents a second sequence of amino acids and **M** represents a fluorescence lifetime modulator which is conjugated to X10, under conditions which permit enzymatic conversion of the fluorescent-modulated enzyme substrate to a modified substrate of general formula (X),

   **FI -** X9-N4-X10- **M** (X)

   wherein N4 represents the deacetylated amino acid, by the action of the deacetylase enzyme;
(b) contacting the products of step (a) with a protease enzyme which is capable of cleaving the modified substrate of the general formula (X) adjacent to residue N4, but is not capable of cleaving the fluorescent-modulated enzyme substrate of general formula (IX) between **FI** and **M;** and
(c) detecting changes in the fluorescence lifetime of the fluorescent moiety **FI,** thereby assaying the activity of the deacetylase enzyme.

This method can be readily applied to screen for inhibitors of the deacetylase enzyme, by adding candidate compounds to be tested for inhibitor activity in step (a).

A specific example of a method of assaying protein deacetylases, for example the histone deacetylases (HDAC), which remove one or more acetyl groups from lysine residues in peptide/protein substrates, via a FLT protease protection approach is shown schematically in Fig. 29. This example is based upon the use of 9-aminoacridine as the fluorescent moiety and the indolyl side-chain of a tryptophan residue as the fluorescence lifetime modulator. However, it will be appreciated that these specific fluorescent moieties and fluorescence lifetime modulator moieties are shown by way of example only and can be replaced by alternative fluorescent moieties and fluorescence lifetime modulator moieties as described herein.

The general methodology described above can be adapted for use with any deacetylase enzyme of interest. Particularly preferred are the histone deacetylase (HDAC) enzymes, including but not limiting to HDAC1-11 and SIRT1-5. In particular, the assay method described above can be applied to any histone deacetylase within the enzyme class EC3.5.1.98, including enzymes falling within one of the three sub-classes HDAC1, HDAC2 and HDAC3.

An example HDAC substrate is illustrated below:
Ac-**Trp**-Xaa-**Lys(Ac)**-Lys(**9AA**)
Xaa = any amino acid.

An example HDAC1 substrate is illustrated below:
Peptide **18:** Ac-I**W**K(Ac)K(**9AA**)-CONH₂

In these substrates, the fluorescent moiety (9AA or another suitable fluorescent moiety) is incorporated at the C-terminus adjacent to the lysine which is deacetylated, with a tryptophan (or another suitable fluorescence lifetime modulator) placed on the N-terminal side of the aforesaid lysine. Protease-induced cleavage following HDAC1 catalysed deacetylation, leads to an increase in fluorescence lifetime as 9AA is separated from the tryptophan residue. Hence, an increase in fluorescence lifetime can be directly correlated to the extent of HDAC activity. Of course, it is envisaged that other similarly designed substrates can be designed depending on the sequence specificity of the HDAC in question.

### Practical applications of the assays

The assay method described herein may all be performed in a high-throughput screening assay format, which enhances the utility of the assays for the screening and identification of inhibitors of the modifying enzyme.

When the assays described herein are used in a screening context, the assays described above are performed in the presence of compounds to be tested for inhibitor activity. The general format of such high-throughput screening assays will be familiar to a person of ordinary skill in the art. Typically compounds will be tested in parallel with suitable assay controls (e.g. no inhibitor, no modifying enzyme, high concentration of a known inhibitor). Compounds may be tested at several different concentrations in parallel. A reduction in modifying enzyme activity in the presence of the test compound, as indicated by a change in the fluorescence lifetime readout of the assay, identifies the test compound as an inhibitor of the modifying enzyme.

The assays of the present invention may typically be performed in the wells of a multiwell plate, e.g. a microtitre plate having 24, 96, 384 or higher densities of wells e.g. 864 or 1536 wells.

High-throughput screening assays for the identification of enzyme inhibitors, typically utilise a highly pure form of the modifying enzyme, e.g. recombinant enzyme. The modifying enzyme, and the corresponding fluorescent-modulated substrate, are added to the wells of a multi-well plate in a suitable enzyme reaction buffer which supports activity of the modifying enzyme, together with the test compound (potential enzyme inhibitor). Typically the compound is tested at serial dilutions over an appropriate concentration range, taking account of the kinetics of the enzyme under test (e.g. 10nM-10µM). The amount of modifying enzyme and the amount of substrate added to the wells is kept constant (except for control wells containing no enzyme). Control wells containing high concentration of a known inhibitor of the enzyme and also wells containing no compound may also be prepared. Prepared plates containing enzyme, fluorescent-modulated enzyme substrate and test compound, plus appropriate controls, are then incubated under conditions which, in the absence of any inhibitor, the modifying enzyme is active. Conversion of the substrate to the modified substrate by the action of the modifying enzyme takes place during this incubation. If the test compound is an inhibitor of the modifying enzyme, then conversion of the fluorescent-modulated substrate to the modified substrate by the enzyme will be inhibited; the extent of inhibition being dependent on the concentration of the inhibitor and its potency as an inhibitor. It is of course critical to the performance of the assay that no conversion of substrate to modifying substrate takes place other than as a result of the activity of the modifying enzyme.

The amount of modifying enzyme added to the assay, and the length of the incubation allowed for conversion of substrate to modified substrate may be selected to optimise the dynamic range of the assay. For example, the amount of the enzyme added and the incubation time may be selected to achieve less than 100% conversion of substrate to modified substrate in the absence of any inhibitor.

Once the incubation of enzyme, substrate and inhibitor is complete, the second enzyme (e.g. protease) is added and fluorescence lifetime monitored using an appropriate instrument (e.g. NanoTaurus™ plate reader). As explained elsewhere herein, the assay may be based on measurement of average fluorescence lifetime, or on measurement of amount of a particular fluorescent species with a specific lifetime corresponding to either the substrate or product, i.e. either specific measurement of cleaved substrate/modified substrate which exhibits a "long" fluorescence lifetime (modulation absent) or specific measurement of uncleaved substrate/modified substrate (modulation still present). In either case, the fluorescence lifetime measurements provide an indication of the activity of the modifying enzyme in the presence of the test compound, for each concentration of test compound used. An IC₅₀ value for the test compound can be determined from a plot of % residual modifying enzyme activity against concentration of the test compound.

### Peptides substrates

Another aspect of the invention provides peptide substrates for use in carrying out the enzyme assays described herein. In particular, the following substrates are provided:
(1) Substrates for methyltransferase assays

   **FI** -X1-N1-X2- **M** (I),

   or

   **M -** X1-N1-X2- **FI** (I')

   wherein X1 represents a first sequence of amino acids, **FI** represents a fluorescent moiety which is conjugated to X1 (or X2 in I'), N1 represents an amino acid residue which is methylated by the action of the methyl transferase, X2 represents a second sequence of amino acids and **M** represents a fluorescence lifetime modulator which is conjugated to X2 (or X1 in I').
   In embodiments of the invention the substrate is:
   Peptide **4: 9AA**-TARK⁹STG**W**-CONH₂. Also described are:
   Peptide **5:** K(**9AA**)QTARK⁹STG**W**-CONH₂
   Peptide **6:** ARTK(**9AA**)QTARK⁹STGG**W**-CONH₂
   Peptide **7:** ART**W**OTARK⁹STGGK(**9AA**)-CONH₂
   Peptide **8: W**QTARK⁹STGGK(**9AA**)-CONH₂
   Peptide **9:** K(**9AA**)ARTK(Me)QTARK⁹STGG**W**-CONH₂
   Peptide **12: W**ARTK⁴QTARK(**9AA**)STGGKAPRKQLAK-CONH₂
   Peptide **13: W**RTK⁴QTARK(**9AA**)STGGKAPRKQLAK-CONH₂
   Peptide **14: W**SGR³GKGGK(**9AA**)GLGKGGAKRHRK-CONH₂
   Peptide **15:** Ac-**W**SGR³GKGGK(9AA)GLGKGGAKRHRK-CONH₂
   Peptide **20:** PRKQLATK(**9AA**)AARK²⁷SAPATGG**W**-CONH₂
(2) Substrates for acetyltransferase assays

   **FI -** X3-N2-X4- **M** (III),

   or

   **M -** X3-N2-X4- **FI** (III')

   wherein X3 represents a first sequence of amino acids, FI represents a fluorescent moiety which is conjugated to X3 (or X4 in III'), N2 represents an amino acid residue which is acetylated by the action of the acetyl transferase, X4 represents a second sequence of amino acids and **M** represents a fluorescence lifetime modulator which is conjugated to X4 (or X3 in III').
   The substrate may be one of the following fluorescent-modulated peptides:
   Peptide **16: W**QTARK(Me)STGGK¹⁴APRK(**9AA**)QLATK-CONH₂
   Peptide 17:**9AA**-STGGK¹⁴APR**W**QLATK-CONH₂
(3) Substrates for deiminase assays

   **FI -** X5-R-X6- **M** (V),

   or

   **M -** X5-R-X6- **FI** (V')

   wherein X5 represents a first sequence of amino acids, **FI** represents a fluorescent moiety which is conjugated to X5 (or X6 in V'), R represents an arginine residue which is converted to citrulline by the action of the deiminase, X6 represents a second sequence of amino acids and **M** represents a fluorescence lifetime modulator which is conjugated to X6 (or X5 in V').
   The substrate may be one of the following fluorescent-modulated peptides:
   Peptide 1: **9AA**-GSTRGSGHWKK-CONH₂
   Peptide 3: K(**9AA**)-HQSTRGSGH**W**KK-CONH₂
(4) Substrates for demethylase enzymes

   **FI -** X7-N3(Me)-X8- **M** (VII),

   or

   **M -** X7-N3(Me)-X8- **FI** (VII')

   wherein X7 represents a first sequence of amino acids, **FI** represents a fluorescent moiety which is conjugated to X7 (or X8 in VII'), N3(Me) represents a methylated amino acid residue (e.g. methylated lysine or methylated arginine) which is demethylated by the action of the demethylase, X8 represents a second sequence of amino acids and **M** represents a fluorescence lifetime modulator which is conjugated to X8 (or X7 in VII').
   The substrate may be one of the following fluorescent-modulated peptides:
   Peptide 10: **9AA-**ATGGVK³⁶(Me)K(Me)PHRY**W**-CONH₂ or
   Peptide 11: **9AA**-ATGGVK³⁵(Me)KPH**W**-CONH₂
(5) Substrates for deacetylase enzymes
**FI -** X9-N4(Ac)-X10- **M** (IX),

or

**M -** X9-N4(Ac)-X10- **FI** (IX')

wherein X9 represents a first sequence of amino acids, **FI** represents a fluorescent moiety which is conjugated to X9 (or X10 in IX'), N4(Ac) represents a acetylated amino acid residue (e.g. acetylated lysine) which is deacetylated by the action of the deacetylase, X10 represents a second sequence of amino acids and **M** represents a fluorescence lifetime modulator which is conjugated to X10 (or X9 in IX').

The substrate may be the following fluorescent-modulated peptide:
Ac-**Trp**-Xaa-**Lys(Ac)**-Lys(**9AA**)
Xaa = any amino acid
or a peptide such as:
Peptide **18:** Ac-IWK(Ac)K(**9AA**)-CONH₂

In the above-described fluorescent-modulated substrates, the moieties **FI** and **M** may form one of the following non-limiting combinations:
the fluorescent moiety 9-aminoacridine (9-AA) with a modulator moiety which is an indolyl moiety, in particular the indolyl side-chain of a tryptophan residue;
the fluorescent moiety 9-aminoacridine (9-AA) with the modulator moiety which is naphthylalanine or a derivative thereof;
the fluorescent moiety 9-aminoacridine (9-AA) with the modulator moiety which is a carbazole moiety or a derivative thereof;
the fluorescent moiety 9-aminoacridine (9-AA) with the modulator moiety phenothiazine or a derivative thereof;
a fluorescent moiety which is acridone or a derivative thereof with a modulator moiety which is an indolyl moiety, in particular the indolyl side-chain of a tryptophan residue; a fluorescent moiety which is quinacridone or a derivative thereof with a modulator moiety which is an indolyl moiety, in particular the indolyl side-chain of a tryptophan residue.

### Kits

A further aspect of the invention relates to kits for carrying out the assay methods described herein. The kits typically comprise an enzyme substrate as defined herein and also a supply of second enzyme (e.g. protease) which cleaves either the substrate or a modified form of the substrate, formed by action of a modifying enzyme on the substrate, between the fluorescent moiety and the fluorescence lifetime modulator moiety, thereby separating a portion of the substrate conjugated to the fluorescent moiety from a portion of the substrate conjugated to the fluorescence lifetime modulator moiety. The kit may additionally contain suitable enzyme reaction buffers, etc., both for the reaction of the modifying enzyme with the substrate, and the reaction of protease with the substrate (or modified form of the substrate).

In particular, the following kits are provided:
(1) Kits for methyltransferase assays
   The kit comprises a fluorescent-modulated substrate having the general structure:

   **FI -** X1-N1-X2- **M** (I),

   or

   **M -** X1-N1-X2- **FI** (I')

   wherein X1 represents a first sequence of amino acids, **FI** represents a fluorescent moiety which is conjugated to X1 (or X2 in I'), N1 represents an amino acid residue which is methylated by the action of the methyl transferase, X2 represents a second sequence of amino acids and **M** represents a fluorescence lifetime modulator which is conjugated to X2 (or X1 in I'); and a protease enzyme which is capable of cleaving said substrate into at least a first portion comprising fluorescent moiety **FI** and a second portion comprising fluorescence lifetime modulator **M.**
   In certain embodiments the kit contains the following fluorescent-modulated peptide:
   Peptide **4: 9AA**-TARK⁹STG**W**-CONH₂. Kits also described herein may contain one of:
   Peptide **5:** K(**9AA**)QT**A**RK⁹STG**W**-CONH₂
   Peptide **6:** ARTK(**9AA**)QTARK⁹STGG**W**-CONH₂
   Peptide **7:** ART**W**QTARK⁹STGGK(**9AA**)-CONH2
   Peptide **8: W**QTARK⁹STGGK(**9AA**)-CONH₂
   Peptide **9:** K(**9AA**)ARTK(Me)QTARK⁹STGG**W**-CONH₂
   Peptide **12: W**ARTK⁴QTARK(**9AA**)STGGKAPRKQLAK-CONH₂
   Peptide **13: W**RTK⁴QTARK(**9AA**)STGGKAPRKQLAK-CONH₂
   Peptide **14: W**SGR³GKGGK(**9AA**)GLGKGGAKRHRK-CONH₂
   Peptide **15:** Ac-**W**SGR³GKGGK(**9AA**)GLGKGGAKRHRK-CONH₂
   Peptide **20:** PRKQLATK(**9AA**)AARK²⁷SAPATGG**W**-CONH₂
   and a supply of the protease Endo-LysC or Endo-ArgC.
(2) Kits for acetyltransferase assays
   The kit comprises a fluorescent-modulated substrate having the general structure:

   **FI -** X3-N2-X4- **M** (III),

   or

   **M -** X3-N2-X4- **FI** (III')

   wherein X3 represents a first sequence of amino acids, **FI** represents a fluorescent moiety which is conjugated to X3 (or X4 in III'), N2 represents an amino acid residue which is acetylated by the action of the acetyl transferase, X4 represents a second sequence of amino acids and **M** represents a fluorescence lifetime modulator which is conjugated to X4 (or X3 in III'); and a protease enzyme which is capable of cleaving said substrate into at least a first portion comprising fluorescent moiety **FI** and a second portion comprising fluorescence lifetime modulator **M.**
   The kit may contain one of the following fluorescent-modulated peptide substrates:
   Peptide **16: W**QTARK(Me)STGGK¹⁴APRK(**9AA**)QLATK-CONH₂
   Peptide **17:9AA**-STGGK¹⁴APR**W**QLATK-CONH₂
   and a supply of the protease Endo-LysC.
(3) Kits for deiminase assays
   The kit comprises a fluorescent-modulated substrate having the general structure:

   **FI -** X5-R-X6- **M** (V),

   or

   **M -** X5-R-X6- **FI** (V')

   wherein X5 represents a first sequence of amino acids, FI represents a fluorescent moiety which is conjugated to X5 (or X6 in V'), R represents an arginine residue which is converted to citrulline by the action of the deiminase, X6 represents a second sequence of amino acids and **M** represents a fluorescence lifetime modulator which is conjugated to X6 (or X5 in V'); and a protease enzyme which is capable of cleaving said substrate into at least a first portion comprising fluorescent moiety **Fl** and a second portion comprising fluorescence lifetime modulator **M.**
   The kit may comprise one of the following fluorescent-modulated peptide substrates:
   Peptide 1: **9AA**-OSTRGSGHWKK-CONH₂.
   Peptide 3: K(**9AA**)-HQSTRGSGHWKK-CONH₂
   and a supply of the protease trypsin.
(4) Kits for demethylase enzymes
   The kit comprises a fluorescent-modulated substrate having the general structure:

   **FI -** X7-N3(Me)-X8- **M** (VII),

   or

   **M -** X7-N3(Me)-X8- **FI** (VII')

   wherein X7 represents a first sequence of amino acids, FI represents a fluorescent moiety which is conjugated to X7 (or X8 in VII'), N3(Me) represents a methylated amino acid residue (e.g. methylated lysine or methylated arginine) which is demethylated by the action of the demethylases, X8 represents a second sequence of amino acids and **M** represents a fluorescence lifetime modulator which is conjugated to X8 (or X7 in VII'); and a protease enzyme which is capable of cleaving a modified form of said substrate in which residue N3(Me) is demethylated into at least a first portion comprising fluorescent moiety **FI** and a second portion comprising fluorescence lifetime modulator **M.**
   The kit may comprise one of the following fluorescent-modulated peptide substrate:
   Peptide 10: **9AA**-ATGGVK³⁶(Me)K(Me)PHRY**W**-CONH₂
   and a supply of the protease Endo-LysC.
   The kit may comprise one of the following fluorescent-modulated peptide substrate:
   Peptide 11: **9AA**-ATGGVK³⁵(Me)KPH**W**-CONH₂
   and a supply of the protease trypsin.
(5) Kits for deacetylase enzymes
   The kit comprises a fluorescent-modulated substrate having the general structure:

   **FI -** X9-N4(Ac)-X10- **M** (IX),

   or

   **M**-X9-N4(Ac)-X10**-Fl** (IX')

   wherein X9 represents a first sequence of amino acids, **FI** represents a fluorescent moiety which is conjugated to X9 (or X10 in IX'), N4(Ac) represents a acetylated amino acid residue (e.g. acetylated lysine) which is deacetylated by the action of the deacetylase, X10 represents a second sequence of amino acids and **M** represents a fluorescence lifetime modulator which is conjugated to X10 (or X9 in IX'); and a protease enzyme which is capable of cleaving a modified form of said substrate in which residue N4(Ac) is deacetylated into at least a first portion comprising fluorescent moiety **FI** and a second portion comprising fluorescence lifetime modulator **M.**

The kit may comprise the following fluorescent-modulated peptide substrate:
Ac-**Trp**-Xaa-**Lys(Ac)**-Lys(**9AA**)
Xaa = any amino acid
or a peptide such as:
Peptide **18:** Ac-IWK(Ac)K(9AA)-CONH₂
and a supply of trypsin.

In the above-described kits, the moieties **FI** and **M** in the fluorescent-modulated substrate may form one of the following non-limiting combinations:
the fluorescent moiety 9-aminoacridine (9-AA) with a modulator moiety which is an indolyl moiety, in particular the indolyl side-chain of a tryptophan residue;
the fluorescent moiety 9-aminoacridine (9-AA) with the modulator moiety which is naphthylalanine or a derivative thereof;
the fluorescent moiety 9-aminoacridine (9-AA) with the modulator moiety which is a carbazole moiety or a derivative thereof;
the fluorescent moiety 9-aminoacridine (9-AA) with the modulator moiety phenothiazine or a derivative thereof;
a fluorescent moiety which is acridone or a derivative thereof with a modulator moiety which is an indolyl moiety, in particular the indolyl side-chain of a tryptophan residue;
a fluorescent moiety which is quinacridone or a derivative thereof with a modulator moiety which is an indolyl moiety, in particular the indolyl side-chain of a tryptophan residue.

### Examples

The invention will be further understood with reference to the following experimental examples:

### Example 1 - FLT Protease Protection Assay for Deiminases

A means of assaying the deiminase class of enzymes, which convert arginine residues in peptide/protein substrates to citrulline, via a FLT protease protection approach is shown in Fig. 1. Here, the substrate lifetime of 9-aminoacridine (9AA) (or another suitable reporter) is modulated by the presence of a tryptophan residue in the sequence (or another known modulator of fluorescence lifetime). Treatment of the substrate with a protease that cleaves after arginine will induce proteolysis and remove modulation of the fluorescence lifetime by the tryptophan residue. After arginine conversion to citrulline by an appropriate deiminase enzyme, then treatment of the product peptide with the protease does not induce cleavage after the arginine and the lifetime of the fluorophore (9AA) remains modulated.

### (A) PAD4 Proof-of-Concept FLT Protease Protection Assay

Recombinant human PAD4 enzyme is commercially available from suppliers such as origene, abnova or modiquest research

To realise the FLT protease protection approach for deiminases, a number of conditions must be satisfied:
1) The design of an appropriate peptide substrate containing a recognition sequence for the deiminase, a suitably incorporated tryptophan residue (or other FLT modulator) and a long lifetime fluorophore (e.g. 9-aminoacridine).
2) The correct placement of the modulator and fluorophore, such that the FLT of the latter is sufficiently reduced in the intact peptide. The modulator and fluorophore must be located on opposite fragments after protease cleavage to result in a cleavage dependent increase in FLT of the fluorophore.
3) The use of a suitable protease that selectively cleaves the peptide C-terminal to the arginine residue that is citrullinated. The protease must be unable to cleave the citrullinated product.

In order to meet requirements 1-3, the peptide sequences shown in Fig. 2 were designed and synthesised for assaying the deiminase PAD4 by an FLT protease protection approach.
Peptide **1:** 9AA-QSTRGSGHWKK-CONH₂
Peptide **2:** K(9AA)-QSTRGSGHWKK-CONH₂
Peptide **3:** K(9AA)-HQSTRGSGHWKK-CONH₂

Three variants were synthesised in order to increase the probability of obtaining suitable FLT modulation whilst maintaining activity against PAD4.

The peptides were synthesised by standard Fmoc solid phase peptide synthesis (SPPS) as described in the Experimental section. Peptides **2** and **3** included Boc-Lys(Fmoc)-OH at the N-terminus to enable orthogonal side chain deprotection. Peptides were labelled with 3-(9-aminoacridin-2-yl)-propionic acid on resin using HOCt/DIC activation and then cleaved using a standard TFA cleavage cocktail. Peptides were purified by reverse-phase HPLC and aliquoted with reference to the molar extinction coefficient for 9AA (8735 M⁻¹cm⁻¹at 405 nm in 1:1 MeCN/water)

### FLT Measurements of Peptide 1-3

Peptides 1-3 were dissolved in PAD4 assay buffer (20 mM Tris pH 8.0, 200 µM CaCl₂, 5 mM DTT, 0.01 % CHAPS) at 1 µM concentration in a 384-well black microplate (20 µl per well). FLTs were measured using a NanoTaurus Fluorescence Lifetime Plate Reader (Edinburgh Instruments Ltd.) as described in the Experimental section. Measurements were performed in triplicate and analysed over a 7 h period.

After initial stabilisation, the FLTs of peptides **1** and **3** remain stable over time (Fig. 3). In contrast, the FLT of peptide **2** steadily increases over 3 h before beginning to plateau. The reduced starting lifetime of peptide **3** compared to peptides **1** and **2** reflects the presence of a histidine residue adjacent to the site of attachment of 9AA. Like tryptophan, histidine residues are known to modulate the FLT of fluorophores (Marmé, N., Knemeyer, J-P., Sauer, M., and Wolfrum, J., Bioconjugate Chem., 2003, 14, 1133-1139).

### Protease Cleavage of Peptides 1-3

Trypsin is a commonly available protease that cleaves C-terminal to arginine or lysine residues in peptides and proteins. The designed peptides **1-3** (Fig. 2) have a single arginine residue and two lysine residues at the C-terminus. It is anticipated that cleavage at arginine will lead to an increase in FLT of 9AA due to its separation from the modulating tryptophan residue. In contrast, and important for the assay's utility, cleavage at lysine will not lead to the separation of dye and modulator, and hence, should have no effect on the FLT of the former.

To test the effects of protease cleavage on the FLT of peptides **1-3,** each peptide was dissolved in PAD4 assay buffer (20 µl) at 1 µM concentration in a 384-well black microplate. To each well was added 10 µl of trypsin solution containing 5 nM, 2.5 nM, 1.25 nM, or 0.625 nM enzyme respectively. The FLTs were measured using a NanoTaurus plate reader at regular intervals over 45 min. As a control, the corresponding peptides containing citrulline in place of arginine were tested in an analogous manner.

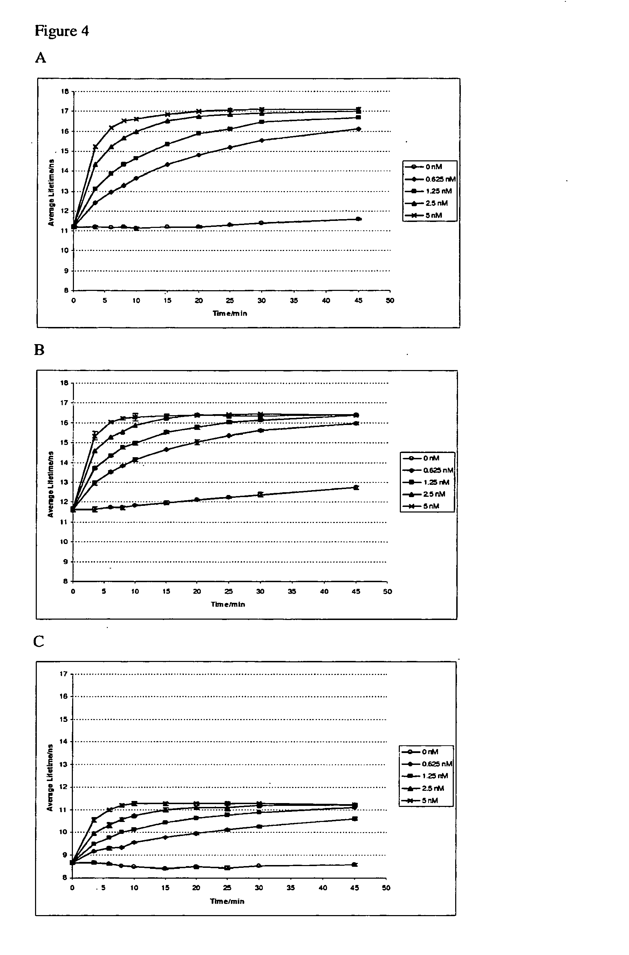

The FLT of all three peptides increased as a function of trypsin concentration, consistent with separation of 9AA from the modulating tryptophan residue (Fig. 4A-C). The FLT increase was peptide dependent such that peptide **1** (6 ns) > peptide **2** (4.8 ns) > peptide **3** (2.6 ns). It should be noted that there was some drift in the no trypsin control for peptide **2,** consistent with the effects observed for this peptide in buffer alone (Fig. 3). The reduced FLT range for peptide **3** can be partly explained by the continuing presence of the modulating histidine residue adjacent to 9AA after cleavage.

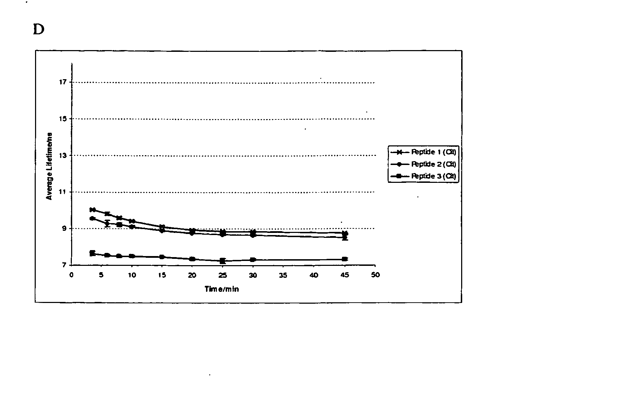

In contrast to the significant increases in FLT observed for peptides **1-3** after trypsin treatment, no FLT increases were observed for the citrullinated analogues, even after 45 min incubation (Fig. 4D). This finding is consistent with the expected inability of trypsin to cleave adjacent to citrulline residues.

With the protease protection aspect of the assay validated, attention turned to determining if peptides **1-3** were viable substrates for PAD4.

### PAD4 Assays with Peptides 1-3

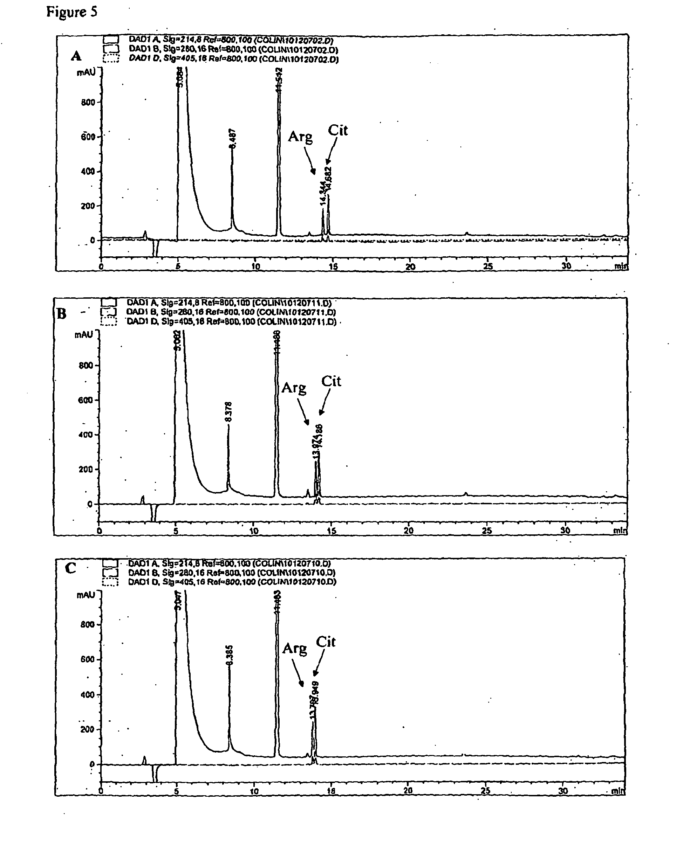

In order to establish if peptides **1-3** were citrullinated by PAD4, a reverse-phase HPLC method was developed to monitor the consumption of substrate and formation of product. As a first step, 1:1 mixtures of each peptide and its citrullinated equivalent were prepared at 15 µM concentration in PAD4 assay buffer and monitored by RP-HPLC using a Luna C18 column and 0-73 % MeCN + 0.1 % TFA gradient over 30 mins. Using this gradient it was possible to separate peaks corresponding to substrate and product peptides, both of which had later retention times than peaks arising from buffer components, thereby simplifying the analysis (Fig. 5).

With a suitable HPLC method in hand, PAD4 assays were set up for each of the peptide substrates and aliquots taken at regular time intervals with product formation monitored by RP-HPLC. Each assay was performed in a micro-centrifuge tube containing 15 µM peptide and 30 nM PAD4 (Origene) in assay buffer (300 µl). The assays were performed at room temperature and samples taken at 0 min, 30 min, and 60 min time intervals.

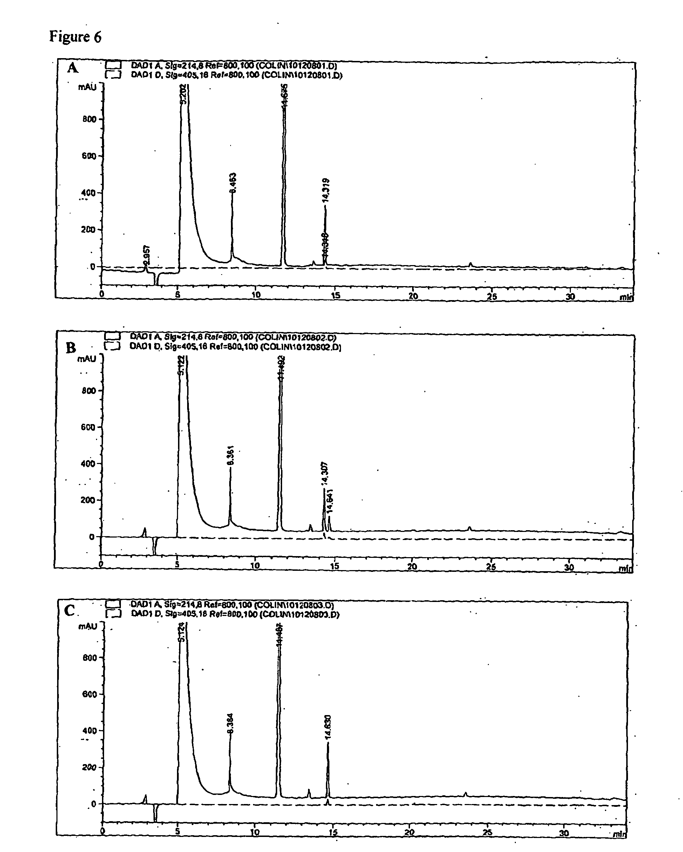

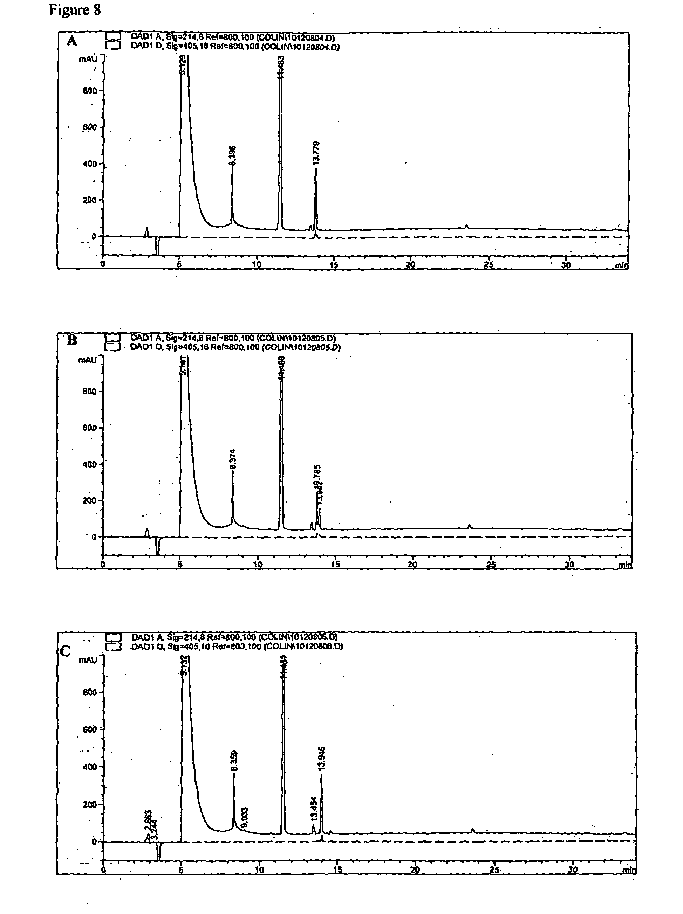

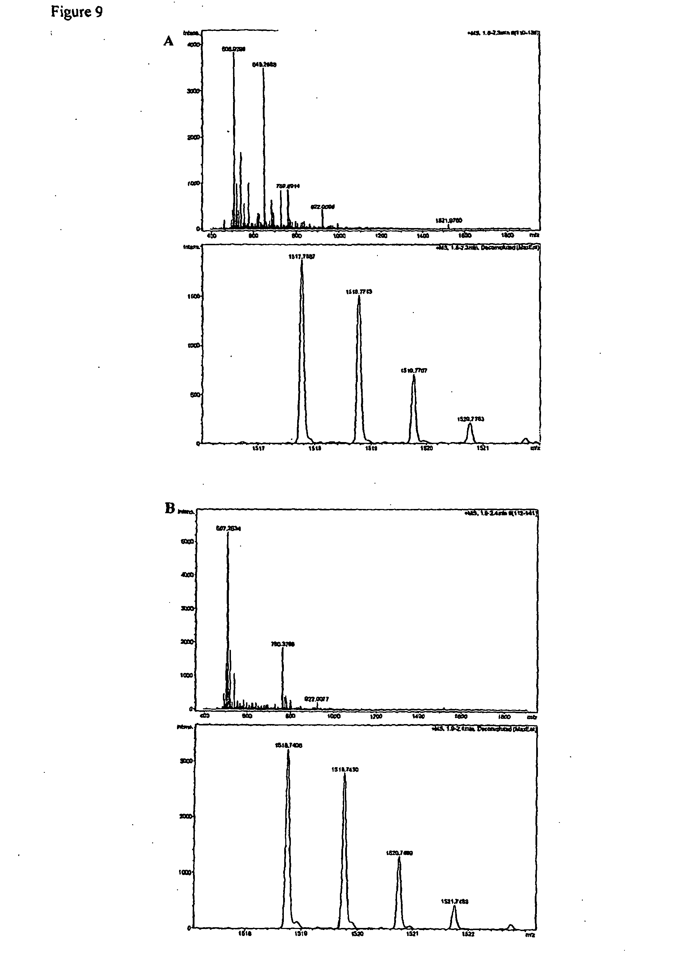

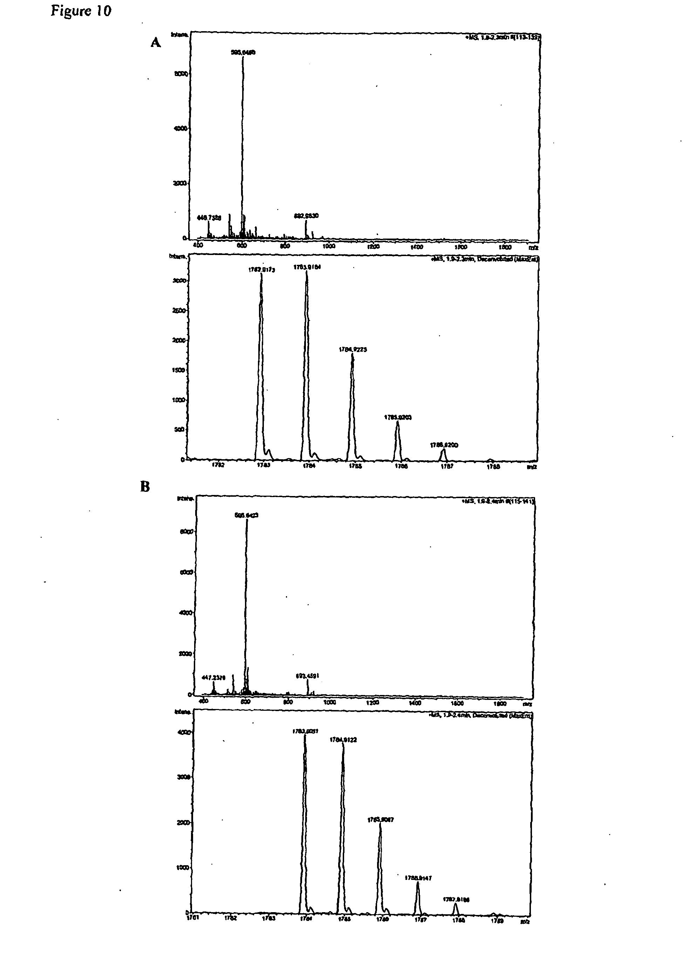

Each assay's progress over the course of 1 h is shown via the HPLC spectra in Fig. 6-8. For the assays with peptide **1** (Fig. 6) and peptide **3** (Fig. 8) as substrates, the spectra clearly show the formation of a new peak with later retention time than the substrate after 30 mins, with almost complete disappearance of substrate evident after 1 h. To confirm that the newly formed species corresponded to citrullinated product, the peaks were collected and analysed by ESI-MS. This method confirmed the masses to be 1 amu greater than the starting peptides in each case, consistent with the conversion of arginine to citrulline (Fig. 9 and 10).

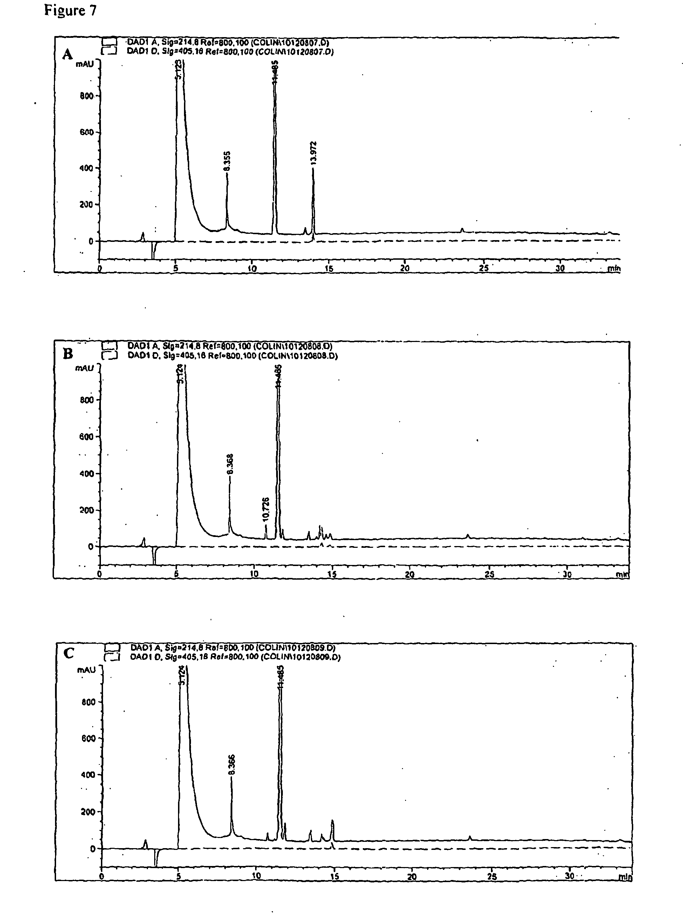

In contrast to the success with peptides **1** and **3,** the assay using peptide **2** as substrate resulted in decomposition of the peptide as evidenced by the appearance of numerous peaks in the HPLC spectrum over the assay time course (Fig. 7). No discernible mass could be obtained for the breakdown peaks so it was not possible to confirm if citrullination had occurred. The apparent failure of this peptide as a substrate is consistent with the lack of stability observed during FLT studies (Fig. 3) and during the protease cleavage analysis (Fig. 4B). Further studies using peptide **2** were not performed, with attention concentrated on peptides **1** and **3** that had displayed activity in the PAD4 assay.

In order to test if citrullination of peptides **1** and **3** by PAD4 had conferred protection against trypsin, the assay mixtures for these peptides (described above) were diluted to 1 µM concentration with assay buffer and 20 µl of each added to three wells of a 384-well black microplate. The FLTs were measured using the NanoTaurus plate reader as described previously. 10 µl of trypsin (final concentration 10 nM) was then added to each well and the plate incubated at room temperature for 10 mins before the FLTs were re-measured. Peptide only controls were included at the same concentration for comparison.

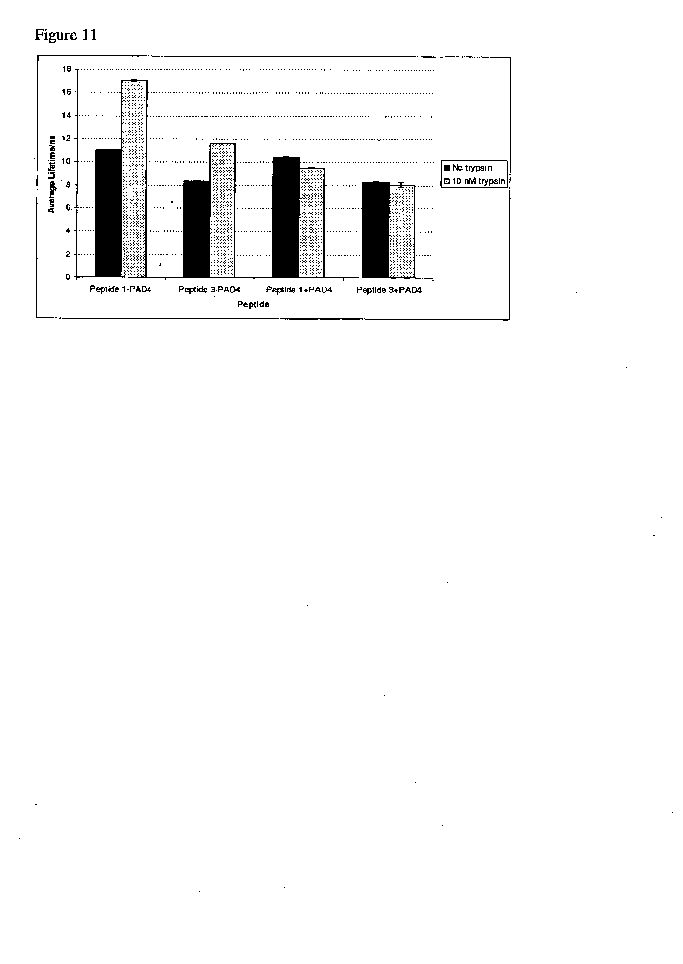

In the absence of PAD4, the FLT of peptides **1** and **3** increased by 6.1 ns and 3.3 ns respectively after incubation with 10 nM trypsin (Fig. 11). In contrast, no increase in FLT was observed for those peptides exposed to PAD4 prior to incubation with trypsin. These findings are consistent with PAD4 catalysed citrullination of the substrates offering protection to protease treatment.

In summary, the following conclusions can be drawn:
1) Peptides **1** and **3** are viable substrates for PAD4.
2) FLT increases of up to 6 ns (in the case of peptide 1) are achievable after treatment with a suitable protease i.e. trypsin, thereby affording an excellent assay dynamic range.
3) Citrullination of peptides **1** and **3** by PAD4 confers protection from protease-induced cleavage, as reflected by a lack of increase in FLT.

### FLT Protease-Protection Assay for PAD4

Due to the significantly larger FLT change exhibited by peptide 1 compared to peptide **3,** this peptide was chosen as the substrate for the microplate assay. Assays were performed in a 20 µl volume in a 384-well black microplate and contained peptide 1 (1 µM) and PAD4 enzyme (500 pM, 250 pM, or 125 pM) in assay buffer. Reactions were stopped at regular intervals by the addition of 10 µl trypsin (10 nM). FLTs were measured immediately prior to trypsin addition, and again after 10 min incubation at room temperature. All measurements were performed in triplicate using a NanoTaurus plate reader.

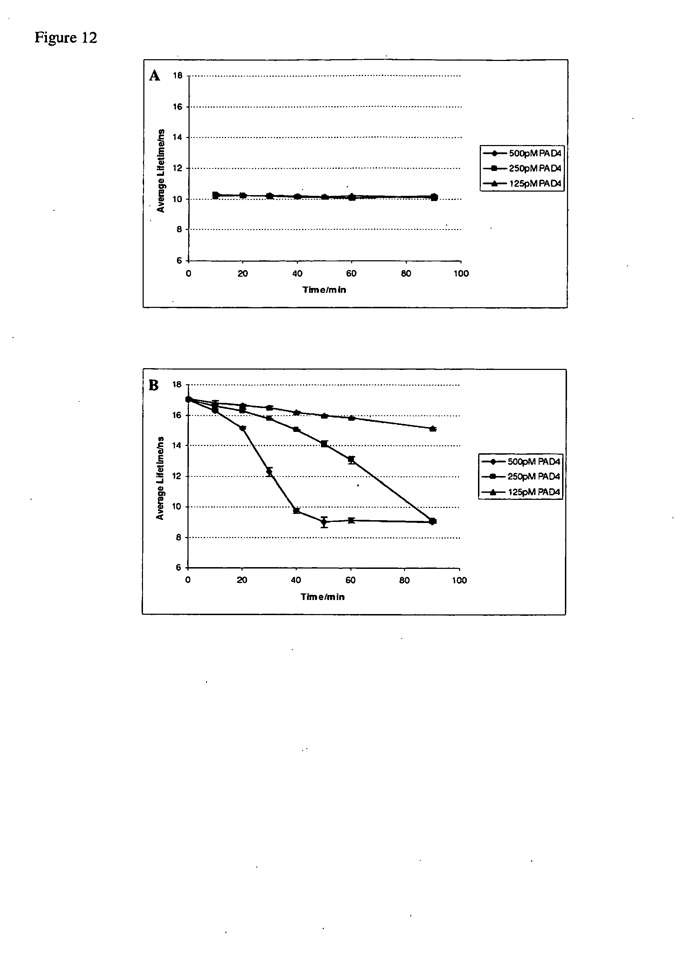

Prior to the addition of trypsin, the FLT of all assays wells were similar (~ 10 ns) with no significant variation observed for different assay time points or PAD4 concentrations (Fig. 12A). In contrast, FLTs measured after incubation with trypsin displayed time and PAD4 concentration dependent decreases (Fig. 12B), as progressive citrullination conferred protease protection, resulting in continued modulation of the FLT of peptide 1 by the intrinsic tryptophan residue.

### Substrate Titration

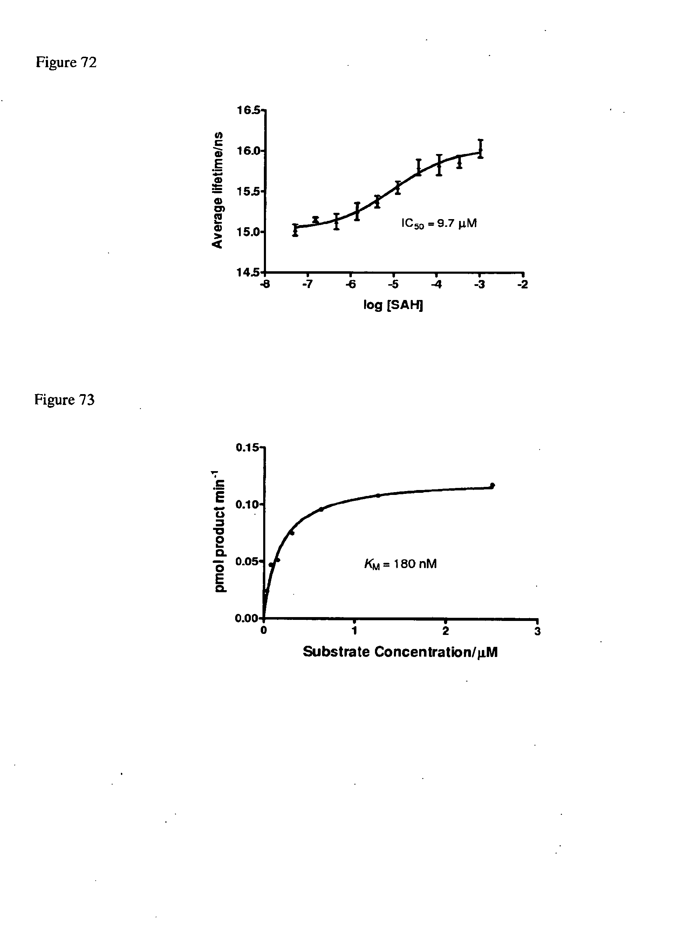

The dependence of the PAD4 assay on peptide substrate concentration was investigated. The assay was performed in a 384-well microplate at room temperature for 1 h in assay buffer (20 µl) using 250 pM PAD4. Concentrations of peptide 1 were varied from 2.5 µM - 0.04 µM. On completion of the reaction, trypsin (10 nM) was added to each well and FLTs measured following a 10 min incubation period. The data was converted to rate of product formation by reference to a standard curve and then fitted using a Michaelis-Menten model in GraphPad Prism to determine the substrate *K*_{M} From Fig. 73, a *K*_{M} of 180 nM was determined for substrate **1.**

### Z'-Factor

To demonstrate that the PAD4 FLT assay was compatible with high-throughput screening (HTS), a value for the Z'-factor was determined. The Z'-factor is a measure of a particular assay's robustness, with values > 0.7 considered excellent (Zhang, J. H.; Chung, T. D.; Oldenburg, K. R. J. Biomol. Screen. 1999, 4, 67-73).

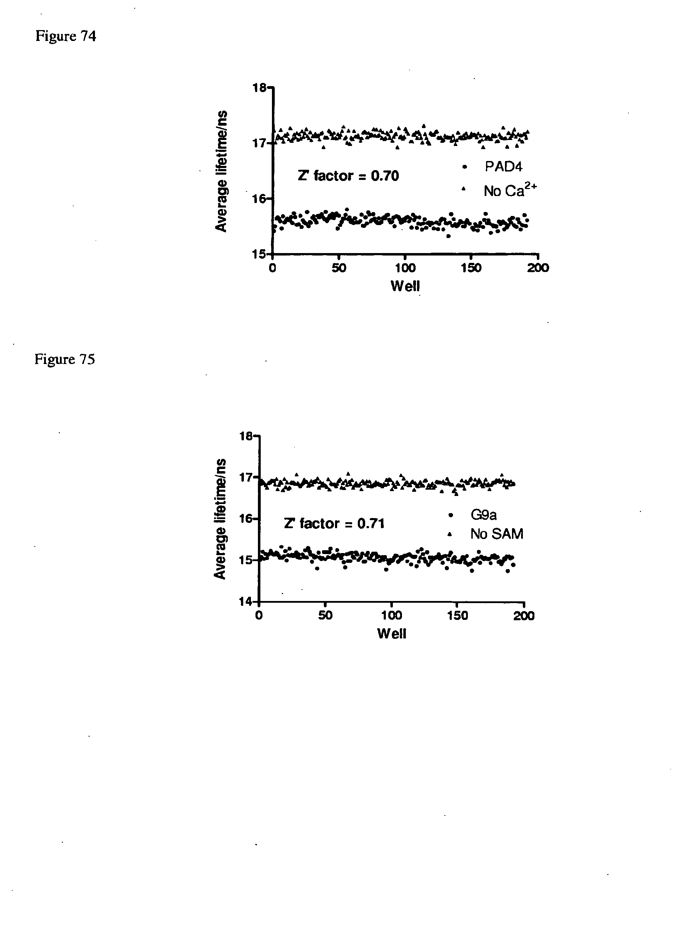

Assays were set up in a 384-well microplate, with half the plate containing PAD4 (125 pM) and peptide 1 (200 nM) in assay buffer, whereas the other half contained PAD4 (125 pM) and peptide 1 (200 nM) in assay buffer without CaCl₂. Final assay volume was 20 µl and the plate was incubated at room temperature for 1 h at which point trypsin (10 nM final concentration) in assay buffer (10 µl) was added to each well. Following a further 10 min incubation at room temperature the FLTs were measured and a Z'-factor of 0.70 calculated (Fig. 74).

These results demonstrate that the FLT protease protection assay for deiminases is transferable to a microplate format, facilitating its use for drug screening and HTS applications.

In conclusion, an FLT protease protection assay for the deiminase class of enzymes, employing PAD4 as a representative example has been demonstrated. Peptide substrates were designed that incorporated a PAD4 recognition sequence, 9-aminoacridine fluorophore, and modulating tryptophan residue. FLT studies confirmed the lifetime of 9AA to be reduced whilst the peptide sequence was intact, whereas upon treatment with the protease trypsin, the FLT increased as a result of cleavage of the peptide causing liberation of the dye from the modulator. Two of the peptides were shown to be substrates for PAD4 in an *in vitro* assay, with citrullination of the single arginine residue in the sequence resulting in protection from protease-induced cleavage. This protection was quantified by a reduced FLT for those peptides exposed to PAD4 compared to controls which contained no enzyme.

It is envisaged that this assay technology will be transferable to other deiminase enzymes by employing appropriately designed substrates.

### (B) FLT Protease Protection Assay for PAD2 Deiminase

In a further exemplification of the FLT protease protection approach to assaying deiminases, peptidylarginine deiminase 2 (PAD2) was validated as a suitable target.

Dysregulation of PAD2 has been implicated in the autoimmune diseases multiple sclerosis and rheumatoid arthritis as well as neurodegenerative disorders such as Alzheimer's disease and Creutzfeldt-Jakob disease (Jang, B. et al., Acta Neuropathologica, 2010, 119, 199 to 210)

Recombinant human PAD2 is commercially available from suppliers such as Modiquest Research.

Peptide 1 (Fig. 1), previously confirmed as a substrate for PAD4, was tested in an assay with PAD2 with conversion to citrullinated product monitored by RP-HPLC and ESI-MS.
Peptide **1: 9AA**-QSTRGSGH**W**KK-CONH₂

Accordingly, peptide **1** (15 µM) was incubated with PAD2 (30 nM) in assay buffer (20 mM Tris pH 8, 200 µM CaCl2, 5 mM DTT, 0.01 % CHAPS) (300 µl) and aliquots taken at regular time intervals and analysed by RP-HPLC. The assays were performed at room temperature and samples taken at 0 min, 30 min, and 120 min time intervals

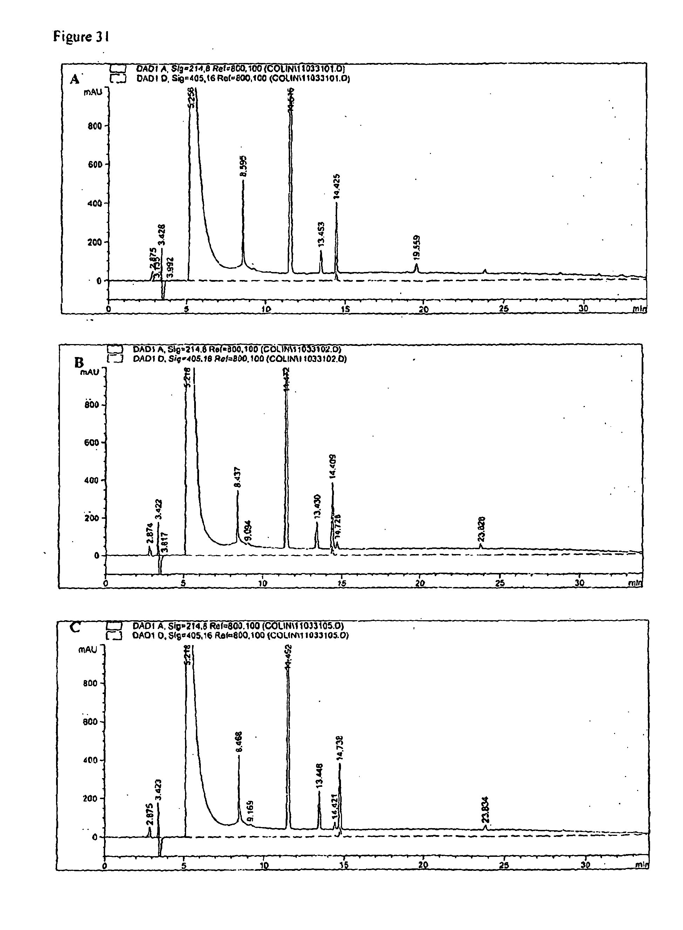

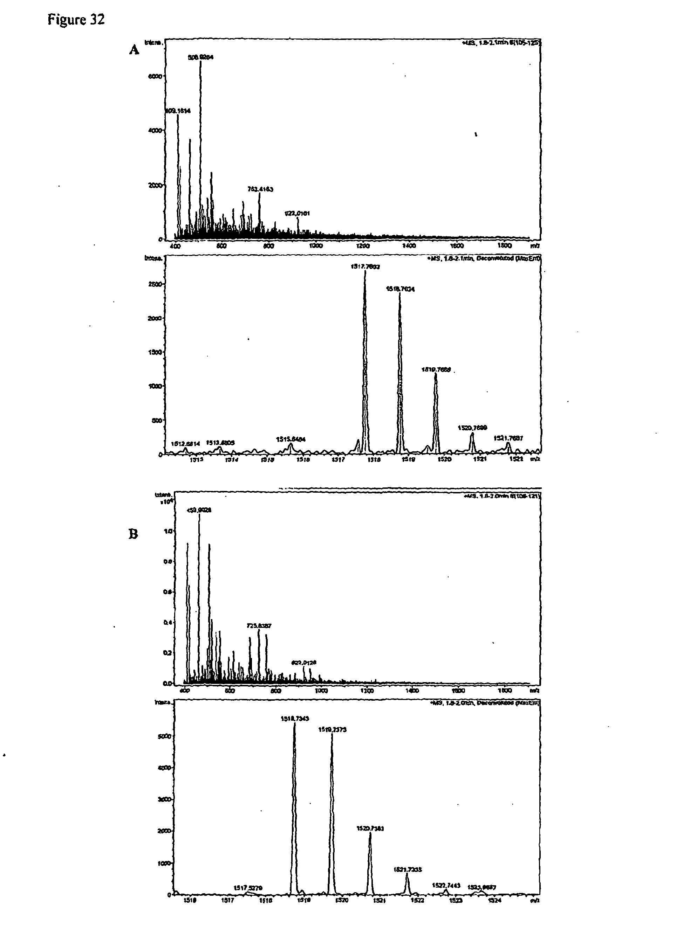

The assay's progress over the course of 2 h is shown via the HPLC spectra in Fig. 31. At t = 0, only a peak corresponding to the peptide substrate is present (t_{R} = 14.4 min). After 30 min a new peak with 405 nm absorbance is present (t_{R} = 14.7 min), and after 2 h this peak has grown predominant with only a small peak for substrate detectable. To confirm that the new peak corresponded to citrullinated product, it was collected and analysed by ESI-MS. The mass found (*m*/*z* 1518.7) was 1 amu greater than the substrate mass, consistent with conversion of the guanidinyl group of the arginine residue to urea (Fig. 32).

In order to test if citrullination of peptide **1** by PAD2 had conferred protection against trypsin, the assay mixture was diluted to 1 µM concentration with assay buffer and 20 µl added to three wells of a 384-well black microplate. 10 µl of trypsin (final concentration 10 nM) was added to each well and the plate incubated at room temperature for 10 mins before the fluorescence lifetimes (FLTs) were measured using the NanoTaurus plate reader as described in the Experimental section. Peptide only controls were included at the same concentration for comparison.

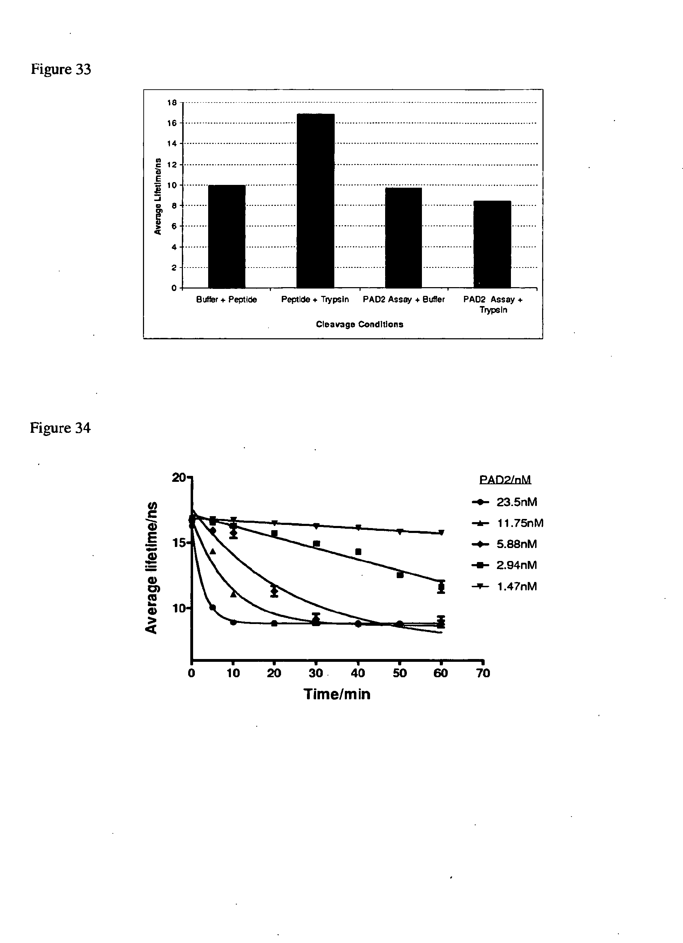

In the absence of PAD2, the FLT of peptide **1** increased by 6.9 ns after incubation with 10 nM trypsin (Fig. 33). In contrast, no increase in FLT was observed for peptide **1** exposed to PAD2 prior to incubation with trypsin. These findings are consistent with PAD2 catalysed citrullination of peptide **1** offering protection to protease treatment.

In summary, peptide **1** is a valid substrate for assaying PAD2, with activity monitored by a decrease in FLT as a result of citrullination conferring protection to protease-induced cleavage.

### FLT Protease-Protection Assay for PAD2

Next, the FLT protease protection assay for PAD2 was demonstrated in a microplate format. Assays were performed in a 20 µl volume in a 384-well black microplate and contained peptide 1 (1 µM) and PAD2 enzyme (23.5 nM, 11.8 nM, 5.9 nM, 2.9 nM, or 1.5 nM) in assay buffer. Reactions were stopped at regular intervals by the addition of 10 µl trypsin (10 nM). FLTs were measured following 10 min incubation with trypsin at room temperature. All measurements were performed in triplicate using a NanoTaurus plate reader.

FLTs measured after incubation with trypsin displayed time and PAD2 concentration dependent decreases (Fig. 34), as progressive citrullination conferred protease protection, resulting in continued modulation of the FLT of peptide 1 by the intrinsic tryptophan residue. Citrullination is therefore reported by a decrease in the measured fluorescence lifetime of the assay.

These results demonstrate that the FLT protease protection assay for PAD2 is transferable to a microplate format, facilitating its use for drug screening and HTS applications.

### Example 2 - FLT Protease Protection Assay for Protein Methyltransferases

A means of assaying protein methyltransferases (PMT), which transfer one or more methyl groups to lysine or arginine residues in peptide/protein substrates, via a FLT protease protection approach is shown in Fig. 13. Here, the substrate lifetime of 9-aminoacridine (9AA) (or another suitable reporter) is modulated by the presence of a tryptophan residue in the sequence (or another known modulator of fluorescence lifetime). Treatment of the substrate with a protease that cleaves after lysine or arginine will induce proteolysis and remove modulation of the fluorescence lifetime by the tryptophan residue. After lysine or arginine methylation by an appropriate PMT enzyme, then treatment of the product peptide with the protease does not induce cleavage after the lysine or arginine and the lifetime of the fluorophore (9AA) remains modulated.

Recombinant PKMT (e.g. G9a, Set7/9, GLP, EZH2) and PRMT (PRMT1, PRMT3, PRMT4/CARM1, PRMT5) enzymes are commercially available from suppliers such as BPS Bioscience, Millipore, New England Biolabs, or Sigma-Aldrich.

Suitable proteases for the approach outlined in Fig. 13 are Endo-LysC (for assaying PKMT) and Endo-ArgC (for assaying PRMT).

### (A) G9a Proof-of-Concept FLT Protease Protection Assay

The PKMT G9a was selected as a representative example for proof of concept studies as it is a well-characterised enzyme (Chin, H. G., Pradhan, M., Esteve, P-O., Patnaik, D., Evans Jr., T. C., and Pradhan, S., Biochemistry, 2005, 44, 12998-13006) (Patnaik, D., Chin, H. G., Esteve, P-O., Benner, J., Jacobsen, S. E., and Pradhan, S., J. Biol. Chem., 2004, 279, 53247-53258) histone peptides of varying lengths have been reported as substrates (Rathert, P., Dhayalan, A., Murakami, M., Zhang, X., Tamas, R., Jurkowska, R., Komatsu, Y., Shinkai, Y., Cheng, X., and Jeltsch, A., Nat. Chem. Biol., 2008, 4, 344-346) and it is commercially available from a number of suppliers. G9a preferentially transfers a methyl group to the residue on the N-terminal tail of histone H3. Prolonged exposure to G9a can lead lysine-9 to become di- or tri-methylated. A protease protection assay for G9a has recently been reported using Caliper's microfluidic shift technology (Wigle, T. J., Provencher, L. M., Norris, J. L., Jin, J., Brown, P. J., Frye, S. V., Janzen, W. P., Chem. Biol., 2010, 17, 695-704).

In the first instance, a series of peptides were synthesised as potential substrates for G9a based on the histone H3 N-terminal sequence (Fig. 14). As was exemplified for the deiminase class of enzymes, the viability of the FLT-based protease protection approach requires the incorporation of a long lifetime fluorophore (e.g. 9AA) and a modulating residue (e.g. tryptophan) on opposite sides of the target residue (in this case lysine-9). The incorporation of these moieties must not detrimentally affect recognition by G9a, however, they must be suitably placed so that modulation of the FLT of the fluorophore is sufficient for a working assay range.
Peptide **4:** 9AA-TARK⁹TGW-CONH₂
Peptide **5:** K(9AA)QTARK⁹STGW-CONH₂
Peptide **6:** ARTK(9AA)QTARK⁹STGGW-CONH₂
Peptide **7:** ARTWQTARK⁹STGGK(9AA)-CONH₂
Peptide **8:** WQTARK⁹STGGK(9AA)-CONH₂
Peptide **9:** K(9AA)ARTK(Me)QTARK⁹STGGW-CONH₂

The peptides were synthesised by standard Fmoc solid phase peptide synthesis (SPPS) as described in the Experimental section. Peptides **5** and **9** included Boc-Lys(Fmoc)-OH whereas peptides **6-8** included Fmoc-Lys(ivDde)-OH at the site of dye attachment to enable orthogonal side chain deprotection. Peptides were labelled with 3-(9-aminoacridin-2-yl)-propionic acid on resin using HOCt/DIC activation and then cleaved using a standard TFA cleavage cocktail. Peptides were purified by reverse-phase HPLC and aliquoted with reference to the molar extinction coefficient for 9AA (8735 M⁻¹cm⁻¹ at 405 nm in 1:1 MeCN/water).

Endo-LysC was chosen as the protease as it is known to cleave C-terminal to lysine residues but not when such residues are methylated.

Assays were set up in a 384-well black microplate (Greiner) with each well containing peptide substrate (1 µM), S-adenosylmethionine (SAM) (100 µM), and G9a (Millipore, 500 nM) in 20 µl of assay buffer (20 mM Tris.HCl pH 8.0, 25 mM NaCl, 1 mM DTT, 0.025 % Tween-20). Assays were performed at room temperature for 2 h and then FLTs measured using a NanoTaurus plate reader (Edinburgh Instruments Ltd.). Each well was then treated with 2 nM protease (Endo-LysC) (10 µl) and the plate incubated at room temperature for 60 min. The FLTs were then re-measured. Controls were included containing no G9a or no protease. The results of the assay are shown in Fig. 15.

In the absence of G9a treatment, the largest protease-dependent increase in FLT was observed for peptide **4** (5.8 ns), with the smallest found for peptide **9** (2.4 ns). This observation was not unexpected, as the separation distance between 9AA and tryptophan is smallest in peptide **4** and largest in peptide **9.** Comparing the increase in FLT upon protease treatment for wells with and without G9a, it is apparent that the increase is less for those exposed to G9a for all peptides tested indicating a degree of protease protection. Peptide **9** was unique in appearing to offer complete protease protection (i.e. FLT after protease treatment remained the same as the peptide control) implying a high degree of lysine methylation. This peptide had lysine-4 pre-methylated to prevent cleavage at this residue and it may be that small modifications at this site are better tolerated by G9a than deletion or substitution of the residue. Comparing the protease protection offered by the other five peptide substrates, there appeared little difference between each of them (Fig. 15). Hence, it was decided to move forward with peptides **4** and **9** as the former offered the greatest dynamic range, whereas the latter appeared a better substrate for G9a.

### G9a Titration

Peptides **4** and **9** were incubated with SAM (100 µM) and varying concentrations of G9a (Abcam) in 20 µl of assay buffer in a 384-well black microplate (Greiner). The assays were performed at room temperature for 2 h after which FLTs were measured as previously described. 2 nM Endo-LysC (10 µl per well) was then added and FLTs re-measured following 10 min and 60 min incubation with the protease.

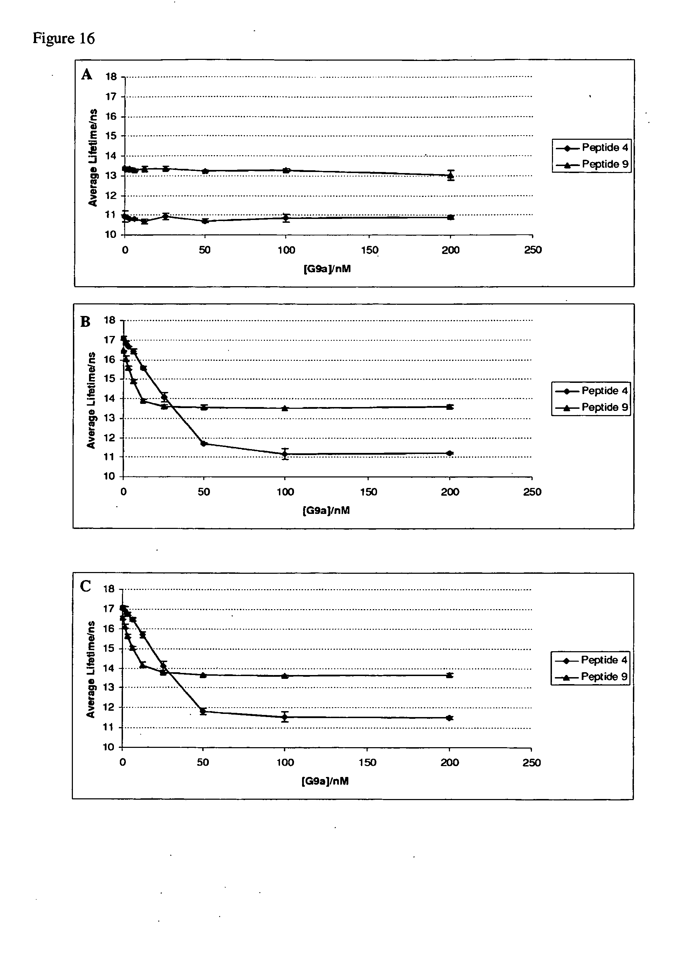

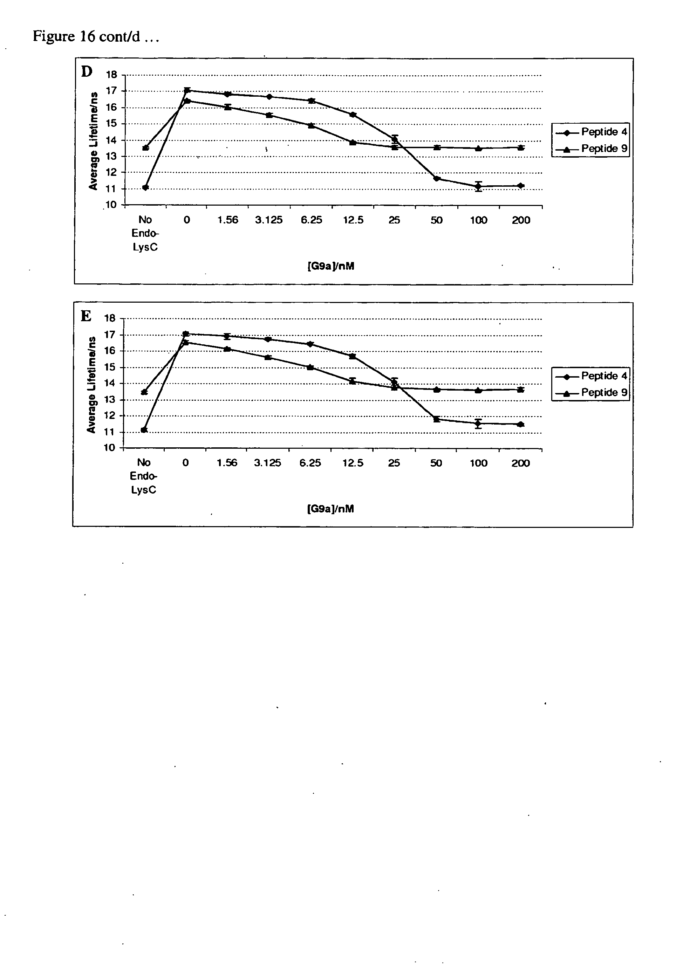

From the data shown in Fig. 16A, no change in FLT of either peptide was apparent until after the addition of protease i.e. lysine methylation does not directly induce a change in FLT. Thus, differences in FLT of assay wells compared to control wells upon protease cleavage are a direct result of G9a activity.

50 nM of G9a was sufficient to confer complete protease protection for peptides **4** and **9** (Fig. 16B-E). This concentration was ten-fold less than that used to generate similar data using G9a from Millipore (Fig. 15) emphasising the greater activity of the Abcam enzyme. The data also shows that peptide **9** is a more active substrate than peptide **4,** as evidenced by protease protection occurring at lower G9a concentrations for this peptide (Fig. 16B-E). This finding is consistent with the longer sequence of peptide **9,** compared to peptide **4,** conferring a greater degree of selectivity for G9a.

Comparing Fig. 16B with 16C and 16D with 16E, it is clear that prolonged incubation (60 min vs 10 min) with Endo-LysC does not lead to an appreciable change in FLT, indicating that complete cleavage has occurred after 10 min. Therefore, data was collected after 10 min incubation with Endo-LysC in all subsequent experiments.

### Evidence for Methylation

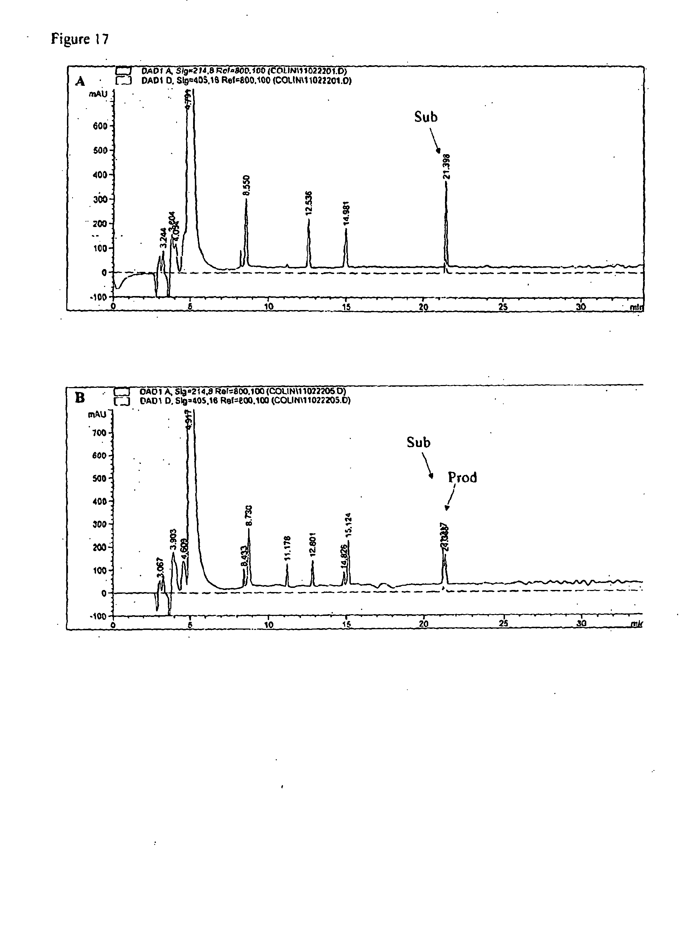

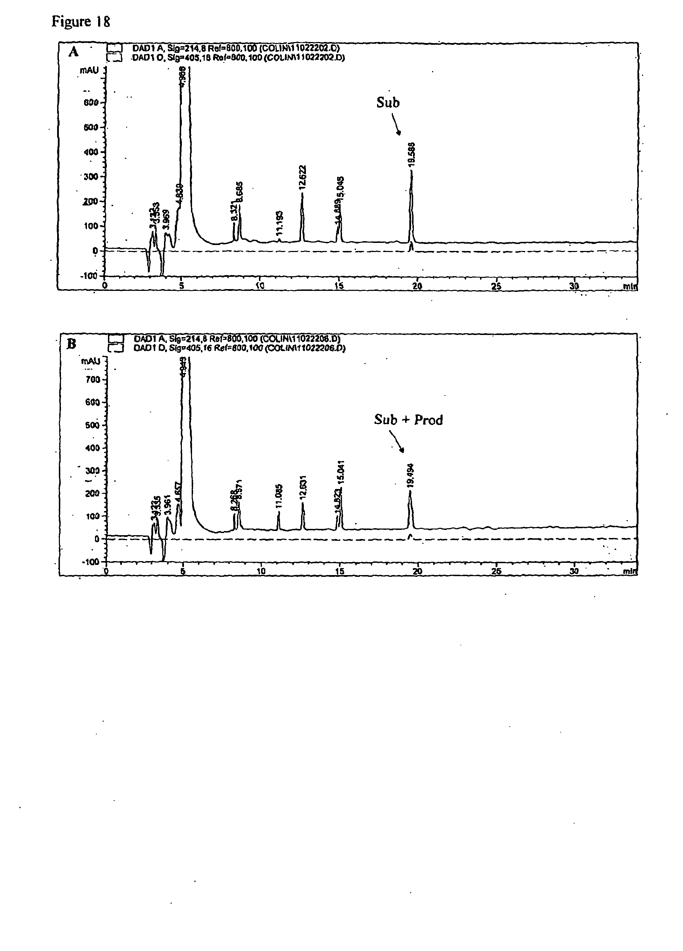

In order to confirm that methylation of the substrates was indeed occurring in the presence of G9a, assays using peptides **4** and **9** were performed in micro-centrifuge tubes and samples taken at regular time intervals and analysed by RP-HPLC and ESI-MS. Assay conditions were as follows: peptide substrate (15 µM), G9a (100 nM), SAM (100 µM) in assay buffer (150 µl). Analysis was performed using a Luna C18 column with a gradient of 0-50 % MeCN + 0.1% TFA over 30 mins. The relevant spectra are shown in Fig. 17 and 18.

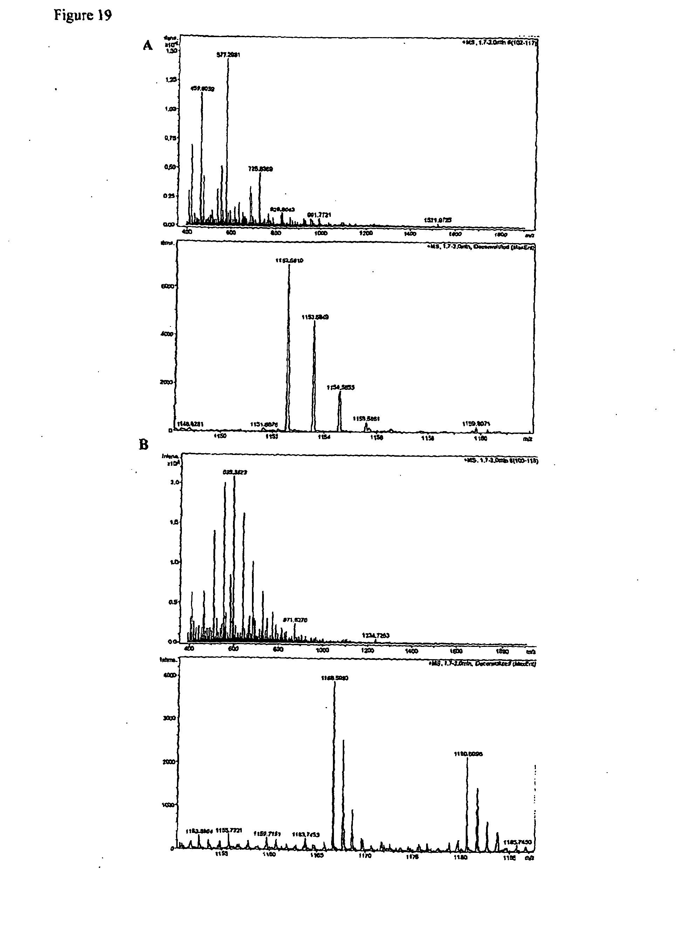

For the assay with peptide **4** as substrate, a second peak with a later retention time than the substrate appears after prolonged incubation with G9a (Fig. 17B). When using peptide **9,** no such second peak is visible but this could be due to insufficient separation using the current gradient method. Relevant peaks were collected and analysed by ESI-MS for evidence of methylation (Fig. 19-20).

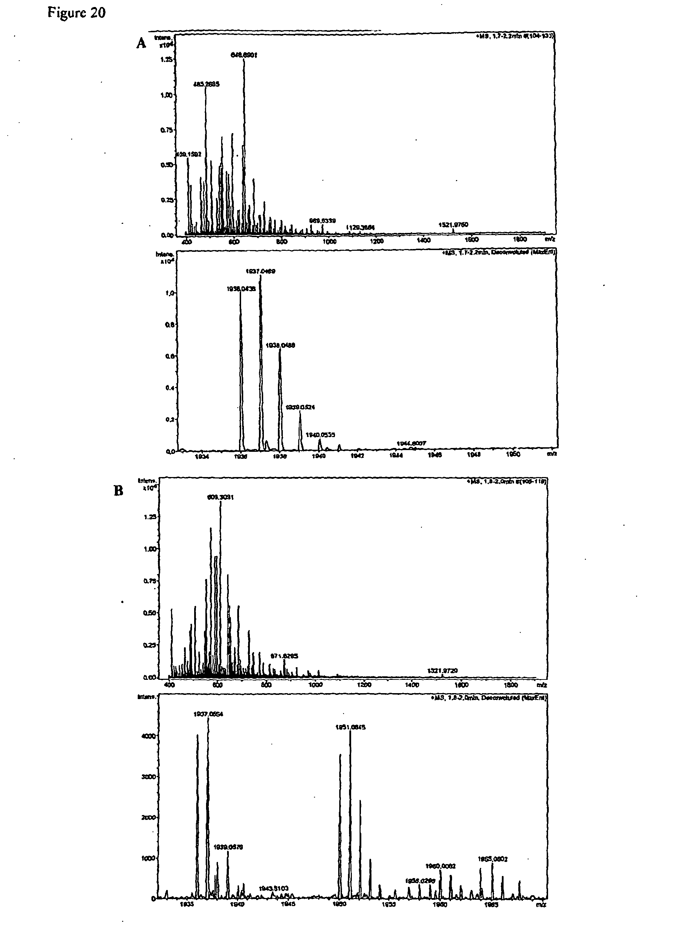

ESI-MS analysis of the peak with later retention time than peptide **4** revealed it to correspond to methylated product, with the correct masses clearly visible for monomethylated (m/z 1166) and dimethylated (m/z 1180) material (Fig 19B). As no clearly defined second peak was visible for the assay with peptide **9,** the whole peak containing a 405 nm absorbance was collected and analysed by ESI-MS. At t=0, unsurprisingly, this peak only contained the mass for substrate (m/z 1936) (Fig. 20A). However, after 6h incubation with G9a, whilst some evidence of substrate remains, there are now clear signals for monomethylated (m/z 1950) and dimethylated (m/z 1964) product (Fig. 20B). Hence, these results provide direct evidence that peptides **4** and **9** are converted to their methylated equivalents after exposure to the methyltransferase G9a.

### G9a Assay Time-course

Assays were performed in a 384-well microplate using peptides **4** and **9** (1 µM) under varying G9a concentrations with reactions started with SAM (100 µM) and stopped at regular intervals by the addition of Endo-LysC (2 nM). After 10 min incubation, FLTs were measured for evidence of protease protection induced by G9a catalysed lysine methylation.

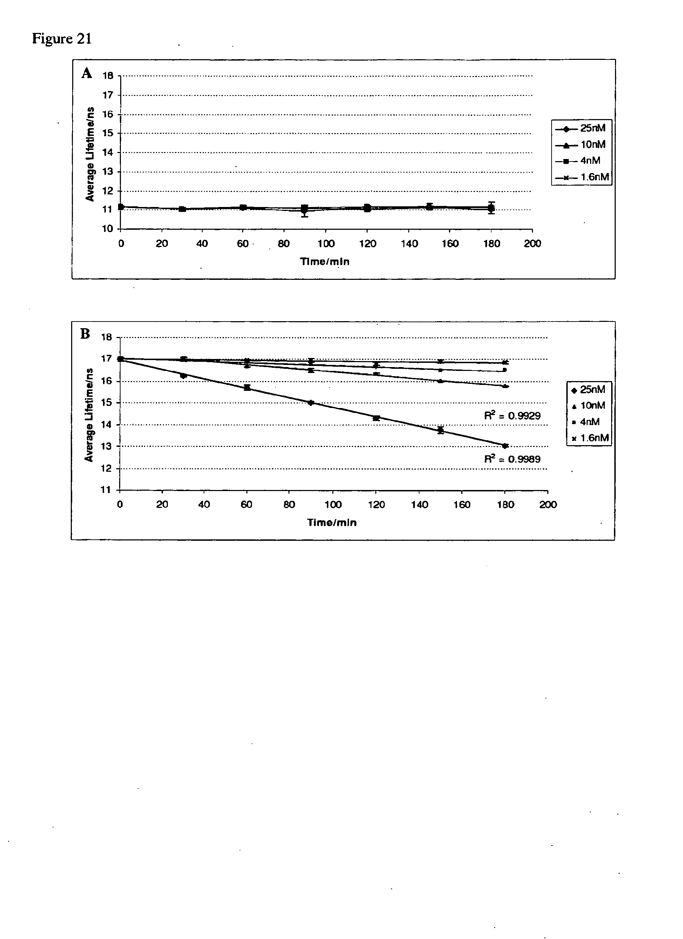

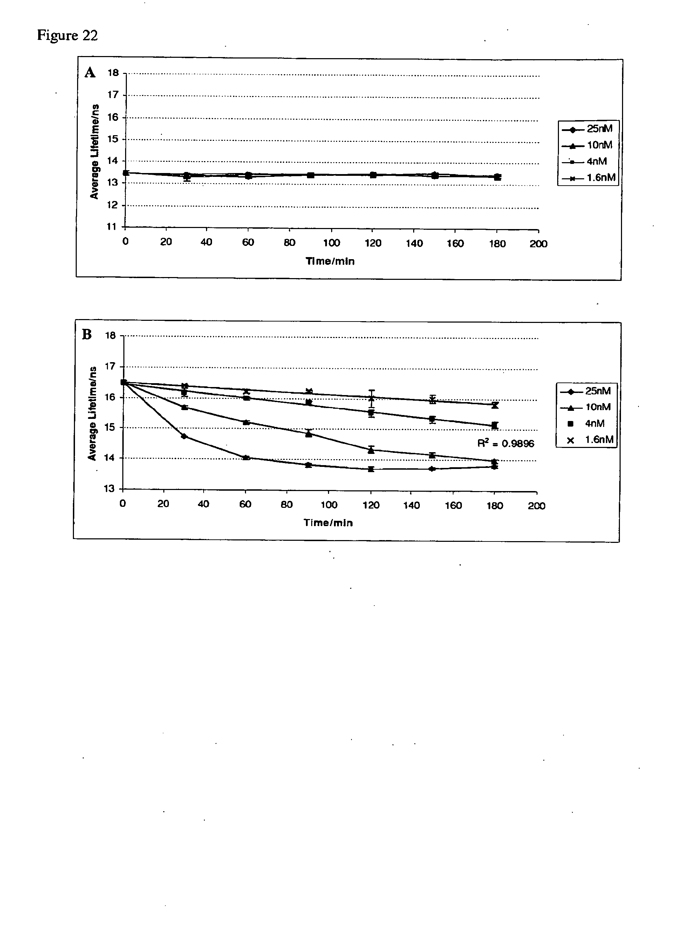

As can be seen from Fig. 21-22, there is no change in FLT of either peptide prior to addition of Endo-LysC. However, after addition and incubation with the protease, the extent of FLT modulation varies as a function of G9a concentration and assay time, consistent with methylation of lysine conferring protease protection. At t = 0, treatment with Endo-LysC results in a FLT increase from 11.2 ns to 17.0 ns (for peptide **4**) and 13.5 ns to 16.5 ns (for peptide **9**) which is consistent with no modulation and complete cleavage of the substrate by the protease. Linearity of reaction is evident for many of the time-courses.

### Dependence on SAM

The dependence of the G9a assay on SAM was investigated using both peptide **4** and **9** as substrates. Assays were performed in a 384-well microplate in assay buffer (20 µl) using either 25 nM or 10 nM G9a and 1 µM substrate. SAM concentrations were varied from 200 µM - 0.4 µM and assays were incubated for 2 h at room temperature. On completion, Endo-LysC (2 nM) was added to each well and FLTs measured following a 10 min incubation period. The data shown in Fig. 23 confirms the dependence of G9a activity on SAM concentration with a *K*ₘ (app) = 3 - 11 µM.

### Inhibitor Titration

In order to demonstrate that the FLT protease protection assay for G9a was suitable for inhibitor screening, the known, non-SAM competitive, G9a inhibitor BIX-01294 was purchased (Merck Chemicals Ltd.) and tested in the assay. Conditions were as follows: BIX-01294 (200 µM - 20 nM), G9a (25 nM or 10 nM), SAM (10 µM), and peptide substrate (1 µM) in assay buffer (20 µl). Reactions were performed in a 384-well microplate at room temperature for 2 h and stopped by the addition of Endo-LysC (2 nM).

With peptide **9** as the substrate, an IC₅₀ = 1.6 µM was determined for BIX-01294 (Fig. 24), consistent with a literature value of 1.9 µM (Chang, Y., Zhang, X., Horton, J. R., Upadhyay, A. K., Spannhoff, A., Liu, J., Snyder, J. P., Bedford, M. T., and Cheng, X., Nat. Struct. Biol., 2009, 16, 312-317) . In contrast, the assay with peptide **4** returned an IC₅₀ ≈ 28 nM (failure to plateau at low inhibitor concentrations) (Fig. 24). The discrepancy in the values may be due to a different mechanism of inhibition, as BIX-01294 is known to be a histone H3 competitive inhibitor and the differences in sequence of peptides **4** and **9** may affect inhibitor binding.

### Z'-Factor

To demonstrate that the G9a FLT assay was compatible with high-throughput screening . (HTS), a value for the Z'-factor was determined.

Assays were set up in a 384-well microplate, with half the plate containing G9a (BPS Bioscience) (625 pM), peptide **9** (1 µM) and SAM (100 µM), whereas the other half contained G9a (625 pM), peptide **9** (1 µM) and no SAM. Final assay volume was 20 µl and the plate was incubated at room temperature for 1 h at which point Endo-LysC (2 nM final concentration) in assay buffer (10 µl) was added to each well. Following a further 10 min incubation at room temperature the FLTs were measured and a Z'-factor of 0.71 calculated, demonstrating the assay's suitability for HTS screening (Fig. 75).

In conclusion, novel peptide substrates have been designed for the lysine methyltransferase G9a incorporating a long lifetime fluorophore (9AA) and aromatic modulator residue (tryptophan) for use in a FLT protease protection assay. Methylation following incubation with G9a has been demonstrated for two of the peptides and this process has been shown to confer protease protection as confirmed by continued FLT modulation following addition of the protease Endo-LysC. The assay's performance was verified by conducting enzyme, SAM, and inhibitor titrations, and a Z'-factor of 0.71 confirmed the assay was suitable for HTS applications.

It is envisaged that such an approach would not be restricted to G9a but transferable to other protein methyltransferases using appropriately designed peptide substrates. Other long lifetime fluorophores and/or modulators could also be employed.

### (B) FLT Protease Protection Assay for Set7/9 lysine methyltransferase

In a further exemplification of the FLT protease protection approach to assaying protein methyltransferases, Set7/9, a lysine methyltransferase, was validated as a suitable target.

Set7/9 methylates Lys4 of histone H3 among other protein targets (Wilson et al., Cell 2002, 111, 105-115). Recombinant Set7/9 is commercially available from suppliers such as BPS Bioscience.

Peptide **12,** consisting of the histone H3 N-terminal sequence and incorporating a tryptophan residue at the N-terminus and a 9-aminoacridine fluorophore attached to the side-chain of the lysine-9 residue, was synthesised as a substrate for Set7/9.
Peptide **12:** WARTKQTARK(9AA)STGGKAPRKQLAK-CONH₂

### Evidence of Methylation

Peptide **12** (15 µM) was incubated with Set7/9 (172 nM) and S-adenosylmethionine (SAM) (100 µM) in assay buffer (20 mM Tris pH 8, 25 mM NaCl, 1 mM DTT, 0.025 % Tween-20) (200 µl) and aliquots taken at regular time intervals and analysed by RP-HPLC and ESI-MS to ascertain the degree of methlyation of the peptide substrate by Set7/9. The assay was performed at room temperature and samples taken after 0 h, 2 h, and 6 h time intervals respectively.

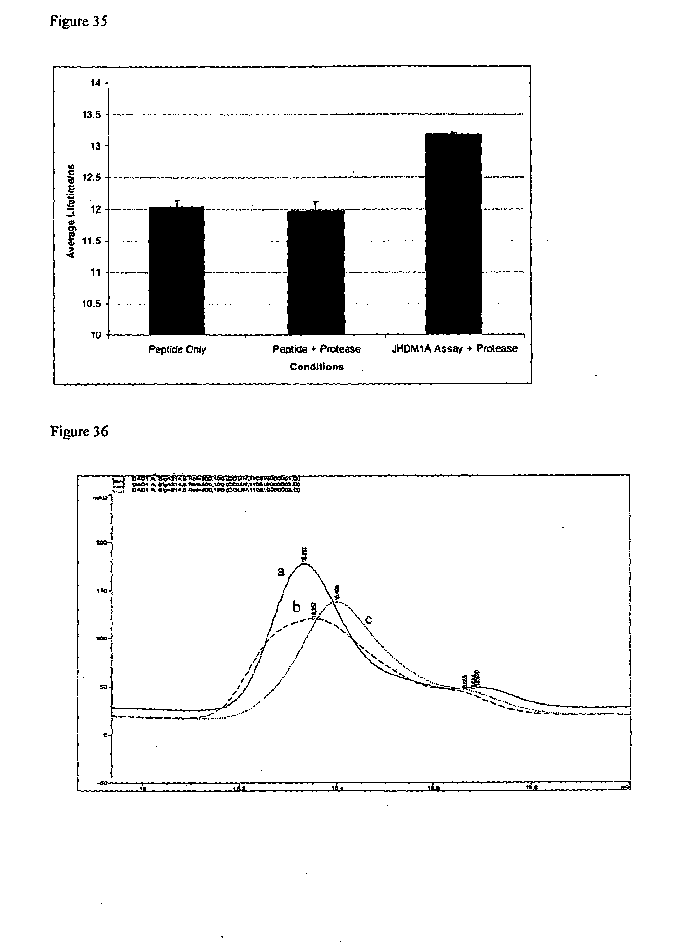

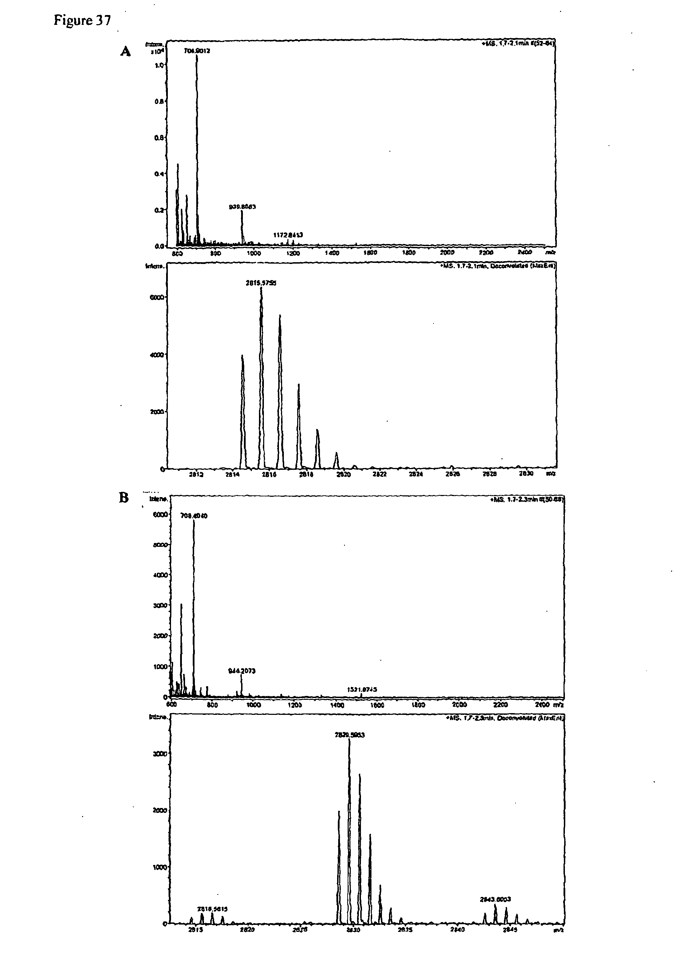

The assay's progress over the course of 6 h is shown via the HPLC spectra in Fig. 36. After 6 h there is a clear shift in retention time from t_{R} = 18.3 min (at t = 0 h) to t_{R} = 18.4 min. Analysis of the peaks by ESI-MS confirmed a mass shift of 14 amu, consistent with the addition of a methyl group (Fig. 37). After 6 h, very little of the substrate mass (*m*/*z* 2814) was detected with *mlz* 2828, corresponding to monomethylated peptide, present as the predominant species. A small peak corresponding to dimethylated peptide (*m*/*z* 2842) was also observed (Fig. 37).

In order to test if methylation of peptide **12** by Set7/9 had conferred protection against Endo-LysC catalysed cleavage, the assay mixture was diluted to 1 µM concentration with assay buffer and 20 µl of the resulting solution added to three wells of a 384-well black microplate. 10 µl of Endo-LysC (final concentration 2 nM) was added to each well and the plate incubated at room temperature for 15 mins before the FLTs were measured using a NanoTaurus plate reader. Peptide only controls were included at the same concentration for comparison.

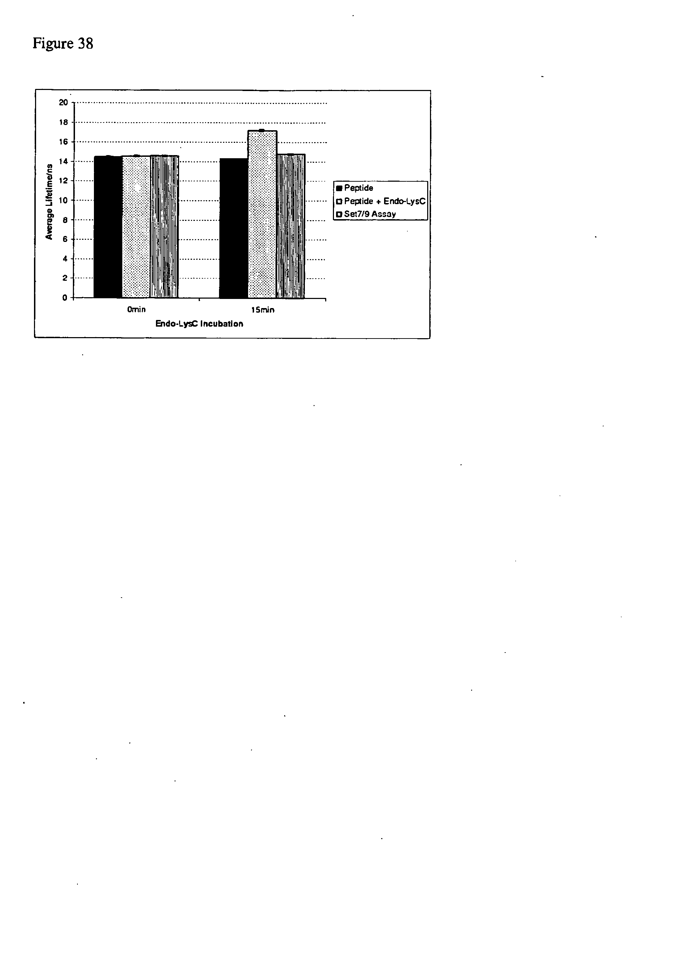

In the absence of Set7/9, the FLT of peptide **12** increased by 2.4 ns after incubation with 2 nM Endo-LysC (Fig. 38). In contrast, no significant increase in FLT was observed for peptide **12** exposed to Set7/9 prior to incubation with Endo-LysC. These findings are consistent with Set7/9 catalysed methylation of peptide **12** offering protection to protease induced cleavage.

### Set7/9 Assay Time-course

Assays were performed in a 20 µl volume in a 384-well black microplate and contained peptide **12** (1 µM) and Set7/9 enzyme (100 nM, 50 nM, 25 nM, or 12.5 nM) in assay buffer. Reactions were stopped at regular intervals by the addition of 10 µl Endo-LysC (final concentration 2 nM). FLTs were measured following 15 min incubation with Endo-LysC at room temperature. All measurements were performed in duplicate using a NanoTaurus plate reader.

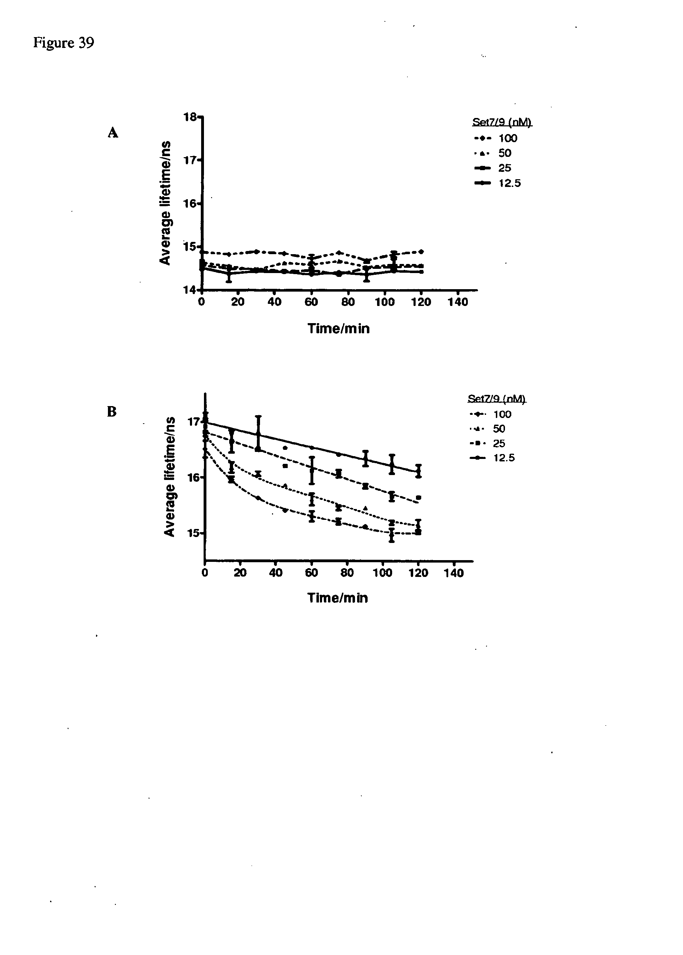

As can be seen from Fig. 39A, there is little-change in the FLT of peptide **12** prior to addition of Endo-LysC. However, after addition and incubation with the protease, the extent of FLT modulation varies as a function of Set7/9 concentration and assay time, consistent with methylation of lysine conferring protease protection (Fig. 39B). The overall lifetime change between substrate and product was approximately 2.4 ns. In summary, methylation by Set7/9 is reported by a decrease in the measured fluorescence lifetime of the assay.

In order to increase the overall dynamic range of the assay, peptide **13** was synthesised. This peptide is one amino acid shorter than peptide **12,** due to the deletion of an alanine residue close to the N-terminus. It was expected that this alteration would increase the modulation of the FLT of the substrate by bringing the tryptophan in closer proximity to the 9AA fluorophore. The measured FLT of peptide **13** was 13 ns, approximately 1.5 ns shorter than for peptide **12,** hence peptide **13** offered the opportunity to increase the dynamic range of the Set7/9 assay. However, for such an increase to be realised, peptide **13** must be successfully methylated by Set7/9. Therefore, to confirm the activity of peptide **13** in a Set7/9 assay, the time-course described above was repeated using the same conditions that were used to for peptide **12.**
Peptide **13:** WRTKQTARK(9AA)STGGKAPRKQLAK-CONH₂

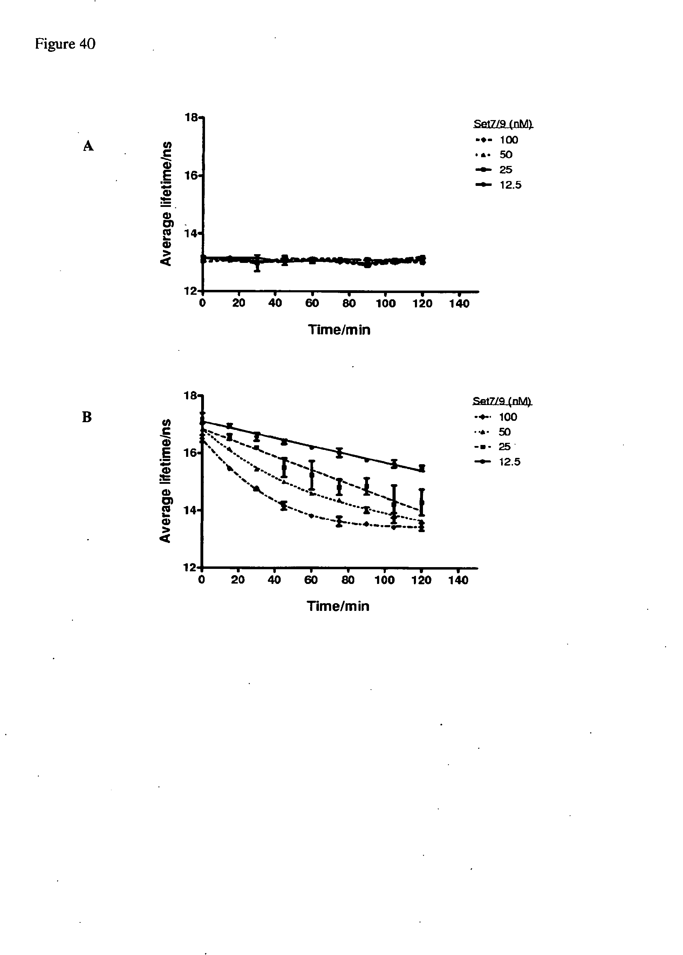

No change in the FLT of peptide **13** was evident until addition of the protease, Endo-LysC (Fig. 40A). However, following incubation with Endo-LysC for 15 min at room temperature, the FLT of the assay wells varied as a function of Set7/9 concentration and assay time, consistent with methylation of peptide **13** conferring protease protection (Fig. 40B). Importantly, the maximum range of the assay had now increased to 4 ns, thereby offering improvements in sensitivity. Hence, further validation of the Set7/9 FLT assay was performed with peptide **13** as the substrate.

### S-adenosylmethionine (SAM) Titration

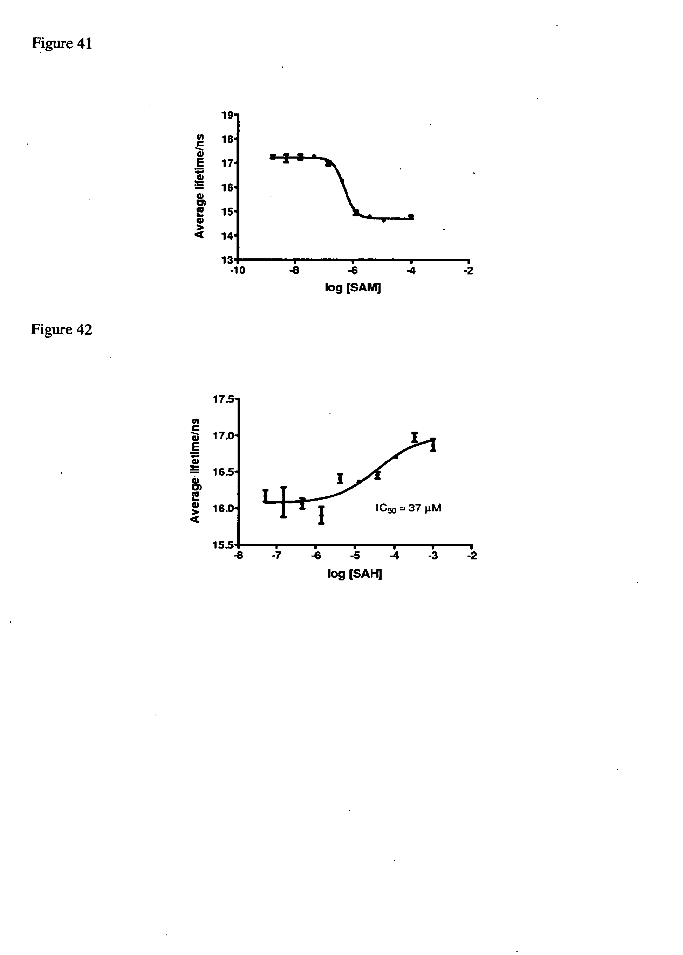

The dependence of the Set7/9 assay on SAM co-factor was investigated using peptide 13 as the substrate. The assay was performed in a 384-well microplate at room temperature for 2 h in assay buffer (20 µl) using 25 nM Set7/9 and 1 µM substrate. SAM concentrations were varied from 100 µM -1.7 nM. On completion, Endo-LysC (2 nM) was added to each well and FLTs measured following a 20 min incubation period. The data shown in Fig. 41 confirms the dependence of Set7/9 activity on SAM concentration with a *K*ₘ (app) = 500 nM.

### S-adeonsylhomocysteine Inhibitor Titration

In order to demonstrate that the FLT protease protection assay for Set7/9 was suitable for inhibitor screening, the known SAM competitive inhibitor, S-adenosylhomocysteine (SAH) was purchased (Sigma) and tested in the assay. Conditions were as follows: SAH (1 mM - 1.3 µM), Set7/9 (50 nM), SAM (500 nM), and peptide **13** (1 µM) in assay buffer (20 µl). Reactions were performed in a 384-well microplate at room temperature for 2 h and stopped by the addition of Endo-LysC (2 nM).

An IC₅₀ = 37 µM was determined for SAH (Fig. 42), which compares with a reported IC₅₀ of 16 µM which was obtained using a lower concentration of SAM (Gauthier, N.; Caron, M.; Pedro, L.; Arcand, M.; Blouin, J.; Labonté, A.; Normand, C.; Paquet, V.; Rodenbrock, A.; Roy, M.; Rouleau, N.; Beaudet, L.; Padrós, J.; Rodriguez-Suarez, R. J. Biomol. Screen., 2012, 17, 49-58).

### Z'-Factor

To demonstrate that the Set7/9 FLT assay was compatible with high-throughput screening (HTS) approaches, a value for the Z'-factor was determined.

Assays were set up in a 384-well microplate, with half the plate containing Set7/9 (25 nM), peptide **13** (1 µM), and SAM (100 µM), whereas the other half contained Set7/9 (25 nM), peptide **13** (1 µM), but with SAM replaced by assay buffer. Final assay volume was 20 µl and the plate was incubated at room temperature for 2 h at which point Endo-LysC (2 nM final concentration) in assay buffer (10 µl) was added to each well. Following a further 15 min incubation at room temperature the FLTs were measured and a Z'-factor of 0.81 calculated (Fig. 43).

In conclusion, two novel peptides (peptides **12** and **13**) have been validated as substrates for the lysine methyltransferase. Set7/9 in a FLT protease protection assay. The basis of the assay is the use of 9AA-labelled peptide substrates incorporating an aromatic moiety (e.g. tryptophan) that modulates the FLT of 9AA until the modulator and fluorophore are separated by the action of the protease Endo-LysC, which specifically cleaves adjacent to lysine residues. Lysine methylation by Set7/9 confers protection from Endo-LysC cleavage, thereby maintaining modulation of the peptide's FLT. Hence, lysine methylation is reported by a decrease in the measured FLT of the assay.

The FLT Set7/9 assay performance was verified by conducting enzyme, SAM and inhibitor titrations, and a Z'-factor of 0.81 confirmed the assay was suitable for HTS applications.

This study provides further exemplification of the FLT protease protection assay for protein lysine methyltransferases and extends the approach to a lysine-4 histone methyltransferase, Set7/9.

### (C) PRMT5 Proof-of-Concept FLT Protease Protection Assay

PRMT5 is a suitable candidate for proof-of-concept FLT protease protection assays for PRMTs. This enzyme is commercially available from BPS Bioscience and preferentially symmetrically methylates arginine-3 on histone H4 and arginine-8 on histone H3 (Branscombe et al., J Biol.Chem 2001, 276, 32971-32976). A suitable substrate for the FLT assay is shown below:
Peptide **15:** Ac-WSGRGKGGK(9AA)GLGKGGAKRHRK-CONH₂

Peptide **15** is based on the N-terminal sequence of histone H4, with 9AA attached via a lysine side-chain and a tryptophan residue incorporated at the N-terminus to modulate the FLT of 9AA in the intact peptide. Treatment with Endo-ArgC would be expected to cleave the peptide at arginine-3 (numbered from the serine residue adjacent to the N-terminus) thereby separating 9AA from tryptophan leading to an increase in FLT. Methylation of arginine-3 by PRMT5 would prevent cleavage by Endo-ArgC thereby maintaining modulation of the FLT of 9AA. In contrast, the presence of a PRMT5 inhibitor would prevent arginine methylation thereby leaving the peptide susceptible to protease-induced cleavage at arginine-3. Hence, the FLT of 9AA would increase and the extent of the increase could be correlated to the degree of inhibition.

### Endo-ArgC Cleavage

To examine protease cleavage of peptide **15,** assays were prepared in a 384-well microplate containing peptide **15** (1 µM) and varying concentrations of Endo-ArgC (Merck Chemicals Ltd.) (10 nM - 1.25 nM) in assay buffer (20 mM Tris pH 8, 25 mM NaCl, 1 mM DTT, 0.025 % Tween-20) (30 µl). FLTs were measured at regular intervals over one hour with measurements performed in triplicate on a NanoTaurus plate reader.

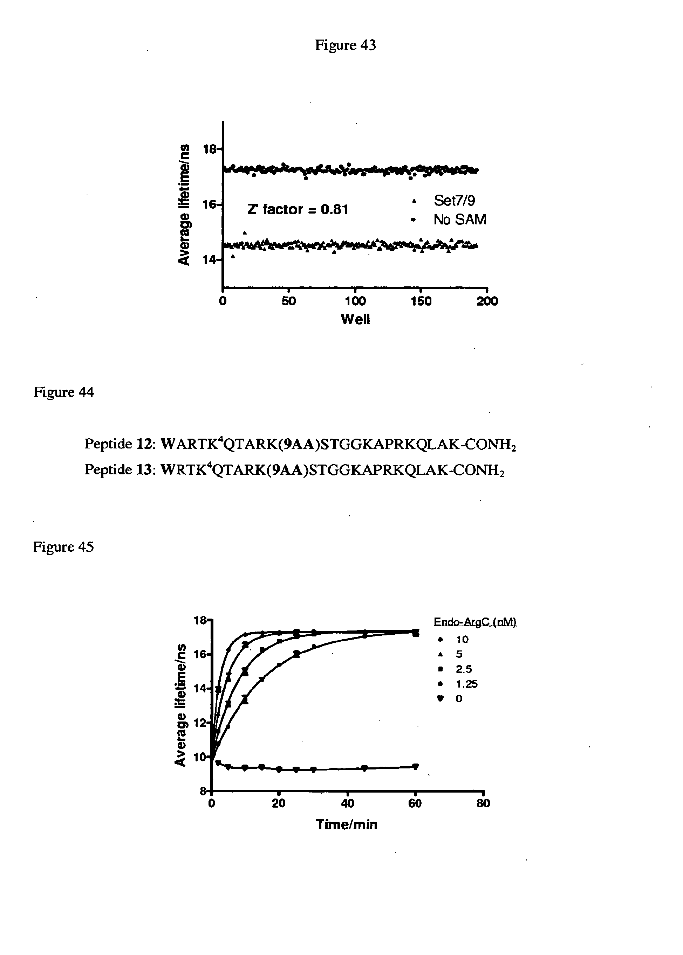

Endo-ArgC successfully cleaved peptide **15** leading to an increase in FLT from 9.4 ns to 17.2 ns (Fig. 45). This large change in FLT offered the potential of a sensitive assay for PRMT5.

### Evidence of Methylation

Peptide **15** (15 µM) was incubated with PRMT5 (150 nM) and S-adenosylmethionine (SAM) (100 µM) in assay buffer (20 mM Tris pH 8, 25 mM NaCl, 1 mM DTT, 0.025 % Tween-20) (200 µl) and aliquots taken at regular time intervals and analysed by RP-HPLC and ESI-MS to ascertain the degree of methlyation of the peptide substrate by PRMT5. The assay was performed at room temperature and samples taken after 0 h, 3.5 h, and 17.5 h time intervals respectively.

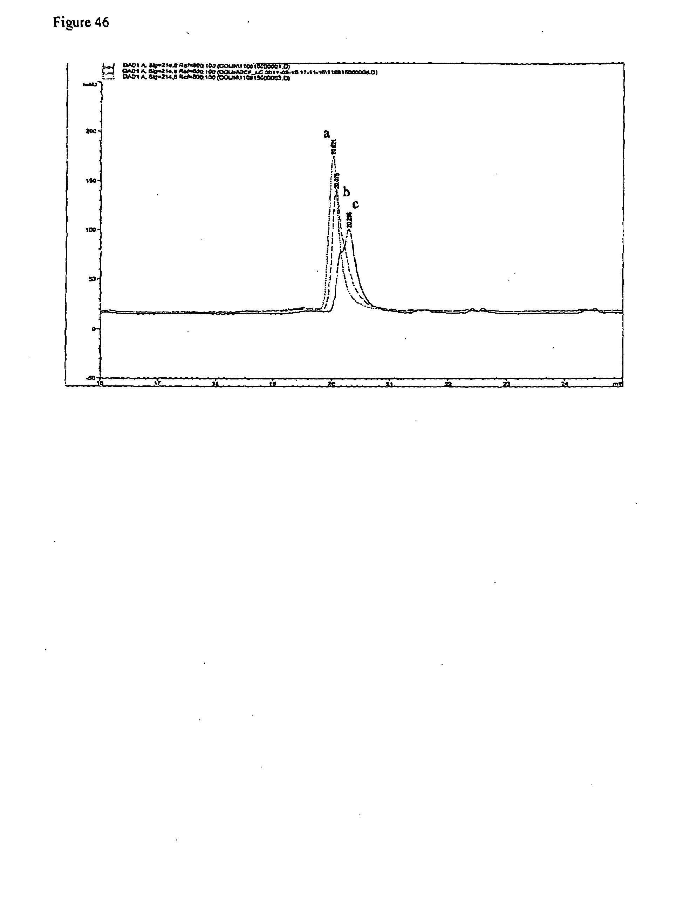

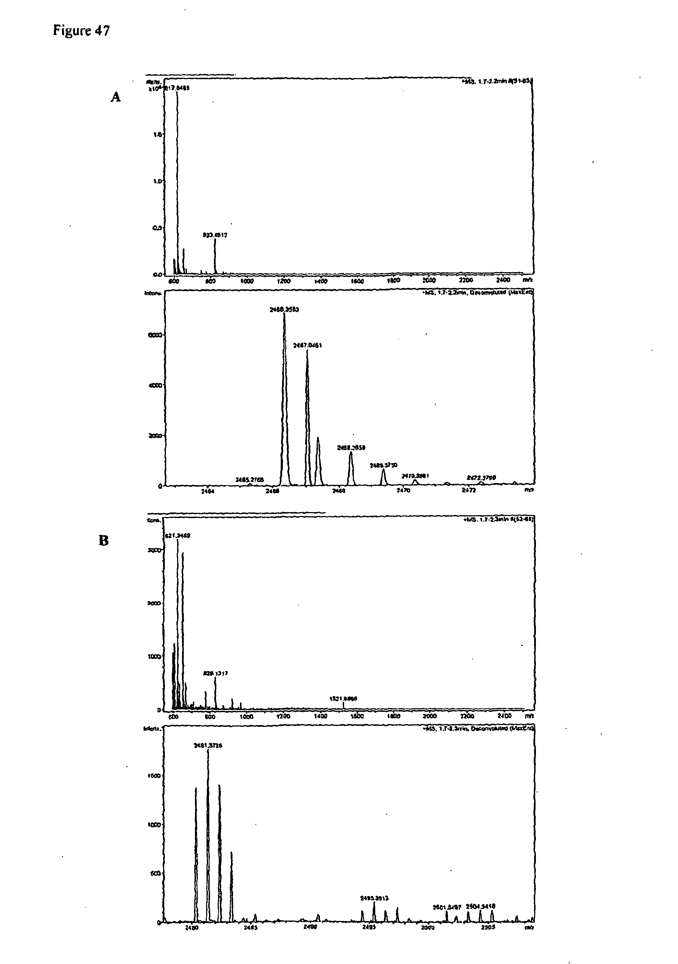

The assay's progress over the course of 17.5 h is shown via the HPLC spectra in Fig. 46. After 17.5 h there is a clear shift in retention time from t_{R} = 20.0 min (at t = 0 h) to t_{R} = 20.3 min. Analysis of the peaks by ESI-MS confirmed a mass shift from the substrate (*m*/*z* 2466) to monomethylated product (*m*/*z* 2480) (Fig. 47). In addition, a small peak corresponding to dimethylated peptide (*m*/*z* 2494) was also observed (Fig. 47).

Although from RP-HPLC the reaction had not yet reached completion, the effect on the FLT after treatment with protease was tested by diluting the assay mixture 15-fold with assay buffer and adding 20 µl of the resulting solution to three wells of a 384-well microplate. 10 µl of Endo-ArgC (final concentration 10 nM) was added to each well and the plate incubated at room temperature for 60 mins before FLTs were measured. Peptide only controls were included at the same concentration for comparison. Note, the Endo-ArgC used here was from a different supplier (Sigma) and proved to be less active than the batch used to generate the data in Fig.45. Hence, a higher concentration and longer incubation time were used.

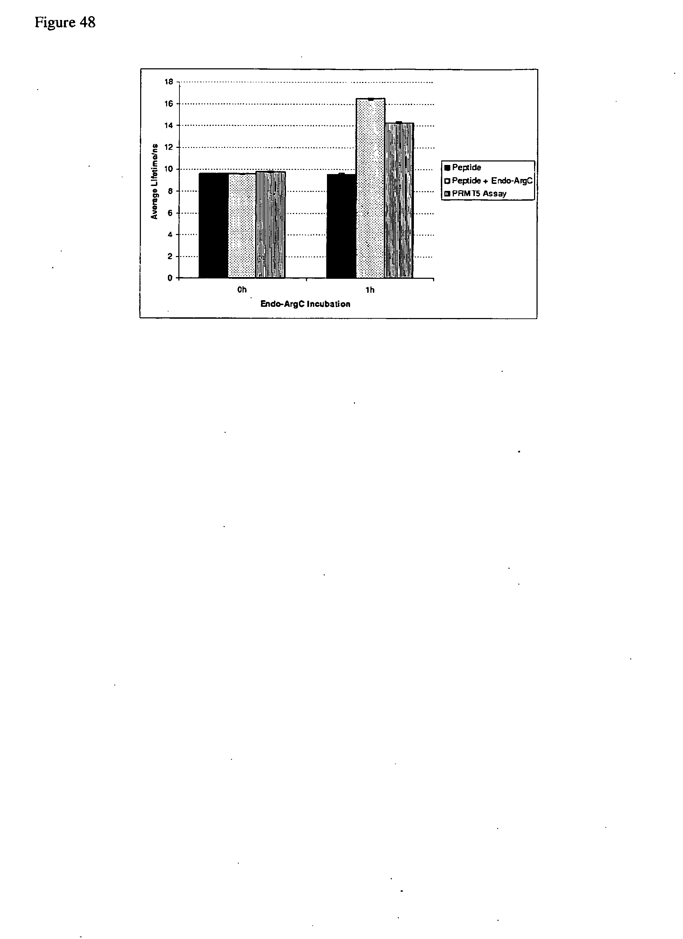

In the absence of PRMT5, the FLT of peptide **15** increased by 6.9 ns after incubation with 10 nM Endo-ArgC (Fig. 48). In contrast, the increase in FLT was 4.7 ns for peptide **15** exposed to PRMT5 prior to incubation with Endo-ArgC. This increase can be attributed to the remaining substrate present in the assay mixture, with the full increase not realised due to PRMT5 catalysed methylation providing protease protection.

### PRMT5 Assay Time-course

Assays were performed in a 20 µl volume in a 384-well black microplate and contained peptide **15** (1 µM) and PRMT5 enzyme (100 nM, 50 nM, 25 nM, or 12.5 nM) in assay buffer. Reactions were stopped at regular intervals by the addition of 10 µl Endo-ArgC (final concentration 1.25 nM). FLTs were measured following 15 min incubation with Endo-ArgC at room temperature. All measurements were performed in duplicate using a NanoTaurus plate reader.

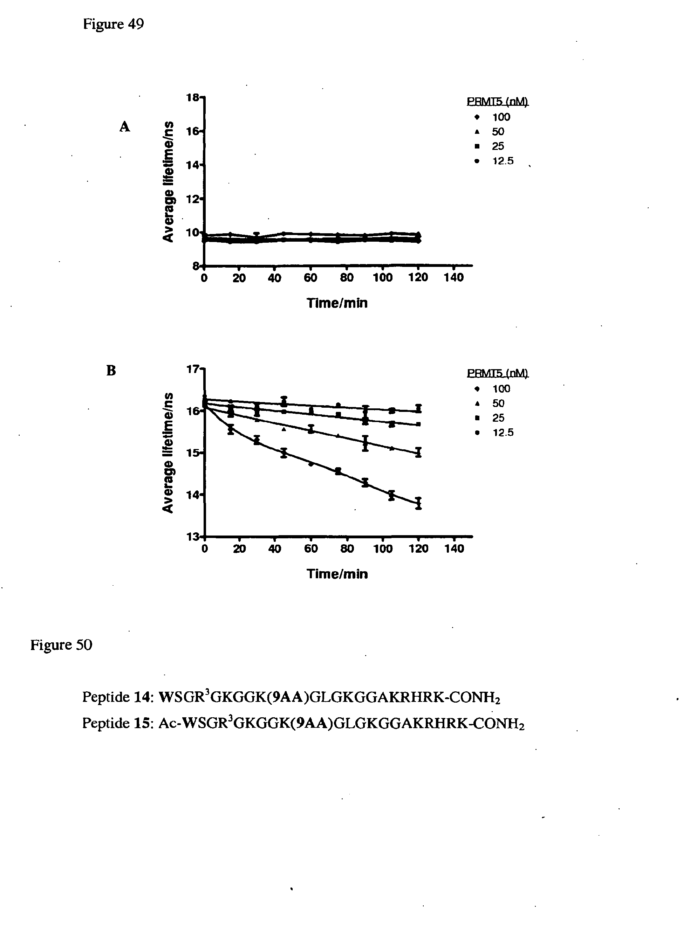

As can be seen from Fig. 49, there is no change in FLT of peptide **15** prior to addition of Endo-ArgC. However, after addition and incubation with the protease, the extent of FLT modulation varies as a function of PRMT5 concentration and assay time, consistent with methylation of arginine conferring protease protection. Hence, arginine methylation by PRMT5 is reported by a decrease in the measured fluorescence lifetime of the assay.

### Sinefungin Inhibitor Titration

In order to demonstrate that the FLT protease protection assay for PRMT5 was suitable for inhibitor screening, the known generic SAM competitive inhibitor, sinefungin was purchased (Sigma) and tested in the assay. Conditions were as follows: sinefungin (2 mM - 0.1 µM), PRMT5 (50 nM), SAM (10 µM) and peptide **15** (1 µM) in assay buffer (20 µl). Reactions were performed in triplicate in a 384-well microplate at room temperature for 3 h and stopped by the addition of Endo-ArgC (1 nM). An IC₅₀ of 13 µM was determined for sinefungin (Fig. 51). An IC₅₀ value for this inhibitor against PRMT5 has not been previously reported in the peer-reviewed literature.

In summary, this work provides exemplification of a FLT protease protection assay for protein arginine methyltransferases with Endo-ArgC employed as the specific protease. Whilst exemplification was performed using PRMT5, such an approach is extendable to other PRMTs using appropriately designed peptide substrates.

### (D) FLT Protease Protection Assay for EZH2 Lysine Methyltransferase

In a further exemplification of the FLT protease protection approach to assaying protein methyltransferases, EZH2, a lysine methyltransferase, was validated as a suitable target.

EZH2 methylates lysine-27 on histone H3 and is active when in complex with four other proteins: EED, SUZ12, RbAp48 and AEBP2. The human recombinant five-member complex is available from BPS Bioscience. Mutations in the EZH2 enzyme have been associated with increased risk of malignancy in certain forms of non-Hodgkin's lymphomas (Morin, R. D.; Johnson, N. A.; Severson, T. M.; Mungall, A. J.; An, J.; Goya, R.; Paul, J. E.; Boyle, M.; Woolcock, B. W.; Kuchenbauer, F.; Yap, D.; Humphries, R. K.; Griffith, O. L.; Shah, S.; Zhu, H.; Kimbara, M.; Shashkin, P.; Charlot, J. F.; Tcherpakov, M.; Corbett, R.; Tam, A.; Varhol, R.; Smailus, D.; Moksa, M.; Zhao, Y.; Delaney, A.; Qian, H.; Birol, I.; Schein, J.; Moore, R.; Holt, R.; Horsman, D. E.; Connors, J. M.; Jones, S.; Aparicio, S.; Hirst, M.; Gascoyne, R. D.; Marra, M. A. Nat. Genet. 2010, 42, 181-185). Hence, assays to facilitate the search for small molecule inhibitors of EHZ2 are highly sought after by drug discovery groups.

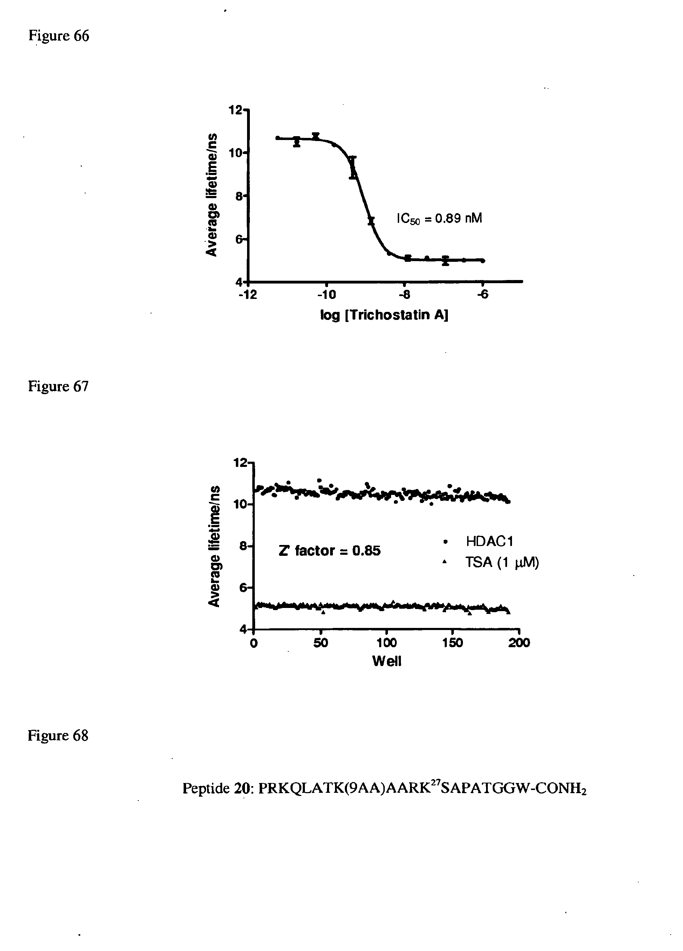

Peptides based on H3K27 sequences are suitable substrates for the EZH2 enzyme complex and such a peptide was modified to include a 9AA fluorophore and a tryptophan residue to enable the development of a FLT protease protection assay for this enzyme (Fig. 68)
Peptide **20:** PRKQLATK(9AA)AARK²⁷SAPATGGW-CONH₂

Exposure of peptide **20** to a lysine specific protease, such as Endo-LysC, would cleave adjacent to lysine-27, leading to the separation of 9AA from the modulating effect of the tryptophan residue resulting in an increase in FLT. In contrast, when lysine-27 is methylated by the action of EZH2, cleavage at this residue by Endo-LysC is no longer possible, thereby maintaining the modulation of the FLT of 9AA. Hence, EZH2 methylation is reported by a decrease in the measured FLT of the assay.

### Endo-LysC Cleavage of Peptide 20

The protease cleavage of peptide **20** was investigated by performing assays in a 384-well hi-base microplate containing peptide **20** (1 µM) in the presence of varying concentrations of Endo-LysC (4 - 0 nM) in assay buffer (20 mM Bicine pH 7.6, 50 mM NaCl, 0.002 % Tween-20, 0.005 % BSA, 1 mM DTT (20 µl). FLTs were measured at regular intervals over one hour with measurements performed in triplicate on a NanoTaurus plate reader.

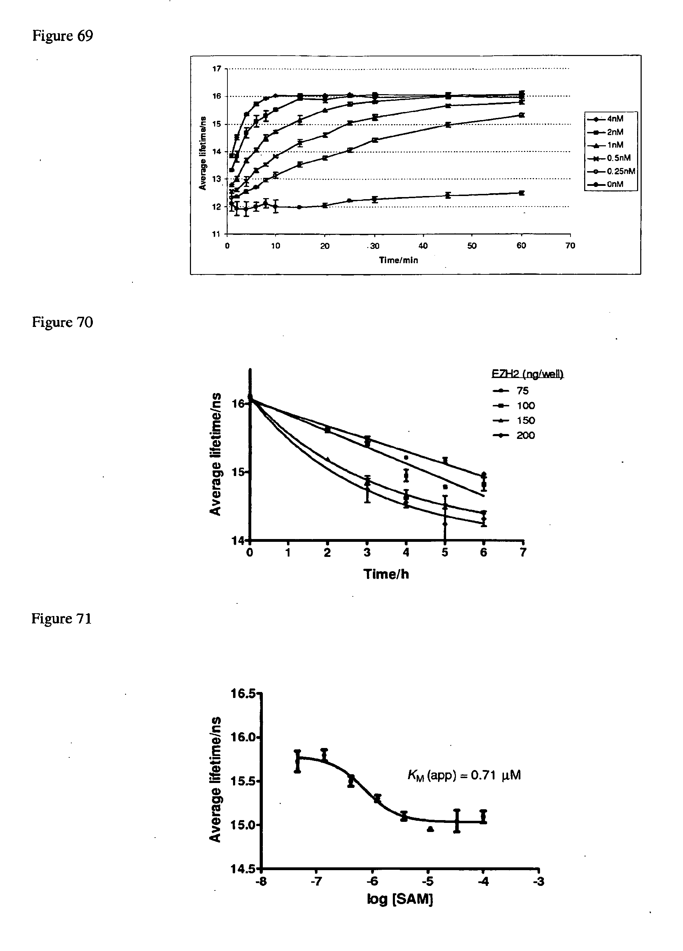

Cleavage of peptide **20** by Endo-LysC resulted in a time and concentration dependent increase in FLT as cleavage at lysine-27 led to the separation of 9AA from the modulating effect of tryptophan (Fig. 69). The FLT increased from 12 ns to 16 ns, providing a good dynamic range for the assay.

### EZH2 Assay Time-course

Assays were performed in a 10 µl volume in a 384-well hi-base black microplate and contained peptide **20** (1 µM), SAM (3 µM) and EZH2 complex (200 ng/well - 75 ng/well) in assay buffer (20 mM Bicine pH 7.6, 50 mM NaCl, 0.002 % Tween-20, 0.005 % BSA, 1 mM DTT). Reactions were stopped at regular intervals by the addition of 10 µl Endo-LysC (final concentration 4 nM). FLTs were measured following a 35 min incubation with Endo-LysC at room temperature. All measurements were performed in duplicate on a NanoTaurus plate reader.

As can be seen from Fig. 70, following incubation with protease, the measured FLT decreases as a function of EZH2 concentration and assay time consistent with methylation of lysine-27 conferring protection to protease induced cleavage. The responses were linear with respect to 75 and 100 ng/well of EZH2 with the latter concentration chosen for use in subsequent experiments. Note that the assay was run for extended periods of time due to the low turnover rate of the EZH2 complex. In summary, methylation by EZH2 is reported by a decrease in the measured fluorescence lifetime of the assay.

### S-adenosylmethionine (SAM) Titration

The dependence of the EZH2 assay on SAM co-factor was investigated using peptide **20** as the substrate. The assay was performed in a 384-well hi-base microplate at room temperature for 5 h in assay buffer (10 µl) using 100 ng/well EZH2 complex and 1 µM substrate. SAM concentrations were varied from 100 µM - 0.05 µM. On completion, Endo-LysC (4 nM) was added to each well and FLTs measured following a 15 min incubation period. The data was fitted to a variable slope dose-response model in Graph Pad Prism to determine the *K*_{M} (app) for SAM.

From Fig. 71, a *K*_{M} (app) of 0.71 µM was determined for SAM in an EZH2 assay. This value is in close agreement to a literature value of between 0.21 - 0.30 µM, determined in a radiometric assay format (Sneeringer, C. J.; Scott, M. P.; Kuntz, K. W.; Knutson, S. K.; Pollock, R. M.; Richon, V. M.; Copeland, R. A. Proc. Natl. Acad. Sci. U.S.A 2010, 107, 20980-20985).

### S-adenosylhomocysteine (SAH) Inhibitor Titration

In order to demonstrate that the FLT protease protection assay for EZH2 was suitable for inhibitor screening, the known generic SAM competitive inhibitor, SAH was purchased (Sigma) and tested in the assay. Conditions were as follows: SAH (1 mM - 0.05 µM), EZH2 complex (100 ng/well), SAM (3 µM) and peptide 20 (1 µM) in assay buffer (10 µl). Reactions were performed in triplicate in a 384-well hi-base microplate at room temperature for 5 h and stopped by the addition of Endo-LysC (4 nM).

An IC₅₀ of 9.7 µM was determined for SAH (Fig. 72). An IC₅₀ value for this inhibitor against EZH2 has not been previously reported in the peer-reviewed literature.

In summary, peptide 20 has been validated as a substrate for the lysine methyltransferase EZH2 in a FLT protease protection assay. The basis of the assay is the use of a 9AA-labelled peptide substrate incorporating an aromatic moiety (e.g. tryptophan) that modulates the FLT of 9AA until the modulator and fluorophore are separated by the action of the protease Endo-LysC, which specifically cleaves adjacent to lysine residues. Lysine methylation by EZH2 confers protection from Endo-LysC cleavage, thereby maintaining modulation of the peptide's FLT. Hence, lysine methylation is reported by a decrease in the measured FLT of the assay.

The FLT EZH2 assay performance was verified by conducting enzyme, SAM and inhibitor titrations.

This study provides further exemplification of the FLT protease protection assay for protein lysine methyltransferases and extends the approach to a lysine-27 histone methyltransferase, EZH2.

### Example 3 - FLT Protease Protection Assay for Protein Demethylases

A means of assaying protein demethylases (PDM), which remove one or more methyl groups from lysine or arginine residues in peptide/protein substrates, via a FLT protease protection approach is shown in Fig. 25. Here, the substrate lifetime of 9-aminoacridine (9AA) (or another suitable reporter) is modulated by the presence of a tryptophan residue in the sequence (or another known modulator of fluorescence lifetime). Treatment of the substrate with a protease that cleaves after lysine or arginine will not induce proteolysis and the lifetime modulation by the tryptophan remains. After lysine or arginine demethylation by an appropriate PDM enzyme, then treatment of the product peptide with the protease induces cleavage after the lysine or arginine and the lifetime of the fluorophore (9AA) is no longer modulated.

Recombinant PKDM (e.g. LSD1, Jumonji-domain classes) enzymes are commercially available from suppliers such as BPS Bioscience, Millipore, or Enzo-Life Sciences. The only PRDM identified to date is JMJD6, (Cheng, B., Chen, Y., Zhao, Y., Bruick, R. K., Science, 2007, 318, 444-447) although recent reports have contradicted its purported demethylase activity, citing its dominant role is to catalyse hydroxylation of lysine residues (Webby, C. J., Wolf, A., Gromak, N., Dreger, M., Kramer, H., Kessler, B., Neilsen, M. L., Schmitz, C., Butler, D. S., Yates III, J. R., Delahunty, C. M., Hahn, P., Lengeling, A., Mann, M., Proudfoot, N. J., Schofield, C. J., and Böttger, A., Science, 2009, 325, 90-93).

JHDM1A is a suitable candidate for proof-of-concept FLT protease protection assays for PKDMs. This enzyme is commercially available from BPS Bioscience and preferentially demethylates lysine-36 on histone H3 converting it from di- or mono-methylated states to the native unmethylated residue (Tsukada, U. et al., Nature, 2006, 439, 811-826). A suitable substrate for the FLT assay is Peptide **10** shown in Fig. 26.

In such an example as shown in Fig. 26, the FLT of 9AA is modulated by the presence of the tryptophan residue incorporated close to the C-terminus. Demethylation of lysine-36 by JHDM1 A would permit protease-induced cleavage at this residue, thereby relieving modulation and resulting in an increase in the FLT of 9AA. In contrast, the presence of a JHDM1 A inhibitor would prevent lysine demethylation thereby leaving the peptide resistant to protease-induced cleavage at lysine-36. Hence, the FLT of 9AA would remain modulated and the extent of the modulation could be correlated to the degree of demethylation. A suitable protease for this purpose would be Endo-LysC, which will not cleave methylated lysines (lysine-37 is protected as the monomethyl derivative to prevent non-specific cleavage of this residue). Endo-LysC is commercially available from suppliers such as Thermo-Fisher.

### JHDM1A Assay

The assay was performed in a 20 µl volume in a 384-well black microplate and contained peptide **10** (1 µM) and JHDM1 A enzyme (150 nM) in assay buffer (50 mM Tris, pH 7.4, 100 µM sodium ascorbate, 10 µM ammonium ferrous sulfate, 100 µM α-ketoglutarate, 0.01 % Tween-20). Control wells containing peptide only were also included. After 5 h incubation at room temperature, 10 µl of Endo-LysC (30 nM) was added to the wells and the plate incubated at room temperature for up to 120 min. FLTs were then measured in triplicate on a NanoTaurus plate reader.

The FLT of the peptide **10** control did not increase after incubation with protease, consistent with the methylated lysine conferring protease protection, resulting in continued modulation of the FLT of 9AA by the intrinsic tryptophan residue (Fig. 35). In contrast, the FLT of peptide **10** after reaction with JHDM1A increased by over 1 ns following protease treatment. This result is consistent with demethylation of lysine-36 by JHDM1A making the peptide susceptible to cleavage by Endo-LysC (Fig. 35). Therefore, demethylation of the substrate by the demethylase, and hence demethylase activity, is reported by an increase in the measured fluorescence lifetime.

These results demonstrate that the FLT protease protection strategy can be extended to assay the protein demethylase target class.

### Example 4 - FLT Protease Protection Assay for Histone Acetyltransferases

A means of assaying histone acetyltransferases (HAT), which acetylate lysine residues in peptide/protein substrates, via a FLT protease protection approach is shown in Fig. 27. Here, the substrate lifetime of 9-aminoacridine (9AA) (or another suitable reporter) is modulated by the presence of a tryptophan residue in the sequence (or another known modulator of fluorescence lifetime). Treatment of the substrate with a protease that cleaves after lysine will induce proteolysis and remove modulation of the fluorescence lifetime by the tryptophan residue. After lysine acetylation by an appropriate HAT enzyme, then treatment of the product peptide with the protease does not induce cleavage after the lysine and the lifetime of the fluorophore (9AA) remains modulated.

Recombinant HAT enzymes (e.g. Gcn5, PCAF, Hat1, p300) are commercially available from suppliers such as abnova or millipore.

Peptide substrates based on histone N-terminal sequences incorporating a fluorophore and modulating residue on opposite sides of a lysine residue which is the site of acetylation for a particular HAT would form the basis of a FLT protease protection assay for this class of enzymes. Peptide 16, is a substrate based on histone H3 sequence with K14 the site of acetylation by PCAF (Fig. 28).
Peptide **16:** WQTARK(Me)STGGK¹⁴APRK(9AA)QLATK-CONH₂

### Endo-LysC Cleavage of Peptide 16

The protease cleavage of peptide **16** was investigated by performing assays in a 384-well microplate containing peptide **16** (1 µM) in the presence or absence of Endo-LysC (25 nM) in assay buffer (50 mM Tris pH 8, 0.1 mM EDTA, 1 mM DTT, 0.05 % BSA) (30 µl). FLTs were measured at regular intervals over one hour with measurements performed in triplicate on a NanoTaurus plate reader.

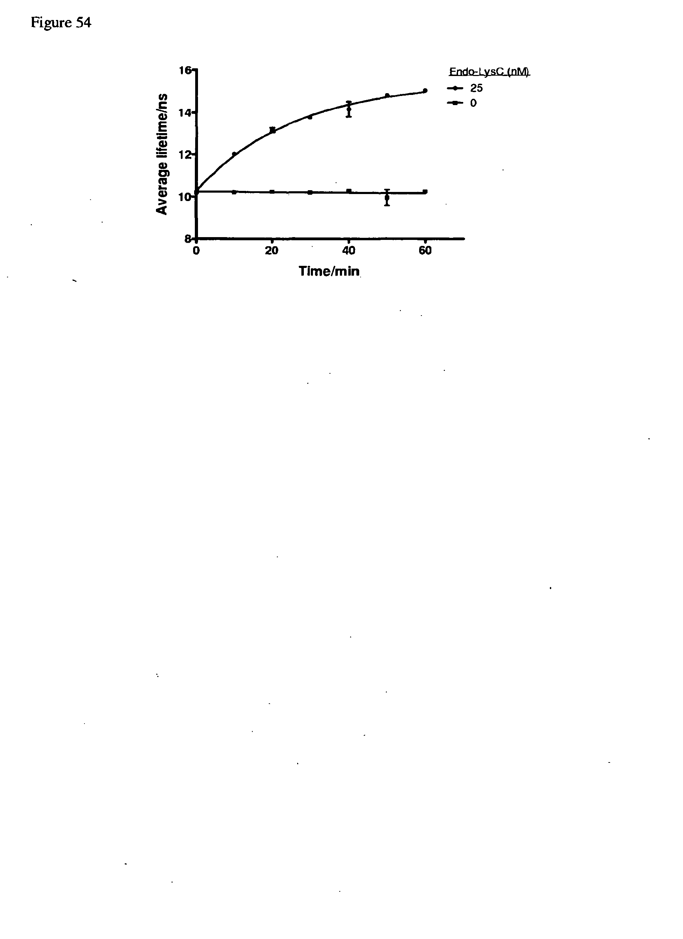

The FLT of peptide **16** increased from 13.9 ns to 15.7 ns when incubated with Endo-LysC for one hour (Fig. 52). In contrast, in the absence of Endo-LysC the FLT remained constant over this time period. These results are consistent with cleavage at lysine-14 by Endo-LysC leading to separation of the 9AA fluorophore from the modulating effect of the tryptophan residue, resulting in an increase in FLT of 9AA. A a second substrate was also designed in which the 9AA and tryptophan were in closer proximity (Fig. 53). The cleavage reaction with Endo-LysC detailed above was repeated with peptide **17** with the results shown in Fig. 54.
Peptide **17:** 9AA-STGGK¹⁴APRWQLATK-CONH₂

For peptide **17,** the FLT increased from 10.2 ns to 15.0 ns after exposure to Endo-LysC, providing an assay range of 4.8 ns, a significant improvement compared to the 1.8 ns range observed with peptide 16. Furthermore, the curve had not reached a plateau after 1 h, providing the option of increasing the assay range still further by prolonging the cleavage reaction.

### Evidence of Acetylation

Peptide 17 (30 µM) was incubated with PCAF (100 nM) and acetyl co-enzyme A (AcCoA) (50 µM) in assay buffer (50 mM Tris pH 8, 0.1 mM EDTA, 1 mM DTT, 0.05 % BSA) (250 µl) and aliquots taken at regular time intervals and analysed by RP-HPLC and ESI-MS to ascertain the degree of acetylation of the peptide substrate by pCAF. The assay was performed at room temperature and samples taken after 0 min, 30 min, 60 min and 90 min time intervals respectively.

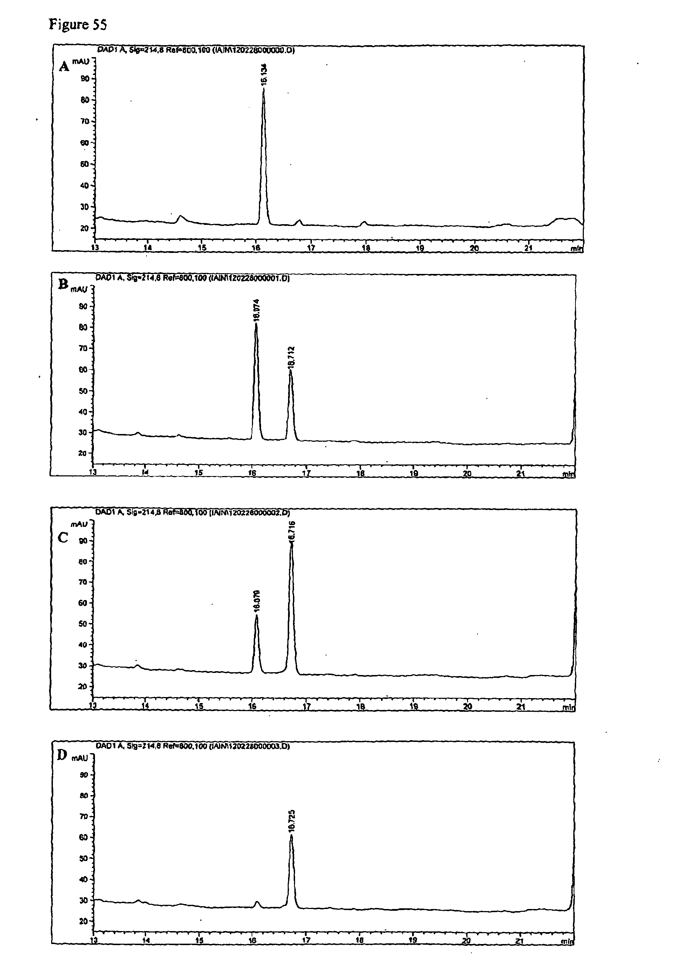

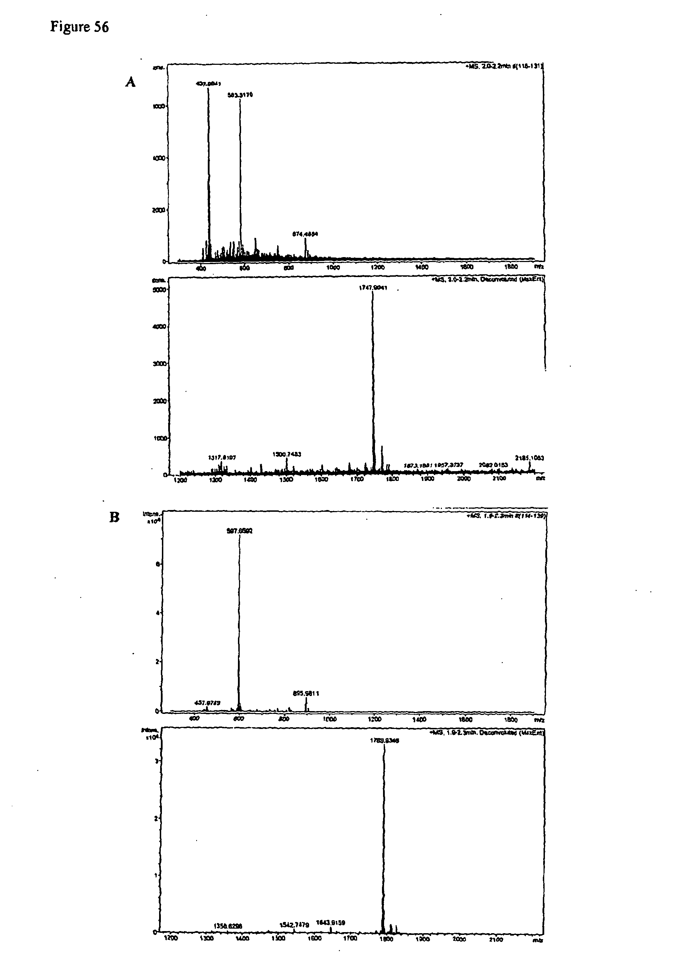

The assay's progress over the course of 90 min is shown via the HPLC spectra in Fig. 55. After 30 min there is a notable appearance of a new peak (t_{R} = 16.7 min) separated from the substrate peak (t_{R} = 16.1 min). Analysis of the peaks by ESI-MS confirmed the peak at t_{R} = 16.7 min to have a mass 42 amu more than the substrate, consistent with the addition of an acetyl group (Fig. 56). By 90 min, the substrate peak is negligible, having been replaced by the peak for acetylated product. Hence, this experiment confirms that peptide **17** is a good substrate for PCAF.

### PCAF Assay Time-course

Assays were performed in a 20 µl volume in a 384-well black microplate and contained peptide **17** (1 µM), AcCoA (50 µM), and PCAF enzyme (250 nM - 31 nM) in assay buffer. Reactions were stopped at regular intervals by the addition of 10 µl Endo-LysC (final concentration 25 nM). FLTs were measured following 90 min incubation with Endo-LysC at room temperature. All measurements were performed in duplicate on a NanoTaurus plate reader.

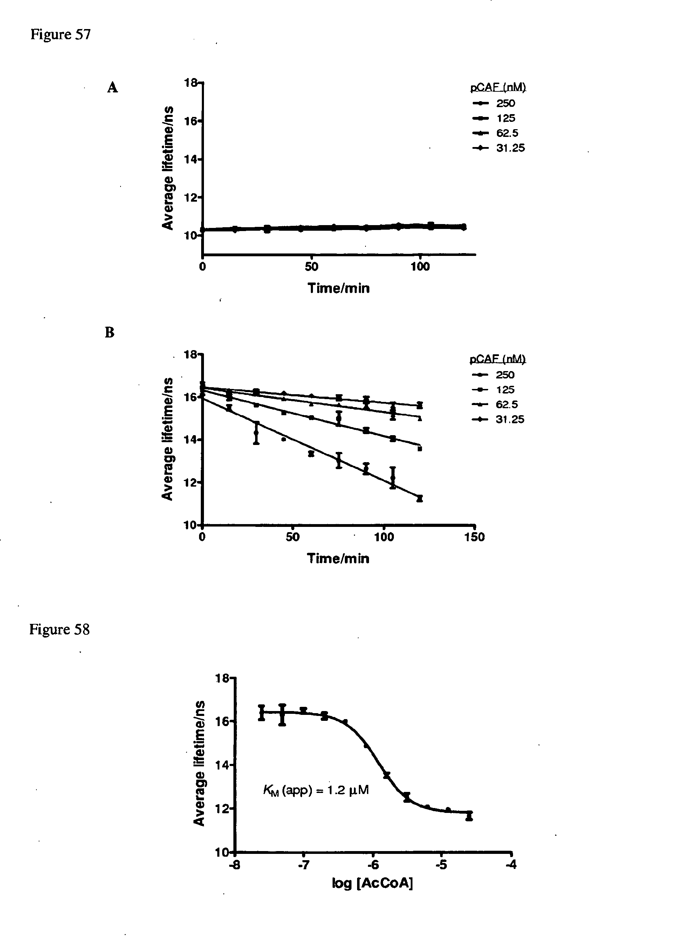

As can be seen from Fig. 57A, the change in the FLT of peptide 17 is negligible prior to the addition of Endo-LysC. However, after addition and incubation with the protease, the FLT decreases as a function of PCAF concentration and assay time, consistent with acetylation of lysine conferring protection to protease induced cleavage (Fig. 57B). In summary, acetylation by PCAF is reported by a decrease in the measured fluorescence lifetime of the assay.

### AcCoA Titration

The dependence of the PCAF assay on AcCoA concentration was investigated. The assay was performed in a 384-well microplate at room temperature for 2 h in assay buffer (20 µl) using 125 nM PCAF and 1 µM peptide **17.** Concentrations of AcCoA were varied from 25 µM - 0.02 µM. On completion of the reaction, Endo-LysC (25 nM) was added to each well and FLTs measured following a 90 min incubation period. The data was fitted to a variable slope dose-response model in GraphPad Prism to determine the apparent *K*_{M} for AcCoA.

From Fig. 58, a *K*_{M} (app) of 1.2 µM was determined for AcCoA in a PCAF assay. This value is consistent with a reported *K*_{M} of 1.6 µM (Poux, A. N; Cebrat, M.; Kim, C. M.; Cole, P. A.; Marmorstein, R. Proc. Natl. Acad. Sci. U.S.A. 2002, 99, 14065-14070).

In summary, peptide **17** has been validated as a substrate for the histone acetyltransferase PCAF in a FLT protease protection assay. The basis of the assay is the use of a 9AA-labelled peptide substrate incorporating an aromatic moiety (e.g. tryptophan) that modulates the FLT of 9AA until the modulator and fluorophore are separated by the action of the protease Endo-LysC, which specifically cleaves adjacent to lysine residues. Lysine acetylation by pCAF confers protection from Endo-LysC cleavage, thereby maintaining modulation of the peptide's FLT. Hence, lysine acetylation is reported by a decrease in the measured FLT of the assay.

The FLT PCAF assay performance was verified by conducting enzyme and SAM titrations.

This study provides exemplification of the FLT protease protection assay for histone acetyltransferases using PCAF as a representative example.

### Example 5 - FLT Protease Protection Assay for Histone Deacetylases

A means of assaying histone deacetylases (HDAC), which deacetylate lysine residues in peptide/protein substrates, via a FLT protease protection approach is shown in Fig. 29. Here, the substrate lifetime of 9-aminoacridine (9AA) (or another suitable reporter) is modulated by the presence of a tryptophan residue in the sequence (or another known modulator of fluorescence lifetime). The substrate is protected from cleavage by lysine specific proteases due to the acetylated nature of the lysine residue, thereby maintaining FLT modulation of the fluorophore. Deacetylation by an appropriate HDAC renders the peptide susceptible to protease-induced cleavage at lysine, leading to separation of the fluorophore from the modulating residue and a concomitant increase in FLT.Recombinant HDAC enzymes (e.g. HDAC1-11, SIRT1-5) are commercially available from suppliers such as Millipore, Signalchem, Sigma-Aldrich, Enzo Life Sciences or BPS Bioscience.

Substrates can be adapted for use in an FLT-based protease protection assay as shown in Fig. 30. Here, the fluorophore (9AA) is incorporated at the C-terminus adjacent to the lysine which is deacetylated, with a tryptophan (or another suitable modulating residue) placed on the N-terminal side of the aforesaid lysine. Protease-induced cleavage following HDAC catalysed deacetylation, would lead to an increase in FLT as 9AA is separated from the tryptophan residue. Hence, an increase in FLT could be directly correlated to the extent of HDAC activity. Of course, it is envisaged that other similarly designed substrates could be employed depending on the sequence specificity of the HDAC in question.

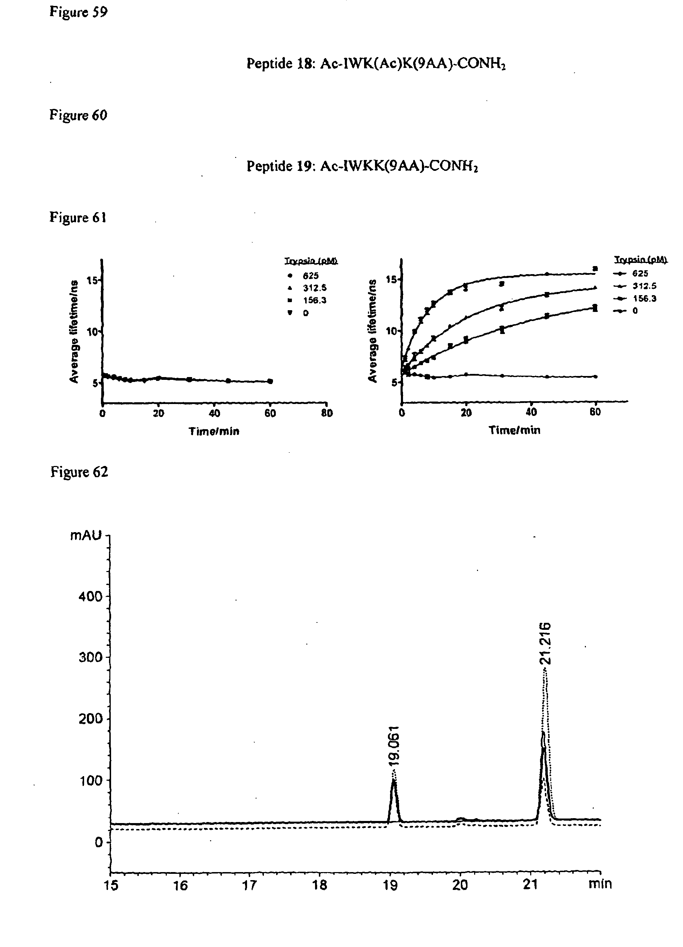

HDAC1 is a suitable candidate for proof-of-concept FLT protease protection assays for histone deacetylases as it is a well characterised enzyme which is commercially available from suppliers such as BPS Bioscience. Although HDAC1 has been reported to deacetylate lysine-27 on histone H3, evidence from the literature suggests that HDAC1 and other members can recognise short peptide substrates containing an acetylated lysine residue of the type shown in Fig. 30 (Riester, D.; Hildmann, C.; Grünewald, S.; Beckers, T.; Schwienhorst, A. Biochem. Biophys. Res. Commun. 2007, 357, 439-445). Hence, a suitable substrate for an HDAC1 FLT protease protection assay is peptide **18** as shown below and in Fig. 59
Peptide **18:** Ac-IWK(Ac)K(9AA)-CONH₂
Peptide **19:** Ac-IWKK(9AA)-CONH₂

On the N-terminal side of the acetylated lysine residue in peptide **18** is a tryptophan residue, while on the C-terminal side there is a lysine residue whose side chain has been labelled with 9AA fluorophore. In the intact substrate, the FLT of 9AA will be modulated by the tryptophan residue, which is located in close proximity. Deacetylation of the lysine residue by HDAC1 to form peptide **19** (Fig. 60) will render the peptide susceptible to cleavage at this site by an appropriate protease, such as trypsin, leading to separation of the 9AA from tryptophan resulting in an increase in FLT. Exposure of the substrate **18** to trypsin would not result in cleavage as the acetylated lysine is not recognised by the protease. Furthermore, the presence of a HDAC1 inhibitor would prevent lysine deacetylation thereby leaving the peptide resistant to protease-induced cleavage. Hence, the FLT of 9AA would remain modulated and the extent of the modulation could be correlated to the degree of inhibition.

### Trypsin Cleavage of Peptides 18 and 19

To examine protease cleavage of peptides **18** and **19,** assays were prepared in a 384-well microplate containing either peptide **18** (1 µM) or **19** (1 µM) and varying concentrations of trypsin (625 pM - 0 pM) in assay buffer (25 mM Tris pH 8, 137 mM NaCl, 2.7 mM KCI, 1 mM MgCl₂, 0.01 % BSA) (30 µl). FLTs were measured at regular intervals over one hour with measurements performed in triplicate on a NanoTaurus plate reader.

No increase in FLT was observed when peptide **18** was exposed to varying concentrations of trypsin, consistent with this protease being unable to cleave adjacent to acetylated lysine (Fig. 61A). In contrast, when the corresponding deacetylated peptide **19** was incubated with trypsin, there was a time and concentration dependent increase in FLT as cleavage at lysine led to the separation of 9AA from the modulating effect of tryptophan (Fig. 61 B). The FLT increased from 5.5 ns to 16 ns, providing an excellent assay range of 10.5 ns.

### Evidence of Deacetylation

Peptide **18** (15 µM) was incubated with HDAC1 (200 nM) in assay buffer (25 mM Tris pH 8, 137 mM NaCl, 2.7 mM KCI, 1 mM MgCl₂, 0.01 % BSA) (200 µl) and aliquots taken at regular time intervals and analysed by RP-HPLC and ESI-MS to ascertain the degree of deacetylation of the peptide substrate by HDAC1. The assay was performed at room temperature and samples taken after 0 h, 2 h, 4 h, and 6 h time intervals respectively.

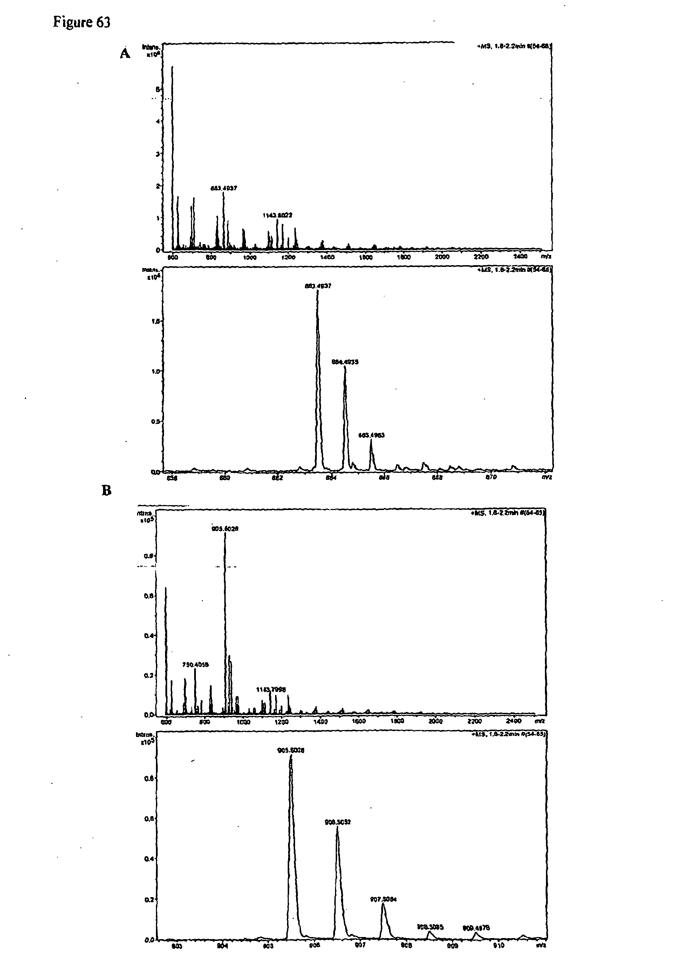

The assay's progress over the course of 6 h is shown via the HPLC spectra in Fig. 62. After 2 h there is a notable appearance of a new peak (t_{R} = 19.1 min) well separated from the substrate peak (t_{R} = 21.2 min). Analysis of the peaks by ESI-MS confirmed the peak at t_{R} = 19.1 min to have a mass 42 amu less than the substrate, consistent with the loss of an acetyl group (Fig. 63). Although the reaction did not appear to have reached completion after 6 h, as evidenced by the continued presence of a substrate peak at t_{R} = 21.2 min, this may have been a result of reagent degradation over time. This experiment confirms that peptide **18** is a substrate for HDAC1.

### HDAC1 Assay Time-course

Assays were performed in a 20 I volume in a 384-well black microplate and contained peptide **18** (1 µM) and HDAC1 enzyme (200 nM - 25 nM) in assay buffer. Reactions were stopped at regular intervals by the addition of 10 µl trypsin (final concentration 10 nM). FLTs were measured following 10 min incubation with trypsin at room temperature. All measurements were performed in duplicate on a NanoTaurus plate reader.

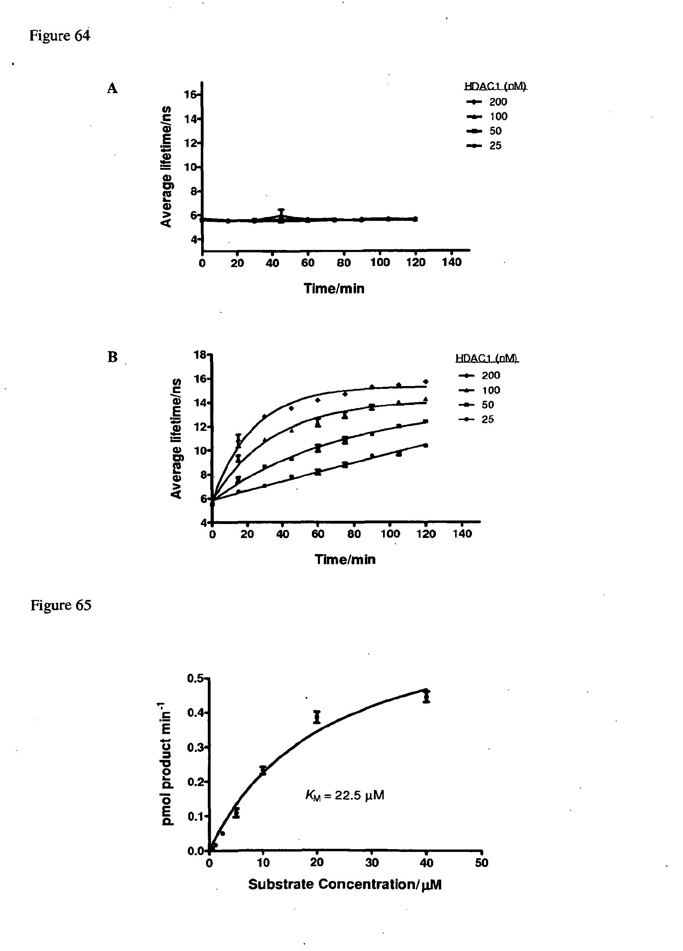

As can be seen from Fig. 64A, the change in the FLT of the assay is negligible prior to the addition of trypsin. However, after addition and incubation with the protease, the FLT increases as a function of HDAC1 concentration and assay time, consistent with deacetylation of lysine rendering the peptide susceptible to protease induced cleavage (Fig. 64B). The use of 200 nM HDAC1 resulted in complete deacetylation after 2 h as indicated by the plateau in FLT value, whereas 25 nM HDAC1 results in a linear increase in signal over time. In summary, deacetylation by HDAC1 is reported by an increase in the measured fluorescence lifetime of the assay.

### Substrate Titration

The dependence of the HDAC1 assay on peptide substrate concentration was investigated. The assay was performed in a 384-well microplate at room temperature for 1 h in assay buffer (20 µl) using 25 nM HDAC1. Concentrations of peptide **18** were varied from 40 µM - 0.3 µM. On completion of the reaction, trypsin (10 nM) was added to each well and FLTs measured following a 10 min incubation period. The data was converted to rate of product formation by reference to a standard curve and then fitted using a Michaelis-Menten model in GraphPad Prism to determine the substrate *K*_{M}.

From Fig. 65, a *K*_{M} of 22.5 µM was determined for substrate **18** in a HDAC1 assay. This value is consistent with those of other short peptide substrates employed in HDAC assays (Riester, D.; Hildmann, C.; Grünewald, S.; Beckers, T.; Schwienhorst, A. Biochem. Biophys. Res. Commun. 2007, 357, 439-445).

### Trichostatin A Inhibitor Titration

In order to demonstrate that the FLT protease protection assay for HDAC1 was suitable for inhibitor screening, the known generic HDAC inhibitor, trichostatin **A** (TSA) was purchased (Sigma) and tested in the assay. Conditions were as follows: TSA (1 µM - 5.6 nM), HDAC1 (25 nM), and peptide **18** (10 µM) in assay buffer (20 µl). Reactions were performed in triplicate in a 384-well microplate at room temperature for 1.5 h and stopped by the addition of trypsin (10 nM).

An IC₅₀ of 0.89 nM was determined for TSA (Fig. 66), which is consistent with a reported IC₅₀ of 1.3 nM from the literature (Halley, F.; Reinshagen, J.; Ellinger, B.; Wolf, M.; Niles; A. M.; Evans, N. J.; Kirkland, T. A.; Wagner, J. M.; Jung, M.; Gribbon, P.; Gul, S. J. Biomol. Screen. 2011, 16, 1227-1235).

### Z'-Factor

To demonstrate that the HDAC1 FLT assay was compatible with high-throughput screening (HTS), a value for the Z'-factor was determined.

Assays were set up in a 384-well microplate, with half the plate containing HDAC1 (25 nM) and peptide **18** (10 µM), whereas the other half contained HDAC1 (25 nM), peptide **18** (10 µM), and TSA inhibitor (1 µM). Final assay volume was 20 µl and the plate was incubated at room temperature for 1.5 h at which point trypsin (10 nM final concentration) in assay buffer (10 µl) was added to each well. Following a further 15 min incubation at room temperature the FLTs were measured and a Z'-factor of 0.85 calculated (Fig. 67), demonstrating the assay's suitability for HTS screening.

In summary, peptide **18** has been validated as a substrate for the histone deacetylase HDAC1 in a FLT protease protection assay. The basis of the assay is the use of a 9AA-labelled peptide substrate incorporating an aromatic moiety (e.g. tryptophan) that modulates the FLT of 9AA until the modulator and fluorophore are separated by the action of the protease trypsin, which specifically cleaves adjacent to lysine (or arginine) residues. Lysine deacetylation by HDAC1 renders the peptide susceptible to trypsin cleavage, leading to an increase in FLT as the 9AA fluorophore is separated from the modulator. Hence, lysine deacetylation is reported by an increase in the measured FLT of the assay.

The FLT HDAC1 assay performance was verified by conducting enzyme, substrate and inhibitor titrations, and a Z'-factor of 0.85 confirms the assay is suitable for HTS applications.

This study provides exemplification of the FLT protease protection assay for histone deacetylases using HDAC1 as a representative example.

### Experimental - materials and methods

The following sections provide further details of the experimental protocols, materials and methods used in Examples 1-5.

### General

Reagents and solvents were purchased from Sigma-Aldrich, Merck Chemicals, New England Biolabs, Thermo-Fisher or Rathburn and used as supplied unless otherwise stated. Human recombinant PAD4 enzyme was purchased from Origene via Insight Biotechnology. Human recombinant G9a enzyme was purchased from Millipore, Abcam, or BPS Bioscience (via AMSBio, UK). Human recombinant PAD2 enzyme was purchased from Modiquest Research, Nijmegen, The Netherlands. Human recombinant JHDM1A, Set7/9, PRMT5, HDAC1 and EZH2/EED/SUZ12/RbAp48/AEBP2 complex were purchased form BPS BioScience via AMSBio, UK and human recombinant PCAF was purchased from Enzo Life Sciences. 3-(9-aminoacridin-2-yl)-propionic acid was prepared by Almac Sciences, Craigavon, NI, and used as supplied. 384-well microplates were purchased from Greiner (# 781076 or 784076).

Mass spectra were obtained on a Bruker microTOF electrospray MS. Analytical RP-HPLC was performed using an Agilent 1100 or 1200 series HPLC system with a Phenomenex Luna C18 column (250 x 4.6 mm) and a gradient of water/acetonitrile containing 0.1 % TFA, with a flow rate of 1 ml/min. Preparative RP-HPLC was performed using a Gilson HPLC system consisting of Gilson 305 pumps, Gilson 811C dynamic mixer and an ABI 783A UV detector, with a Phenomenex Luna C18 column (250 x 21.2 mm) and a gradient of water/acetonitrile containing 0.1 % TFA, with a flow rate of 9 ml/min.

Fluorescence lifetimes were measured on a NanoTaurus Fluorescence Lifetime Plate Reader (Edinburgh Instruments Ltd.) employing time-correlated single photo counting (TCSPC). The excitation source was a semiconductor laser at 405 nm, producing picosecond light pulses at a repetition rate of 5 MHz. Emission was collected through a 438 nm bandpass filter (Semrock) with 20 nm bandwidth. Data was collected to 3000 counts in the peak channel and average lifetimes calculated using a biexponential decay model.

### Peptide Synthesis

Automated solid phase peptide synthesis was carried out on a ABI 433 peptide synthesiser with 0.20-0.25 mMol of Rink amide resin, using standard Fmoc/tBu SPPS chemistry, with 1 mmol of Fmoc-amino acid and HOCt (ethyl-1-hydroxy-1H-1,2,3-triazole-4-carboxylate) or Oxyma Pure (ethyl 2-cyano-2-(hydroxyimino)acetate)/DIC (*N*,*N*'-diisopropylcarbodiimide) coupling reagents per coupling cycle. Unreacted amino groups were capped by treatment with a 0.5 M solution of acetic anhydride in DMF. Fmoc removal was carried out by treatment with a 20 % v/v solution of piperidine in DMF for 20 minutes. Where fluorophore labelling was via a lysine residue, orthogonal deprotection was facilitated by the use of Boc-Lys(Fmoc)-OH or Fmoc-Lys(ivDde)-OH building blocks depending on whether the site of dye attachment was at the N-terminus or within the peptide sequence. The ivDde group was removed on resin by treating with a solution of 3 % hydrazine in DMF.

Manual labelling of peptides with 3-(9-aminoacridin-2-yl)-propionic acid was carried out by dissolving the dye (2 eq wrt resin) in a minimum volume of DMF and activating with 0.5 M HOCt (ethyl-1-hydroxy-1H-1,2,3-triazole-4-carboxylate) (3 eq) and 0.5 M DIC (2 eq) solutions in DMF by ultrasonication for 30 min. The activated dye solution was then added to the resin, pre-swelled in DMF, and the reaction mixture was agitated by ultrasonication at room temperature for 5 h and allowed to stand overnight at room temperature. The resin was filtered and washed thoroughly with DMF and DCM and dried *in vacuo* overnight.

Peptides were cleaved from the solid phase with concomitant side-chain deprotection by treatment with 92.5 % TFA v/v, 5 % H₂O v/v 2.5 % TIS v/v, for 4 h. The resin was removed by filtration and the crude peptide was precipitated from ice-cold ether and collected by centrifugation at 4000 rpm for 5 mins. The crude peptide was then dissolved in MeCN/H₂O (50:50) and lyophilised, then purified to > 95 % purity by preparative HPLC. Labelled peptides were aliquoted based on a molar extinction coefficient for 9AA of 8735 M⁻¹ cm⁻¹ (at 405 nm in 1:1 MeCN/water) with measurements conducted on a Varian Cary 50 UV spectrophotometer.

### Buffer Conditions

PAD4 and PAD2 buffer comprised of 20 mM Tris/HCl at pH 8.0, 200 µM CaCl₂, 5 mM DTT, and 0.01% CHAPS.

G9a, Set7/9, and PRMT5 buffer comprised of 20 mM Tris/HCl at pH 8.0, 25 mM NaCl, 1 mM DTT, and 0.025% Tween-20.

JHDM1A buffer comprised of 50 mM Tris/HCl at pH 7.4, 100 µM sodium ascorbate, 10 µM ammonium ferrous sulfate, 100 µM α-ketoglutarate, and 0.01 % Tween-20. HDAC1 buffer comprised of 25 mM Tris/HCl at pH 8.0, 137 mM NaCl, 2.7 mM KCl, 1 mM MgCl₂, 0.01 % BSA.

pCAF buffer comprised of 50 mM Tris/HCl at pH 8.0, 0.1 mM EDTA, 0.05 % BSA, 1 mM DTT.

EZH2 buffer comprised of 20 mM Bicine at pH 7.6, 50 mM NaCl, 0.002 % Tween-20, 0.005 % BSA, 1 mM DTT.

### Cleavage Assays

Endo-LysC or trypsin in the relevant assay buffer (10 µl) were added to wells in a 384-well microplate. Controls where the protease was replaced with assay buffer were also included. Reactions were started by the addition of peptide substrates in the relevant assay buffer (10 µl) and FLTs measured at regular intervals. All measurements were performed in triplicate.

### PAD4 Assays

For HPLC monitoring, assays were performed at room temperature in micro-centrifuge tubes with a total assay volume of 300 µl. Reactions were started by addition of peptide to solutions containing recombinant PAD4 enzyme. The final concentration of PAD4 in the assay was 30 nM and 15 µM for the peptide. Samples (50 µl) were taken from the assay mixture at the following intervals: 0 min, 30 min, and 60 min and added to a glass HPLC vial (Crawford Scientific) containing 50 µl of water plus 0.1% TFA. The complete 100 µl sample was injected into the HPLC system and relevant peaks collected and diluted in MS buffer (1:1 MeCN/water containing 0.1% formic acid) for analysis.

Where citrullination of the peptide substrate was confirmed, the assay mixtures were diluted 15-fold with assay buffer and transferred to a 384-well microplate (20 µl per well) and FLTs measured. Trypsin (10 µl in assay buffer) was then added to each well and the plate incubated at room temperature for 10 mins. FLTs were then re-measured. All measurements were performed in triplicate and the final concentration of trypsin in the wells was 10 nM.

Alternatively, assays were performed in a 384-well microplate at room temperature with an assay volume of 20 µl per well. To wells containing recombinant PAD4 enzyme in assay buffer (10 µl) was added peptide 1 in assay buffer (10 µl). The plate was shaken and then sealed with a coverslip. The final concentration of PAD4 in the assay varied between 125-500 pM whilst the concentration of peptide 1 was kept constant at 1 µM. Assays were started at different times and stopped together after 90 min by the addition of trypsin (10 µl in assay buffer; final concentration 10 nM). Following incubation at room temperature for 10 min, the microplate was centrifuged at 500 rpm for 1 min and FLTs measured.

For substrate titrations, to wells containing varying concentrations of peptide 1 in assay buffer (10 µl) was added PAD4 in assay buffer (10 µl). The plate was incubated at room temperature for 1 h and then 10 µl of trypsin in assay buffer was added to each well. Following a further 10 min incubation at room temperature, FLTs were measured. All measurements were performed in triplicate.

For Z'-factor determination, half a 384-well microplate was filled with 10 µl of PAD4 and peptide 1 in assay buffer. The remaining wells were filled with 10 µl of PAD4 and peptide 1 in assay buffer containing no CaCl₂. The plate was sealed and incubated at room temperature for 1 h. 10 µl of trypsin in assay buffer was then added to each well and FLTs measured following a 10 min incubation at room temperature. Final concentrations of reagents were: PAD4 (125 pM); peptide 1 (200 nM); trypsin (10 nM).

### PAD2 Assays

For HPLC monitoring, assays were performed at room temperature in micro-centrifuge tubes with a total assay volume of 300 µl. Reactions were started by addition of peptide 1 to solutions containing recombinant PAD2 enzyme. The final concentration of PAD2 in the assay was 30 nM and 15 µM for the peptide. Samples (50 µl) were taken from the assay mixture at the following intervals: 0 min, 30 min, and 120 min and added to a glass HPLC vial (Crawford Scientific) containing 50 µl of water plus 0.1 % TFA. The complete 100 µl sample was injected into the HPLC system and relevant peaks collected and diluted in MS buffer (1:1 MeCN/water containing 0.1 % formic acid) for analysis.

Where citrullination of the peptide substrate was confirmed, the assay mixtures were diluted 15-fold with assay buffer and transferred to a 384-well microplate (20 µl per well) and FLTs measured. Trypsin (10 µl in assay buffer) was then added to each well and the plate incubated at room temperature for 10 mins. FLTs were then re-measured. All measurements were performed in triplicate and the final concentration of trypsin in the wells was 10 nM.

Alternatively, assays were performed in a 384-well microplate at room temperature with an assay volume of 20 µl per well. To wells containing recombinant PAD2 enzyme in assay buffer (10 µl) was added peptide 1 in assay buffer (10 µl). Controls containing peptide or citrullinated peptide only were also included. The plate was shaken and then sealed with a coverslip. The final concentration of PAD2 in the assay varied between 23.5-1.5 nM whilst the concentration of peptide 1 was kept constant at 1 µM. Assays were started at different times and stopped together after 60 min by the addition of trypsin (10 µl in assay buffer; final concentration 10 nM). Following incubation at room temperature for 10 min, the microplate was centrifuged at 500 rpm for 1 min and FLTs measured. All measurements were performed in triplicate.

### G9a Assay

For HPLC monitoring, assays were performed at room temperature in micro-centrifuge tubes with a total assay volume of 150 µl. Reactions were started by addition of SAM (100 µM) to solutions containing recombinant G9a enzyme. The final concentration of G9a in the assay was 100 and 15 µM for the peptide. Samples (50 µl) were taken from the assay mixture at the following intervals: 0 min, 2 h and 6 h and added to a glass HPLC vial (Crawford Scientific) containing 50 µl of water plus 0.1 % TFA. The complete 100 µl sample was injected into the HPLC system and relevant peaks collected and diluted in MS buffer (1:1 MeCN/water containing 0.1 % formic acid) for analysis.

Alternatively, assays were performed in a 384-well microplate at room temperature with an assay volume of 20 µl per well. To wells containing recombinant G9a enzyme in assay buffer (5 µl) was added peptide in assay buffer (10 µl). Reactions were started by the addition of SAM (5 µl). The plate was shaken and then sealed with a coverslip. G9a concentrations varied between 200 nM and 1.5 nM and SAM concentrations between 200 and 0.4 µM. The concentration of peptide was kept constant at 1 µM. Assays were started at different times and stopped together after 2 h by the addition of Endo-LysC (10 µl in assay buffer; final concentration 2 nM). Following incubation at room temperature for 10 min, the microplate was centrifuged at 500 rpm for 1 min and FLTs measured.

For inhibitor titrations, compounds (5 µl) were preincubated with G9a (5 µl) for 30 min at room temperature prior to addition of peptide (5 µl) and SAM (5 µl). Controls containing no SAM or no inhibitor were included.

For Z'-factor determination, half a 384-well microplate was filled, with 10 µl of G9a, peptide 9 and SAM in assay buffer. The remaining wells were filled with 10 µl of G9a and peptide 9 without SAM present. The plate was sealed and incubated at room temperature for 1 h. 10 µl of Endo-LysC in assay buffer was then added to each well and FLTs measured following a 10 min incubation at room temperature. Final concentrations of reagents were: G9a (625 pM); peptide 9 (1 µM); Endo-LysC (2 nM).

### Set7/9 Assays

For HPLC monitoring, assays were performed at room temperature in micro-centrifuge tubes with a total assay volume of 200 µl. Reactions were started by addition of SAM to solutions containing recombinant Set7/9 enzyme and peptide 12. The final concentrations of reagents in the assay were 172 nM (Set7/9), 100 µM (SAM), and 15 µM (peptide 12). Samples (50 µl) were taken from the assay mixture at the following intervals: 0 h, 2 h, and 6 h and added to a glass HPLC vial (Crawford Scientific) containing 50 µl of water plus 0.1 % TFA. The complete 100 µl sample was injected into the HPLC system and relevant peaks collected and diluted in MS buffer (1:1 MeCN/water containing 0.1 % formic acid) for analysis.

Where methylation of the peptide substrate was confirmed, the assay mixture was diluted 15-fold with assay buffer and transferred to a 384-well microplate (20 µl per well) and FLTs measured. Endo-LysC (10 µl in assay buffer) was then added to each well and the plate incubated at room temperature for 15 mins. FLTs were then re-measured. All measurements were performed in triplicate and the final concentration of Endo-LysC in the wells was 2 nM.

Alternatively, assays were performed in a 384-well microplate at room temperature with an assay volume of 20 µl per well. To wells containing recombinant Set7/9 enzyme and peptide **12** or **13** in assay buffer (10 µl) was added SAM (10 µl) in assay buffer. Controls containing peptide only were also included. The plate was shaken and then sealed with a coverslip. The final concentration of Set7/9 in the assay varied between 100 nM and 12.5 nM whilst the concentration of peptide and SAM were kept constant at 1 µM and 100 µM respectively. Assays were started at different times and stopped together after 120 min by the addition of Endo-LysC (10 µl in assay buffer; final concentration 2 nM). Following incubation at room temperature for 15 min, the microplate was centrifuged at 500 rpm for 1 min and FLTs measured. All measurements were performed in duplicate.

For inhibitor titrations, compounds (5 µl) were preincubated with Set7/9 and peptide 13 (5 µl) for 10 min at room temperature prior to addition of SAM (10 µl). Controls containing no SAM or no inhibitor were included.

For Z'-factor determination, 10 µl of a solution containing Set7/9 and peptide 13 in assay buffer was added to each well of a 384-well microplate. To half the plate was added 10 µl of assay buffer whilst to the other half was added SAM in assay buffer (10 µl) to initiate reaction. The plate was sealed and incubated at room temperature for 2 h. Endo-LysC in assay buffer (10 µl) was then added to each well and FLTs measured following a 15 min incubation at room temperature. Final concentrations of reagents were: Set7/9 (25 nM); SAM (100 µM); peptide **13** (1 µM); Endo-LysC (2 nM).

### PRMT5 Assays

For HPLC monitoring, assays were performed at room temperature in micro-centrifuge tubes with a total assay volume of 200 µl. Reactions were started by addition of SAM to solutions containing recombinant PRMT5 enzyme and peptide **15.** The final concentrations of reagents in the assay were 150 nM (PRMT5), 100 µM (SAM), and 15 µM (peptide 15). Samples (50 µl) were taken from the assay mixture at the following intervals: 0 h, 3.5 h, and 17.5 h and added to a glass HPLC vial (Crawford Scientific) containing 50 µl of water plus 0.1 % TFA. The complete 100 µl sample was injected into the HPLC system and relevant peaks collected and diluted in MS buffer (1:1 MeCN/water containing 0.1 % formic acid) for analysis.

Where methylation of the peptide substrate was confirmed, the assay mixture was diluted 15-fold with assay buffer and transferred to a 384-well microplate (20 µl per well) and FLTs measured. Endo-ArgC (10 µl in assay buffer) was then added to each well and the plate incubated at room temperature for 15 mins. FLTs were then re-measured. All measurements were performed in triplicate and the final concentration of Endo-ArgC in the wells was 10 nM.

Alternatively, assays were performed in a 384-well microplate at room temperature with an assay volume of 20 µl per well. To wells containing recombinant PRMT5 enzyme and peptide **15** in assay buffer (10 µl) was added SAM (10 µl) in assay buffer. Controls containing peptide only were also included. The plate was shaken and then sealed with a coverslip. The final concentration of PRMT5 in the assay varied between 100 nM and 12.5 nM whilst the concentration of peptide **15** and SAM were kept constant at 1 µM and 100 µM respectively. Assays were started at different times and stopped together after 120 min by the addition of Endo-ArgC (10 µl in assay buffer; final concentration 1.25 nM). Following incubation at room temperature for 15 min, the microplate was centrifuged at 500 rpm for 1 min and FLTs measured. All measurements were performed in duplicate.

For inhibitor titrations, compounds (5 µl) were preincubated with PRMT5/peptide **15** (5 µl) for 30 min at room temperature prior to the addition of SAM (5 µl). Assays were run for 3 h at room temperature and stopped by the addition of 10 µl Endo-ArgC. FLTs were measured following a 1 h incubation at room temperature. Controls containing no PRMT5 or no inhibitor were also included. All measurements were performed in triplicate.

### EZH2 Assays

Enzyme titrations were performed in a 384-well hi-base microplate at room temperature with an assay volume of 10 µl per well. To wells containing recombinant EZH2 complex in assay buffer (5 µl) was added peptide **20**/SAM mixture in assay buffer (5 µl). Controls containing no EZH2 were also included. The plate was shaken and then sealed with a coverslip. The final concentration of EZH2 in the assay varied between 100 ng/well and 75 ng/well whilst the concentration of peptide **20** and SAM were kept constant at 1 µM and 3 µM respectively. Assays were started at different times and stopped together after 6 h by the addition of Endo-LysC (10 µl in assay buffer; final concentration 4 nM). Following incubation at room temperature for 35 min, the microplate was centrifuged at 500 rpm for 1 min and FLTs measured. All measurements were performed in duplicate.

For SAM titrations, to wells containing varying concentrations of SAM in assay buffer (2.5 µl) was added EZH2 complex in assay buffer (2.5 µl). Reactions were initiated by the addition of peptide **20** in assay buffer (5 µl). The plate was incubated at room temperature for 5 h and then 10 µl of Endo-LysC in assay buffer was added to each well. Following a further 15 min incubation at room temperature, FLTs were measured. All measurements were performed in triplicate.

For inhibitor titrations, compounds (2.5 µl) were preincubated with EZH2 complex (2.5 µl) for 20 min at room temperature prior to addition of peptide **20**/SAM mixture in assay buffer (5 µl). Assays were run for 5 h at room temperature and stopped by the addition of 10 µl Endo-LysC. FLTs were measured following a 1 h incubation at room temperature. Controls containing no EZH2 or no inhibitor were also included. All measurements were performed in triplicate

### JHDM1 A Assays

Assays were performed in a 384-well microplate at room temperature with an assay volume of 20 µl per well. To wells containing recombinant JHDM1A enzyme in assay buffer (10 µl) was added peptide **10** in assay buffer (10 µl). Two sets of control wells containing peptide **10** only were included. The plate was shaken and then sealed with a coverslip. The final concentration of JHDM1 A in the assay was 150 nM and 1 µM for peptide **10.** After incubation at room temperature for 5 h, Endo-LysC (10 µl in assay buffer; final concentration 10 nM) was added to assay wells and one set of control wells. To the other set of control wells was added 10 µl of assay buffer. Following incubation at room temperature for 120 min, the microplate was centrifuged at 500 rpm for 1 min and FLTs measured. All measurements were performed in triplicate.

### PCAF Assays

For HPLC monitoring, assays were performed at room temperature in micro-centrifuge tubes with a total assay volume of 250 µl. Reactions were started by addition of AcCoA to solutions containing recombinant PCAF enzyme and peptide **17.** The final concentrations of reagents in the assay were 100 nM (PCAF), 50 µM (AcCoA), and 30 µM (peptide **17**). Samples (50 µl) were taken from the assay mixture at the following intervals: 0 min, 30 min, 60 min and 90 min and added to a glass HPLC vial (Crawford Scientific) containing 50 µl of water plus 0.1 % TFA. The complete 100 µl sample was injected into the HPLC system and relevant peaks collected and diluted in MS buffer (1:1 MeCN/water containing 0.1 % formic acid) for analysis.

Enzyme titrations were performed in a 384-well microplate at room temperature with an assay volume of 20 µl per well. To wells containing recombinant PCAF enzyme in assay buffer (10 µl) was added peptide **17**/AcCoA mixture in assay buffer (10 µl). Controls containing no PCAF were also included. The plate was shaken and then sealed with a coverslip. The final concentration of PCAF in the assay varied between 250 nM and 31.3 nM whilst the concentration of peptide **17** and AcCoA were kept constant at 1 µM and 50 µM respectively. Assays were started at different times and stopped together after 120 min by the addition of Endo-LysC (10 µl in assay buffer; final concentration 25 nM). Following incubation at room temperature for 90 min, the microplate was centrifuged at 500 rpm for 1 min and FLTs measured. All measurements were performed in duplicate.

For AcCoA titrations, to wells containing varying concentrations of AcCoA (5 µl in assay buffer) was added 5 µl of peptide **17** in assay buffer followed by 10 µl of PCAF in assay buffer. The plate was incubated at room temperature for 2 h and then 10 µl of Endo-LysC in assay buffer was added to each well. Following a further 90 min incubation at room temperature, FLTs were measured. All measurements were performed in triplicate.

### HDAC1 Assays

For HPLC monitoring, assays were performed at room temperature in micro-centrifuge tubes with a total assay volume of 200 µl. Reactions were started by addition of peptide **18** to solutions containing recombinant HDAC1 enzyme. The final concentrations of reagents in the assay were 200 nM (HDAC1), and 15 µM (peptide **18**). Samples (50 µl) were taken from the assay mixture at the following intervals: 0 h, 2 h, 4 h, and 6 h and added to a glass HPLC vial (Crawford Scientific) containing 50 I of water plus 0.1 % TFA. The complete 100 µl sample was injected into the HPLC system and relevant peaks collected and diluted in MS buffer (1:1 MeCN/water containing 0.1 % formic acid) for analysis.

Enzyme titrations were performed in a 384-well microplate at room temperature with an assay volume of 20 µl per well. To wells containing recombinant HDAC1 enzyme in assay buffer (10 µl) was added peptide **18** in assay buffer (10 µl). Controls containing peptide only were also included. The plate was shaken and then sealed with a coverslip. The final concentration of HDAC1 in the assay varied between 200 nM and 25 nM whilst the concentration of peptide was kept constant at 1µM. Assays were started at different times and stopped together after 2 h by the addition of trypsin (10 µl in assay buffer; final concentration 10 nM). Following incubation at room temperature for 10 min, the microplate was centrifuged at 500 rpm for 1 min and FLTs measured. All measurements were performed in duplicate.

For substrate titrations, to wells containing varying concentrations of peptide **18** in assay buffer (10 µl) was added HDAC1 in assay buffer (10 µl). The plate was incubated at room temperature for 1 h and then 10 µl of trypsin in assay buffer was added to each well. Following a further 10 min incubation at room temperature, FLTs were measured. All measurements were performed in triplicate.

For inhibitor titrations, compounds (5 µl) were preincubated with HDAC1 (5 µl) for 30 min at room temperature prior to addition of peptide **18** in assay buffer (10 µl). Assays were run for 1.5 h at room temperature and stopped by the addition of 10 µl trypsin. FLTs were measured following a 10 min incubation at room temperature. Controls containing no HDAC1 or no inhibitor were also included. All measurements were performed in triplicate.

For Z'-factor determination, half a 384-well microplate was filled with 5 µl of assay buffer and 5 µl of HDAC1 in assay buffer. The remaining wells were filled with 5 µl of TSA inhibitor and 5 µl of HDAC1 in assay buffer. After 30 min preincubation at room temperature, 10 µl of peptide **18** in assay buffer was added to each well. The plate was sealed and incubated at room temperature for 1.5 h. 10 µl of trypsin in assay buffer was then added to each well and FLTs measured following a 15 min incubation at room temperature. Final concentrations of reagents were: HDAC1 (25 nM); TSA (1 µM), peptide **18** (10 µM); trypsin (10 nM).

## Claims

1. A method of assaying the activity of a modifying enzyme in a test sample, comprising:
(a) contacting the test sample with a fluorescent-modulated enzyme substrate comprising a linker molecule conjugated to a fluorescent moiety and a fluorescence lifetime modulator moiety configured to modulate the fluorescence lifetime of the fluorescent moiety, wherein the substrate is modified by the action of the modifying enzyme to form a modified substrate, the linker molecule of said modified substrate either being rendered susceptible to cleavage by a second enzyme or protected from cleavage by a second enzyme between the fluorescent moiety and the fluorescence lifetime modulator moiety as a result of the modification, wherein cleavage of the substrate or the modified substrate by the second enzyme separates a portion of the substrate containing the fluorescence lifetime modulator moiety from a portion of the substrate containing the fluorescent moiety; and
(b) detecting formation of the modified substrate by detecting changes in the fluorescence lifetime of the fluorescent moiety as a result of the action of the second enzyme on the modified substrate and/or the substrate, wherein formation of the modified substrate provides an indication of the activity of the modifying enzyme.

2. The method according to claim 1 wherein the linker molecule of the fluorescent-modulated enzyme substrate is a peptide linker and the second enzyme is a protease which cleaves either the peptide linker or a modified peptide linker formed by action of the modifying enzyme on the peptide linker to separate a portion of the substrate containing the fluorescence lifetime modulator moiety from a portion of the substrate containing the fluorescent moiety.

3. The method according to claim 2 wherein the peptide linker is capable of being cleaved by said protease, wherein said cleavage separates a portion of the substrate containing the fluorescence lifetime modulator moiety from a portion of the substrate containing the fluorescent moiety, and modification of the peptide linker by the modifying enzyme protects the modified peptide linker from cleavage by said protease between the fluorescent moiety and the fluorescence lifetime modulator moiety, formation of the modified peptide linker being indicated by a time-dependent decrease in the fluorescence lifetime of the fluorescent moiety after protease treatment, as compared to unmodified peptide linker.

4. The method according to claim 3 wherein the modified peptide linker is formed by methylation of an amino acid residue in the peptide linker, optionally wherein the modifying enzyme is a protein methyl transferase, optionally wherein the protein methyl transferase is a histone lysine methyl transferase enzyme (PKMT), for example G9a, Set7/9, GLP or EZH2,
alternatively wherein the protein methyl transferase is a histone arginine methyl transferase enzyme (PRMT), for example PRMT1, PRMT3, PRMT4/CARM1, or PRMT5.

5. The method according to claim 3 wherein the modified peptide linker is formed by acetylation of an amino acid residue in the peptide linker, optionally wherein the modifying enzyme is an acetyl transferase, optionally wherein the acetyl transferase is a histone acetyl transferase, for example Gcn5, PCAF, Hat1 or p300.

6. The method according to claim 3 wherein the modified peptide linker is formed by deimination of an amino acid residue in the peptide linker, optionally wherein the modifying enzyme is a deiminase, optionally wherein the deiminase is a peptidyl-arginine deiminase, for example PAD1, PAD2, PAD3, PAD4 or PAD6.

7. The method according to claim 2 wherein the peptide linker is not capable of being cleaved by said protease between the fluorescent moiety and the fluorescence lifetime modulator moiety and modification of the peptide linker by the modifying enzyme renders the modified peptide linker susceptible to cleavage by said protease between the fluorescent moiety and the fluorescence lifetime modulator moiety, formation of the modified peptide linker being indicated by a time-dependent increase in the fluorescence lifetime of the fluorescent moiety after protease treatment, as compared to unmodified peptide.

8. The method according to claim 7 wherein the modified peptide linker is formed by demethylation of a methylated amino acid residue in the peptide linker, optionally wherein the modifying enzyme is a demethylase, optionally wherein the demethylase is a histone demethylase, for example LSD1, JHDM1 A or JMJD6.

9. The method according to claim 7 wherein the modified peptide linker is formed by deacetylation of an acetylated amino acid residue in the peptide linker, optionally wherein the modifying enzyme is a deacetylase, optionally wherein the deacetylase is a histone deacetylase, for example HDAC1-11 or SIRT1-5.

10. The method according to any one of claims 1 to 9 wherein the fluorescent moiety is selected from the group consisting of 9-amino acridine and derivatives thereof, acridone derivatives, acridine, quinacridine and acridinium moieties.

11. The method according to any one of claims 1 to 10 wherein the fluorescence lifetime modulator moiety is selected from the group consisting of indolyl moieties, including the indolyl moiety present in the side-chain of the amino acid tryptophan, phenolic moieties, including the phenolic moiety present in the side-chain of the amino acid tyrosine, imidazole moieties, including the imidazole moiety present in the side-chain of the amino acid histidine, and benzyl moieties, including the benzyl side-chain of the amino acid phenylalanine, phenoxy moieties, naphthylalanine moieties, carbazole moieties and phenothiazine moieties.

12. A method of screening for inhibitors of an enzyme, the method comprising assaying the activity of said enzyme using the method of any one of claims 1 to 11 in the presence of a test compound, wherein a reduction in enzyme activity in the presence of the test compound identifies the test compound as an inhibitor of said enzyme.

13. A fluorescent-modulated enzyme substrate comprising a linker molecule conjugated to a fluorescent moiety and a fluorescence lifetime modulator moiety configured to modulate the fluorescence lifetime of the fluorescent moiety, wherein the substrate can be modified by the action of a modifying enzyme to form a modified substrate, the linker molecule of said modified substrate either being rendered susceptible to cleavage by a second enzyme or protected from cleavage by a second enzyme between the fluorescent moiety and the fluorescence lifetime modulator moiety as a result of the modification, wherein cleavage of the substrate or the modified substrate by the second enzyme separates a portion of the substrate containing the fluorescence lifetime modulator moiety from a portion of the substrate containing the fluorescent moiety,
wherein the substrate has the structure 9AA-TARK⁹STGW-CONH₂, with K⁹ representing a lysine residue which is the site of methylation by the action of the PMT enzyme G9a, and 9AA representing 9-aminoacridine.

14. A kit for use in assaying the activity of a methyl transferase, the kit comprising a fluorescent-modulated enzyme substrate according to claim 13 and a protease which cleaves said fluorescent-modulated enzyme substrate between the fluorescent moiety and the fluorescence lifetime modulator moiety.

## Patentansprüche

1. Verfahren zur Untersuchung der Aktivität eines modifizierenden Enzyms in einer Testprobe, das umfasst:
(a) Inkontaktbringen der Testprobe mit einem fluoreszierend modulierten Enzymsubstrat, das ein Linkermolekül umfasst, das mit einer fluoreszierenden Einheit und einer Fluoreszenzlebensdauermodulatoreinheit konjugiert ist, die ausgebildet ist, die Fluoreszenzlebensdauer der fluoreszierenden Einheit zu modulieren, wobei das Substrat durch die Einwirkung des modifizierenden Enzyms unter Bildung eines modifizierten Substrats modifiziert wird, wobei das Linkermolekül des modifizierten Substrats als Ergebnis der Modifikation zwischen der fluoreszierenden Einheit und der Fluoreszenzlebensdauermodulatoreinheit durch ein zweites Enzym gespalten werden kann oder vor einer Spaltung durch ein zweites Enzym geschützt ist, wobei durch das Spalten des Substrats oder des modifizierten Substrats durch das zweite Enzym ein die Fluoreszenzlebensdauermodulatoreinheit enthaltender Teil des Substrats von einem die fluoreszierende Einheit enthaltenden Teil des Substrats abgetrennt wird; und
(b) Erfassen der Bildung des modifizierten Substrats durch Erfassen von Veränderungen in der Fluoreszenzlebensdauer der fluoreszierenden Einheit als Ergebnis der Einwirkung des zweiten Enzyms auf das modifizierte Substrat und/oder das Substrat, wobei die Bildung des modifizierten Substrats ein Anzeichen für die Aktivität des modifizierenden Enzyms ist.

2. Verfahren nach Anspruch 1, worin das Linkermolekül des fluoreszierend modulierten Enzymsubstrats ein Peptidlinker ist und das zweite Enzym eine Protease ist, die entweder den Peptidlinker oder einen modifizierten Peptidlinker spaltet, der durch die Einwirkung des modifizierenden Enzyms auf den Peptidlinker gebildet wurde, um einen die Fluoreszenzlebensdauermodulatoreinheit enthaltenden Teil des Substrats von einem die fluoreszierende Einheit enthaltenden Teil des Substrats abzutrennen.

3. Verfahren nach Anspruch 2, worin der Peptidlinker durch die Protease gespalten werden kann, wobei durch die Spaltung ein die Fluoreszenzlebensdauermodulatoreinheit enthaltender Teil des Substrats von einem die fluoreszierende Einheit enthaltenden Teil des Substrats abgetrennt wird und wobei durch eine Modifikation des Peptidlinkers durch das modifizierende Enzym der modifizierte Peptidlinker vor einer Spaltung zwischen der fluoreszierenden Einheit und der Fluoreszenzlebensdauermodulatoreinheit durch die Protease geschützt ist, wobei die Bildung des modifizierten Peptidlinkers im Vergleich mit dem nicht modifizierten Peptidlinker durch eine zeitabhängige Abnahme der Fluoreszenzlebensdauer der fluoreszierenden Einheit nach der Proteasebehandlung angezeigt wird.

4. Verfahren nach Anspruch 3, worin der modifizierte Peptidlinker durch Methylierung eines Aminosäurerestes in dem Peptidlinker gebildet wird, wobei das modifizierende Enzym optional eine Proteinmethyltransferase ist, wobei die Proteinmethyltransferase optional ein Histon-Lysin-Methyltransferase-Enzym (PKMT), beispielsweise G9a, Set7/9, GLP oder EZH2, ist und
wobei die Proteinmethyltransferase alternativ ein Histon-Arginin-Methyltransferase-Enzym (PRMT), beispielsweise PRMT1, PRMT3, PRMT4/CARM1 oder PRMT5, ist.

5. Verfahren nach Anspruch 3, worin der modifizierte Peptidlinker durch Acetylierung eines Aminosäurerestes in dem Peptidlinker gebildet wird, wobei das modifizierende Enzym optional eine Acetyltransferase ist, wobei die Acetyltransferase optional eine Histon-Acetyl-Transferase, z. B. Gcn5, PCAF, Hat1 oder p300, ist.

6. Verfahren nach Anspruch 3, worin der modifizierte Peptidlinker durch Deiminierung eines Aminosäurerestes in dem Peptidlinker gebildet wird, wobei das modifizierende Enzym optional eine Deiminase ist, wobei die Deiminase optional eine Peptidyl-Arginin-Deiminase, beispielsweise PAD1, PAD2, PAD3, PAD4 oder PAD6, ist.

7. Verfahren nach Anspruch 2, worin der Peptidlinker durch die Protease zwischen der fluoreszierenden Einheit und der Fluoreszenzlebensdauermodulatoreinheit nicht gespalten werden kann und der modifizierte Peptidlinker durch die Modifikation des Peptidlinkers durch das modifizierende Enzym zwischen der fluoreszierenden Einheit und der Fluoreszenzlebensdauermodulatoreinheit durch die Protease gespalten werden kann, wobei die Bildung des modifizierten Peptidlinkers im Vergleich mit dem nicht modifizierten Peptid durch eine zeitabhängige Zunahme der Fluoreszenzlebensdauer der fluoreszierenden Einheit nach der Proteasebehandlung angezeigt wird.

8. Verfahren nach Anspruch 7, worin der modifizierte Peptidlinker durch Demethylierung eines methylierten Aminosäurerestes in dem Peptidlinker gebildet wird, wobei das modifizierende Enzym optional eine Demethylase ist, wobei die Demethylase optional eine Histon-Demethylase ist, beispielsweise LSD 1, JHDM1A oder JMJD6.

9. Verfahren nach Anspruch 7, worin der modifizierte Peptidlinker durch Deacetylierung eines aceylierten Aminosäurerestes in dem Peptidlinker gebildet wird, wobei das modifizierende Enzym optional eine Deacetylase ist, wobei die Deacetylase optional eine Histon-Deacetylase ist, beispielsweise HDAC1-11 oder SIRT1-5.

10. Verfahren nach einem der Ansprüche 1 bis 9, worin die fluoreszierende Einheit unter 9-Amino-Acridin und seinen Derivaten, Acridonderivaten, Acridin, Chinacridin und Acridiniumeinheiten ausgewählt ist.

11. Verfahren nach einem der Ansprüche 1 bis 10, worin die Fluoreszenzlebensdauermodulatoreinheit unter Indolyleinheiten, wobei in der Seitenkette der Aminosäure Tryptophan vorliegende Indolyleinheiten eingeschlossen sind, Phenoleinheiten, wobei in der Seitenkette der Aminosäure Tyrosin vorliegende Phenoleinheiten eingeschlossen sind, Imidazoleinheiten, wobei in der Seitenkette der Aminosäure Histidin vorliegende Imidazoleinheiten eingeschlossen sind, und Benzyleinheiten, wobei die Benzylseitenkette der Aminosäure Phenylalanin eingeschlossen ist, Phenoxyeinheiten, Naphthylalanineinheiten, Carbazoleinheiten und Phenothiazineinheiten ausgewählt ist.

12. Verfahren zum Screening auf Inhibitoren eines Enzyms, wobei das Verfahren umfasst, die Aktivität des Enzyms unter Verwendung des Verfahrens nach einem der Ansprüche 1 bis 11 in Gegenwart einer Testverbindung zu untersuchen, wobei eine Verminderung der Enzymaktivität in Gegenwart der Testverbindung die Testverbindung als Inhibitor des Enzyms identifiziert.

13. Fluoreszierend moduliertes Enzymsubstrat, das ein Linkermolekül umfasst, das mit einer fluoreszierenden Einheit und einer Fluoreszenzlebensdauermodulatoreinheit konjugiert ist, die ausgebildet ist, die Fluoreszenzlebensdauer der fluoreszierenden Einheit zu modulieren, wobei das Substrat durch die Einwirkung eines modifizierenden Enzyms unter Bildung eines modifizierten Substrats modifiziert werden kann, wobei das Linkermolekül des modifizierten Substrats als Ergebnis der Modifikation zwischen der fluoreszierenden Einheit und der Fluoreszenzlebensdauermodulatoreinheit durch ein zweites Enzym gespalten werden kann oder vor einer Spaltung durch ein zweites Enzym geschützt ist, wobei durch das Spalten des Substrats oder des modifizierten Substrats durch das zweite Enzym ein die Fluoreszenzlebensdauermodulatoreinheit enthaltender Teil des Substrats von einem die fluoreszierende Einheit enthaltenden Teil des Substrats abgetrennt wird,
wobei das Substrat die Struktur 9AA-TARK⁹STGW-CONH₂ hat, worin K⁹ einen Lysinrest bedeutet, bei dem es sich um die Methylierungsstelle durch Einwirkung des PMT-Enzyms G9a handelt, und worin 9AA 9-Aminoacridin bedeutet.

14. Kit zur Verwendung bei der Untersuchung der Aktivität einer Methyltransferase, wobei das Kit ein fluoreszierend moduliertes Enzymsubstrat nach Anspruch 13 und eine Protease umfasst, die das fluoreszierend modulierte Enzymsubstrat zwischen der fluoreszierenden Einheit und der Fluoreszenzlebensdauermodulatoreinheit spaltet.

## Revendications

1. Procédé pour analyser l'activité d'une enzyme modificatrice dans un échantillon d'essai, comprenant :
(a) la mise en contact de l'échantillon d'essai avec un substrat d'enzyme à fluorescence modulée comprenant une molécule de liaison conjuguée à un groupement fluorescent et un groupement modulateur de durée de vie de fluorescence configuré pour moduler la durée de vie de fluorescence du groupement fluorescent, le substrat étant modifié par l'action de l'enzyme modificatrice pour former un substrat modifié, la molécule de liaison dudit substrat modifié étant soit rendue susceptible au clivage par une seconde enzyme soit protégée contre le clivage par une seconde enzyme entre le groupement fluorescent et le groupement modulateur de durée de vie de fluorescence en résultat de la modification, le clivage du substrat ou du substrat modifié par la seconde enzyme séparant une portion du substrat contenant le groupement modulateur de durée de durée de vie de fluorescence d'une portion du substrat contenant le groupement fluorescent ; et
(b) la détection de la formation du substrat modifié par détection des variations de durée de vie de fluorescence du groupement fluorescent en résultat de l'action de la seconde enzyme sur le substrat modifié et/ou le substrat, la formation du substrat modifié fournissant une indication de l'activité de l'enzyme modificatrice.

2. Procédé suivant la revendication 1, dans lequel la molécule de liaison du substrat d'enzyme à fluorescence modulée est un groupe peptidique de liaison et la seconde enzyme est une protéase qui clive soit le groupe peptidique de liaison soit un groupe peptidique de liaison modifié formé par l'action de l'enzyme modificatrice sur le groupe peptidique de liaison pour séparer une portion du substrat contenant le groupement modulateur de durée de vie de fluorescence d'une portion du substrat contenant le groupement fluorescent.

3. Procédé suivant la revendication 2, dans lequel le groupe peptidique de liaison est capable d'être clivé par ladite protéase, ledit clivage séparant une portion du substrat contenant le groupement modulateur de durée de vie de fluorescence d'une portion du substrat contenant le groupement fluorescent, et la modification du groupe peptidique de liaison par l'enzyme modificatrice protégeant le groupe peptidique de liaison modifié contre le clivage par ladite protéase entre le groupement fluorescent et le groupement modulateur de durée de vie de fluorescence, la formation du groupe peptidique de liaison modifié étant indiquée par une diminution, en fonction du temps, de la durée de durée de vie de fluorescence du groupement fluorescent après traitement avec la protéase, comparativement au groupe peptidique de liaison non modifié.

4. Procédé suivant la revendication 3, dans lequel le groupe peptidique de liaison modifié est formé par méthylation d'un résidu d'amino-acide dans le groupe peptidique de liaison, éventuellement dans lequel l'enzyme modificatrice est une protéine-méthyl-transférase, éventuellement dans lequel la protéine-méthyl-transférase est une enzyme histone-lysine-méthyl-transférase (PKMT), par exemple G9a, Set7/9, GLP ou EZH2,
en variante dans lequel la protéine-méthyl-transférase est une enzyme histone-arginine-méthyl-transférase (PRMT), par exemple PRMT1, PRMT3, PRMT4/CARM1 ou PRMT5.

5. Procédé suivant la revendication 3, dans lequel le groupe peptidique de liaison modifié est formé par acétylation d'un résidu d'amino-acide dans le groupe peptidique de liaison, éventuellement dans lequel l'enzyme modificatrice est une acétyl-transférase, éventuellement dans lequel l'acétyl-transférase est une histone-acétyl-transférase, par exemple Gcn5, PCAF, Hat1 ou p300.

6. Procédé suivant la revendication 3, dans lequel le groupe peptidique de liaison modifié est formé par désimination d'un résidu d'amino-acide dans le groupe peptidique de liaison, éventuellement dans lequel l'enzyme modificatrice est une désiminase, éventuellement dans lequel la désiminase est une peptidyl-arginine-désiminase, par exemple PAD1, PAD2, PAD3, PAD4 ou PAD6.

7. Procédé suivant la revendication 2, dans lequel le groupe peptidique de liaison n'est pas capable d'être clivé par ladite protéase entre le groupement fluorescent et le groupement modulateur de durée de vie de fluorescence et la modification du groupe peptidique de liaison par l'enzyme modificatrice rend le groupe peptidique de liaison modifié sensible au clivage par ladite protéase entre le groupement fluorescent et le groupement modulateur de durée de vie de fluorescence, la formation du groupe peptidique de liaison modifié étant indiquée par une augmentation, en fonction du temps, de la durée de vie de fluorescence du groupement fluorescent après traitement avec la protéase, comparativement au peptide non modifié.

8. Procédé suivant la revendication 7, dans lequel le groupe peptidique de liaison modifié est formé par déméthylation d'un résidu d'amino-acide méthylé dans le groupe peptidique de liaison, éventuellement dans lequel l'enzyme modificatrice est une déméthylase, éventuellement dans lequel la déméthylase est une histone-déméthylase, par exemple la LSD1, JHDM1A ou JMJD6.

9. Procédé suivant la revendication 7, dans lequel le groupe peptidique de liaison modifié est formé par désacétylation d'un résidu d'amino-acide acétylé dans le groupe peptidique de liaison, éventuellement dans lequel l'enzyme modificatrice est une désacétylase, éventuellement dans lequel la désacétylase st une histone-désacétylase, par exemple HDAC1-11 ou SIRT1-5.

10. Procédé suivant l'une quelconque des revendications 1 à 9, dans lequel le groupement fluorescent est choisi dans le groupe consistant en la 9-amino-acridine et ses dérivés, des dérivés d'acridone, l'acridine, la quinacridine et des groupements acridinium.

11. Procédé suivant l'une quelconque des revendications 1 à 10, dans lequel le groupement modulateur de durée de vie de fluorescence est choisi dans le groupe consistant en des groupements indolyle, y compris le groupement indolyle présent dans la chaîne latérale de l'amino-acide tryptophane, des groupements phénoliques, y compris le groupement phénolique présent dans la chaîne latérale de l'amino-acide tyrosine, des groupements imidazole, y compris le groupement imidazole présent dans la chaîne latérale de l'amino-acide histidine, et des groupements benzyle, y compris la chaîne latérale benzyle de l'amino-acide phénylalanine, des groupements phénoxy, des groupements naphtylalanine, des groupes carbazole et des groupements phénothiazine.

12. Procédé pour sélectionner des inhibiteurs d'une enzyme, le procédé comprenant l'analyse de l'activité de ladite enzyme en utilisant le procédé de l'une quelconque des revendications 1 à 11 en présence d'un composé d'essai, dans lequel une réduction de l'activité de l'enzyme en présence du composé d'essai identifie le composé d'essai en tant qu'inhibiteur de ladite enzyme.

13. Substrat d'enzyme à fluorescence modulée comprenant une molécule de liaison conjuguée à un groupement fluorescent et un groupement modulateur de durée de vie de fluorescence configuré pour moduler la durée de vie de fluorescence du groupement fluorescent, le substrat pouvant être modifié par l'action d'une enzyme modificatrice pour former un substrat modifié, la molécule de liaison dudit substrat modifié étant soit rendue sensible au clivage par une second enzyme soit protégée contre le clivage par une seconde enzyme entre le groupement fluorescent et le groupement modulateur de durée de vie de fluorescence en résultat de la modification, le clivage du substrat ou du substrat modifié par la seconde enzyme séparant une portion du substrat contenant le groupement modulateur de durée de vie de fluorescence d'une portion du substrat contenant le groupement fluorescent,
le substrat ayant la structure 9AA-TARK⁹STGW-CONH₂, avec K⁹ représentant un résidu de lysine qui est le site de méthylation par l'action de l'enzyme PMT G9a, et 9AA représentant la 9-amino-acridine.

14. Kit pour une utilisation dans l'analyse de l'activité d'une méthyl-transférase, le kit comprenant un substrat d'enzyme à fluorescence modulée suivant la revendication 13 et une protéase qui clive ledit substrat d'enzyme à fluorescence modulée entre le groupement fluorescent et le groupement modulateur de durée de vie de fluorescence.
